# EUROPEAN PATENT SPECIFICATION

(11) **EP 1 165 495 B1**
(45) Date of publication and mention of the grant of the patent: **20.04.2005**
(21) Application number: 00909558.9
(22) Date of filing: 16.03.2000
(51) Int. Cl.: C07C 251/44, C07C 69/76, C07C 69/608, C07C 311/07, C07C 317/34, C07C 259/08, A61K 7/48, A61K 7/42

(54) **RESORCINOL DERIVATIVES**
RESORCINDERIVATE
DERIVES DE RESORCINOL

(30) Priority: 22.03.1999 US 125534 P
(43) Date of publication of application: 02.01.2002
(73) Proprietor: PFIZER INC., New York, N.Y. 10017 (US)
(72) Inventor: COLLINGTON, Eric William, Knebworth, Hertfordshire SG3 6AH (GB); PROCTER, Martin James, Walsall, West Midlands WS3 4QX (GB); GEDEN, Joanna, Victoria, Coventry CV5 6YW (GB); BROWNING, Andrew, Francis, S-741 90 Knivsta (SE)
(74) Representative: Hayles, James Richard
(86) International application number: PCT/IB2000/000286
(87) International publication number: WO 2000/056702

(56) References cited:
- EP-A- 0 341 664
- EP-A- 0 526 302
- EP-A- 0 904 774
- DE-A- 2 839 836

## Description

### Field Of The Invention

The present invention relates to the use of certain resorcinol derivatives as skin lightening agents.

### Background Of The Invention

The terms "lightening agent" and "depigmentation agent" are used interchangeably throughout this document.

Skin color in humans arises from a complex series of cellular processes that are carried out within a unique population of cells called melanocytes. Melanocytes are located in the lower part of the epidermis, and their function is to synthesize a pigment, melanin, which protects the body from the damaging effects of ultraviolet radiation.

When skin is exposed to ultraviolet radiation, such as that contained in sunlight, melanocytes increase their synthesis of melanin. Melanin is deposited in melanosomes, which are vesicles found within the cell. The melanosomes are extruded from the cell and carried to the surface of the skin by keratinocytes, which internalize the melanin-containing melanosomes. The end result is that the visible layers of the skin exhibit a brown color typically known as a "tan". The darkness of the color observed in the skin is proportionate to the amount of melanin synthesized by melanocytes and transferred to the keratinocytes.

The mechanism by which skin pigmentation is formed, melanogenesis, is particularly complex and schematically involves the following main steps: Tyrosine→L-Dopa→Dopaquinone→Dopachrome→Melanins. The first two reactions in this series are catalyzed by the enzyme tyrosinase. The activity of tyrosinase is promoted by the action of α-melanocyte stimulating hormone or UV rays to have melanin eventually formed as chromatism in the skin. It is well established that a substance has a depigmenting effect if it acts directly on the vitality of the epidermal melanocytes where melanogenesis normally occurs and/or if it interferes with one of the stages in melanin biosynthesis. The active compounds that are employed in the various methods and compositions of this invention inhibit tyrosinase and thus inhibit or decrease melanin biosynthesis.

There is a strong demand for agents that enable acquired deposition sites, such as spots or freckles, to be restored to a normal skin color. For this purpose, a variety of agents and methods have been developed and put on the market. Examples of such methods are (a) a method wherein vitamin C (L-ascorbic acid) having good reducing ability is administered orally in large amounts, (b) a method wherein glutathione is administered parenterally; (c) a method wherein a peroxide, such as hydrogen peroxide, zinc peroxide, sodium peroxide and the like, which is believed to have the bleaching action of melamine, is administered: and (d) a method wherein vitamin C or cysteine is administered topically in the form of an ointment, cream, lotion or the like. Vitamin C has a problem with respect to stability and becomes so unstable in water-containing systems that they will cause changes in odor and color. Thiol compounds such as glutathione and cysteine do not exhibit a satisfactory depigmental effect since the development of the effect is very slow.

The substances in widest use at the present time as depigmentors are, in particular, hydroquinone and its derivatives, particularly its ethers such as hydroquinone monomethyl ether. These compounds, while effective, are known to produce side effects that can be dangerous. Hydroquinone, use of which is limited to a concentration of 2%, is both irritating and cytotoxic to the melanocyte.

United States Patent 4,526,179 refers to certain hydroquinone fatty esters that have good activity and are less irritating and more stable than hydroquinone.

Japanese Patent Application No. 27909/86 refers to other hydroquinone derivatives that do not have the drawbacks of hydroquinone but that have relatively poor efficacy.

United States Patent 5,449,518 refers to 2,5-dihydoxyphenyl carboxylic acid derivatives as skin depigmentation agents.

European Patent Application EP 341,664A1 refers to certain resorcinol derivatives as tyrosinase inhibitors and skin depigmentation agents.

PCT International Publication WO 99/15148 refers to certain resorcinol derivatives as tyrosinase inhibitors and skin depigmentation agents.

The use of topical depigmention agents that have good efficacy and are harmless is particularly desirable for treating the following: regional hyperpigmentation caused by melanocytic hyperactivity, such as idiopathic melasma occurring either during pregnancy (mask of pregnancy or chloasma) or secondary to estrogen-progesterone contraception; local hyperpigmentation caused by benign melanocytic hyperactivity and proliferation such as lentigo senilis or liver spots; accidental hyperpigmentation such as post-lesional photosensitization and scarring; and certain forms of leukoderma such as vitiligo where, if the injured skin cannot be repigmented, the residual zones of normal skin are depigmented to impart a homogeneous white color to the entire skin.

### Summary of Invention

The resorcinol derivatives of formula I, which are defined below and used in the various methods and compositions of this invention, are useful in preparation of medicaments for the treatment of the foregoing dermatological conditions as well as other dermatological conditions, some of which are referred to later in this document, for which the subject being treated desires, for cosmetic purposes, to lighten or reduce the pigmentation of the skin affected by the condition.

The resorcinol derivatives of formula I are also useful for the preparation of medicaments useful in the treatment of inflammatory disorders such as psoriasis, dermatitis and acne, and for the treatment of dandruff.

The invention thus provides a compound of formula I: 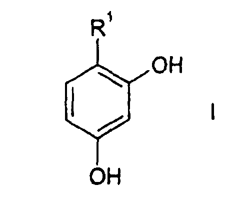 or a pharmaceutically acceptable salt thereof, wherein:
R¹ is a (C₃-C₈)cycloalkyl ring or (C₅-C₈)cycloalkenyl ring, wherein either the cycloalkyl ring or cycloalkenyl ring is substituted by one to three substituents independently selected from the group consisting of cyano; halo; (C₁-C₆)alkyl; aryl; (C₂-C₉)heterocycloalkyl; (C₂-C₉)heteroaryl; aryl(C₁-C₆)alkyl-; =O; =CHO(C₁-C₆)alkyl; amino; hydroxy; (C₁-C₆)alkoxy; aryl(C₁-C₆)alkoxy-; (C₁-C₆)acyl; (C₁-C₆)alkylamino-; aryl(C₁-C₆)alkylamino-; amino(C₁-C₆)alkyl-; (C₁-C₆)alkoxy-CO-NH-; (C₁-C₆)alkylamino-CO-; (C₂-C₆)alkenyl; (C₂-C₆)alkynyl; hydroxy(C₁-C₆)alkyl-; (C₁-C₆)alkoxy(C₁-C₆)alkyl-; (C₁-C₆)acyloxy(C₁-C₆)alkyl-; nitro; cyano(C₁-C₆)alkyl-; halo(C₁-C₆)alkyl-; nitro(C₁-C₆)alkyl-; trifluoromethyl; trifluoromethyl(C₁-C₆)alkyl-; (C₁-C₆)acylamino-; (C₁-C₆)acylamino(C₁-C₆)alkyl-; (C₁-C₆)alkoxy(C₁-C₆)acylamino-; amino(C₁-C₆)acyl-; amino(C₁-C₆)acyl(C₁-C₆)alkyl-: (C₁-C₆)alkylamino(C₁-C₆)acyl-; ((C₁-C₆) alkyl)₂amino(C₁-C₆)acyl-; -CO₂R²; -(C₁-C₆)alkyl-CO₂R²; -C(O)N(R²)₂; -(C₁-C₆)alkyl-C(O)N(R²)₂; R²ON=; R²ON=(C₁-C₆)alkyl-; R²ON=CR²(C₁-C₆)alkyl-; -NR²(OR²); -(C₁-C₆)alkyl-NR²(OR²); -C(O)(NR²OR²); -(C₁-C₆)alkyl-C(O)(NR²OR²); -S(O)ₘR²; wherein each R² is independently selected from hydrogen, (C₁-C₆)alkyl, aryl, or aryl(C₁-C₆)alkyl-; R³C(O)O-, wherein R³ is (C₁-C₆)alkyl, aryl, or aryl(C₁-C₆)alkyl-; R³C(O)O-(C₁-C₆)alkyl-; R⁴R⁵N-C(O)-O-; R⁴R⁵NS(O)₂-; R⁴R⁵NS(O)₂(C₁-C₆)alkyl-; R⁴S(O)₂R⁵N-; R⁴S(O)₂R⁵N(C₁-C₆)alkyl-; wherein m is 0, 1 or 2, and R⁴ and R⁵ are each independently selected from hydrogen or (C₁-C₆)alkyl; -C(=NR⁶)(N(R⁴)₂); or -(C₁-C₆)alkyl-C(=NR⁶)(N(R⁴)₂) wherein R⁶ represents OR² or R² wherein R² is defined as above;
with the proviso that the cycloalkenyl ring is not aromatic;
with the proviso that R¹ must be substituted by at least one of R³C(O)O- (C₁-C₆)alkyl-, R²ON=, R²ON=(C₁-C₆)alkyl-, R²ON=CR²(C₁-C₆)alkyl-, -NR²(OR²), R⁴R⁵NS(O)₂-, R⁴R⁵NS(O)₂(C₁-C₆)alkyl-, R⁴S(O)₂R⁵N-, or R⁴S(O)₂R⁵N(C₁-C₆)alkyl-;
with the proviso that when R¹ is only substituted by one of R²ON=, then R² cannot be hydrogen.

Where R¹ is a cyclohexyl or cyclohexenyl ring, the ring is preferably substituted at the 3- or 4-position, and more preferably at the 4-position.

Where R¹ is a cyclopentyl or cyclopentenyl ring, the ring is preferably substituted at the 3-position.

In a preferred embodiment, R¹ is monosubstituted.

In a further preferred embodiment, R¹ is disubstituted.

In a preferred embodiment, R¹ is substituted as described in claim 5.

In a further preferred embodiment, R¹ is substituted by at least one of R²ON=, R²ON=(C₁-C₆)alkyl-, or R²ON=CR²(C₁-C₆)alkyl-; with the proviso that when R¹ is only substituted by one of R²ON=, then R² cannot be hydrogen.

In a further preferred embodiment, R¹ is substituted by at least one of R²ON=, with the proviso that when R¹ is only substituted by one of R²ON=, then R² cannot be hydrogen.

In a further preferred embodiment, R¹ is substituted by at least one of -NR²(OR²).

In a further preferred embodiment, R¹ is substituted by at least one of R⁴R⁵NS(O)₂-, R⁴R⁵NS(O)₂(C₁-C₆)alkyl-, R⁴S(O)₂R⁵N-, or R⁴S(O)₂R⁵N(C₁-C₆)alkyl-.

In a further preferred embodiment, R¹ is substituted by at least one of R⁴S(O)₂R⁵N-.

In a further preferred embodiment, R¹ is substituted by at least one of R⁴S(O)₂R⁵N(C₁-C₆)alkyl-.

In a further preferred embodiment, R¹ is a (C₃-C₈)cycloalkyl ring or (C₅-C₈)cycloalkenyl ring, wherein either the cycloalkyl ring or cycloalkenyl ring is substituted by one of R³C(O)O-(C₁-C₆)alkyl-, R²ON=, R²ON=(C₁-C₆)alkyl-, R²ON=CR²(C₁-C₆)alkyl-, -NR²(OR²), R⁴R⁵NS(O)₂-, R⁴R⁵NS(O)₂(C₁-C₆)alkyl-, R⁴S(O)₂R⁵N-, or R⁴S(O)₂R⁵N(C₁-C₆)alkyl-; wherein R², R³, R⁴ and R⁵ are as defined above;
with the proviso that the cycloalkenyl ring is not aromatic; and with the proviso that when R¹ is substituted by R²ON=, then R² cannot be hydrogen.
In a further preferred embodiment, R¹ is a (C₃-C₈)cycloalkyl ring or (C₅-C₈)cycloalkenyl ring, wherein either the cycloalkyl ring or cycloalkenyl ring is substituted by one of R³C(O)O-(C₁-C₆)alkyl-, R²ON=, or R⁴S(O)₂R⁵N-; wherein R², R³, R⁴ and R⁵ are as defined above;
with the proviso that the cycloalkenyl ring is not aromatic;
and with the proviso that when R¹ is substituted by R²ON=, then R² cannot be hydrogen.

In a further preferred embodiment, R¹ is substituted by R³C(O)O-(C₁-C₆)alkyl-.

In a further preferred embodiment, R¹ is substituted by R²ON=, R²ON=(C₁-C₆)alkyl-, or R²ON=CR²(C₁-C₆)alkyl-, with the proviso that when R¹ is substituted by R²ON=, then R² cannot be hydrogen.

In a further preferred embodiment, R¹ is substituted by R²ON=, where R² cannot be hydrogen.

In a further preferred embodiment, R¹ is substituted by -NR²(OR²).

In a further preferred embodiment, R¹ is substituted by R⁴R⁵NS(O)₂-, R⁴R⁵NS(O)₂(C₁-C₆)alkyl-, R⁴S(O)₂R⁵N- or R⁴S(O)₂R⁵N(C₁-C₆)alkyl-.

In a further preferred embodiment, R¹ is substituted by R⁴S(O)₂R⁵N-.

In a further preferred embodiment, R¹ is substituted by R⁴S(O)₂R⁵N(C₁-C₆) alkyl-.

The (C₂-C₉)heterocycloalkyl substituent, when present on R¹, is preferably a group of the formula: 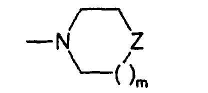 wherein m is as defined above, and
Z is CH₂, NR², O, S, SO, or SO₂.

For any of the aforementioned compounds of the present invention, R' is preferably a group of the formula: 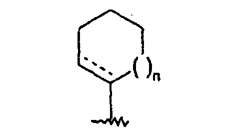 which is substituted as described above for R¹;
wherein n is 0, 1, or 2;
wherein the dashed line indicates an optional double bond at that position.
In a preferred embodiment, n is 0 or 1.

In a further preferred embodiment, n is 0; and the dashed line represents a double bond at that position.

In a further preferred embodiment, n is 1.

In a further preferred embodiment, R¹ is substituted by =O, =NOH, CH₂OH, 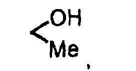 or a combination thereof.

In a further preferred embodiment, n is 0; R¹ is substituted by =NOH; and the dashed line represents a double bond at that position.

In further preferred embodiment, n is 1; and R¹ is substituted by =O, =NOH, CH₂OH, or 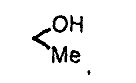 or a combination thereof.

The invention further provides a compound selected from the group consisting of:
O-Benzyl-4-(2, 4-dihydroxyphenyl)cyclohexanone oxime;
(±)-*N*-[3-(2,4-Dihydroxyphenyl)cyclohexyl]methanesulfonamide;
(±)-*O*-Methyl-3-(2,4-dihydroxyphenyl)cyclohexanone oxime;
(±)-*O*-Benzyl-3-(2,4-dihydroxyphenyl)cyclohexanone oxime;
and a pharmaceutically acceptable salt thereof.

The invention further provides a compound selected from the group consisting of:
O-Methyl-4-(2,4-dihydroxyphenyl)cyclohexanone oxime;
(±)-4-[3-(Hydroxyamino)cyclohexyl]-1,3-benzenediol;
cis -*N*-[4-(2,4-Dihydroxyphenyl)cyclohexyl]-1-butanesulfonamide;
*trans-N-*[4-(2,4-Dihydroxyphenyl)cyclohexyl]methanesulfonamide;
*cis-N-*[4-(2,4-Dihydroxyphenyl)cyclohexyl]methanesulfonamide;
*cis*/*trans*- 4- [4-(hydroxyamino)cyclohexyl]-1,3 - benzenediol;
*trans-*4-[4-(methoxyamino)cyclohexyl]-1,3 - benzenediol; and a pharmaceutically acceptable salt thereof.

The present invention further provides a topical pharmaceutical composition for lightening skin or reducing the pigmentation of skin in a human, comprising a pharmaceutically acceptable carrier, and a skin-lightening or pigmentation-reducing amount of a compound of formula 1, as defined above, or a pharmaceutically acceptable salt thereof.

The present invention further provides a cosmetic method of lightening skin or reducing the pigmentation of skin in a human patient, comprising administering to the patient a skin-lightening or skin pigmentation-reducing effective amount of a compound of formula I, as defined above, or a pharmaceutically acceptable salt thereof, until a cosmetically beneficial amount of skin-lightening or skin pigmentation-reduction has been achieved.

The present invention further provides the use of a compound of formula I, as defined above, or a pharmaceutically acceptable salt thereof, in the manufacture of a skin-lightening or pigmentation-reducing medicament.

The present invention further provides a topical or transdermal pharmaceutical composition for the treatment of an inflammatory disorder such as psoriasis, dermatitis or acne, or for the treatment of dandruff, in a human, comprising a pharmaceutically acceptable carrier, and an amount of a compound of formula I, or a pharmaceutically acceptable salt thereof, used in any of the aforementioned pharmaceutical compositions of the present invention, which amount is effective in treating such disorder or condition.

The term "alkyl", as used herein, unless otherwise indicated, includes saturated monovalent hydrocarbon radicals having straight, branched or cyclic moieties or combinations thereof. Any substituents or functional groups on the alkyl group, as indicated herein, can be substituted anywhere on the alkyl group.

The term "aryl", as used herein, refers to phenyl or naphthyl optionally substituted with one or more substituents, preferably from zero to two substituents, independently selected from halogen, OH, (C₁-C₆)alkyl, (C₁-C₆) alkoxy, amino, (C₁-C₆)alkylamino, di-((C₁-C₆)alkyl))amino, nitro, cyano and trifluoromethyl. Any substituents or functional groups on the aryl group, as indicated herein, can be substituted anywhere on the aryl group.

The term "one or more substituents", as used herein, refers to a number of substituents that equals from one to the maximum number of substituents possible based on the number of available bonding sites.

The "halo", as used herein, refers to halogen and, unless otherwise indicated, includes chloro, fluoro, bromo and iodo.

The term "acyl", as used herein, unless otherwise indicated, includes a radical of the general formula RCO wherein R is alkyl, alkoxy, aryl, arylalkyl, or arylalkyloxy and the terms "alkyl" or "aryl" are as defined above.

The term "acyloxy", as used herein, includes O-acyl groups wherein "acyl" is as defined above.

(C₂-C₉)Heterocycloalkyl, when used herein, refers to pyrrolidinyl, tetrahydrofuranyl, dihydrofuranyl, tetrahydropyranyl, pyranyl, thiopyranyl, aziridinyl, oxiranyl, methylenedioxyl, chromenyl, isoxazolidinyl, 1,3-oxazolidin-3-yl, isothiazolidinyl, 1,3-thiazolidin-3-yl, 1,2-pyrazolidin-2-yl, 1,3-pyrazolidin-1-yl, piperidinyl, thiomorpholinyl, 1,2-tetrahydrothiazin-2-yl, 1,3-tetrahydrothiazin-3-yl, tetrahydrothiadiazinyl, morpholinyl, 1,2-tetrahydrodiazin-2-yl, 1,3-tetrahydrodiazin-1-yl, tetrahydroazepinyl, piperazinyl, chromanyl, etc. One of ordinary skill in the art will understand that the connection of said (C₂-C₉)heterocycloalkyl ring can be through a carbon atom or through a nitrogen heteroatom where possible.

(C₂-C₉)Heteroaryl, when used herein, refers to furyl, thienyl, thiazolyl, pyrazolyl, isothiazolyl, oxazolyl, isoxazolyl, pyrrolyl, triazolyl, tetrazolyl, imidazolyl, 1,3,5-oxadiazolyl, 1,2,4-oxadiazolyl, 1,2,3-oxadiazolyl, 1,3,5-thiadiazolyl, 1,2,3-thiadiazolyl, 1,2,4-thiadiazolyl, pyridyl, pyrimidyl, pyrazinyl, pyridazinyl, 1,2,4-triazinyl, 1,2,3-triazinyl, 1,3,5-triazinyl, pyrazolo[3,4-b]pyridinyl, cinnolinyl, pteridinyl, purinyl, 6,7-dihydro-5H-[1]pyridinyl, benzo[b]thiophenyl, 5, 6, 7, 8-tetrahydro-quinolin-3-yl, benzoxazolyl, benzothiazolyl, benzisothiazolyl, benzisoxazolyl, benzimidazolyl, thianaphthenyl, isothianaphthenyl, benzofuranyl, isobenzofuranyl, isoindolyl, indolyl, indolizinyl, indazolyl, isoquinolyl, quinolyl, phthalazinyl, quinoxalinyl, quinazolinyl, benzoxazinyl, etc. One of ordinary skill in the art will understand that the connection of said (C₂-C₉)heterocycloalkyl rings can be through a carbon atom or through a nitrogen heteroatom where possible.

Compounds of formula I may contain chiral centers and therefore may exist in different enantiomeric and diastereomeric forms. This invention relates to all optical isomers, stereoisomers and tautomers of the compounds of formula I, and mixtures thereof, and to all pharmaceutical compositions and methods of treatment defined above that contain or employ them, respectively.

Formula I, as defined above, also includes compounds identical to those depicted but for the fact that one or more hydrogen, carbon or other atoms are replaced by isotopes thereof. Such compounds may be useful as research and diagnostic tools in metabolism pharmacokinetic studies and in binding assays.

The present invention also relates to the pharmaceutically acceptable acid addition and base salts of any of the aforementioned compounds of formula I. The acids which are used to prepare the pharmaceutically acceptable acid addition salts of the aforementioned base compounds of this invention are those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate (i.e., 1,1-methylene-bis-(2-hydroxy-3-naphthoate)) salts.

### Detailed Description of the Invention

The compounds of formula I may be prepared as described in the following reaction schemes and discussion. Unless otherwise indicated, n, m, R², R³, R⁴, R⁵, Z, and structural formula I in the reaction schemes and discussion that follow are as defined above.

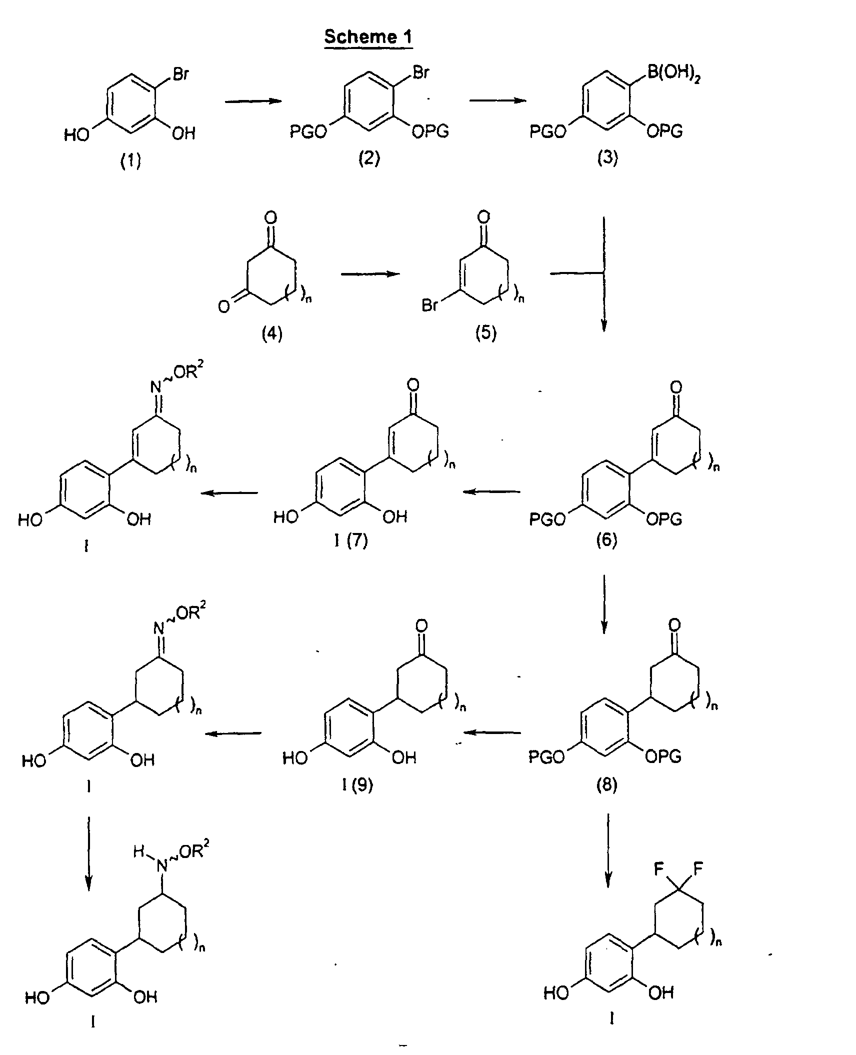

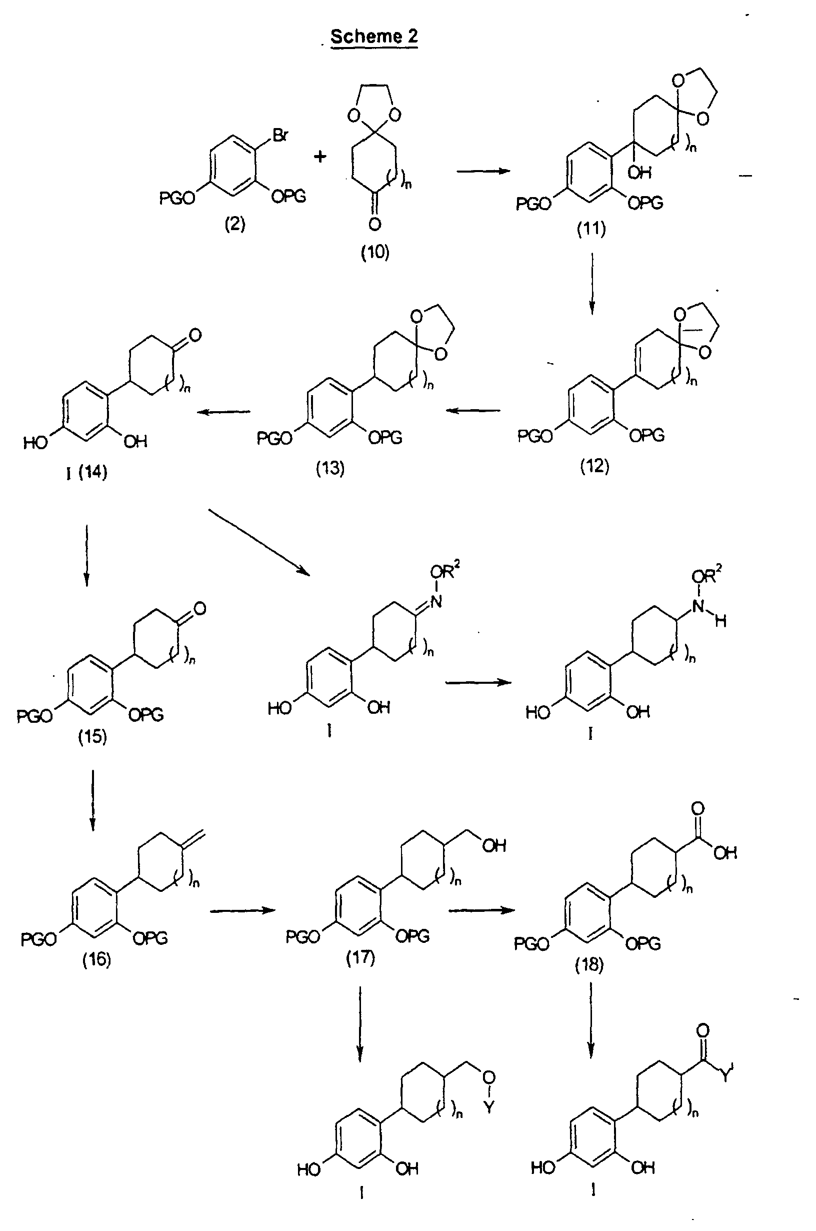

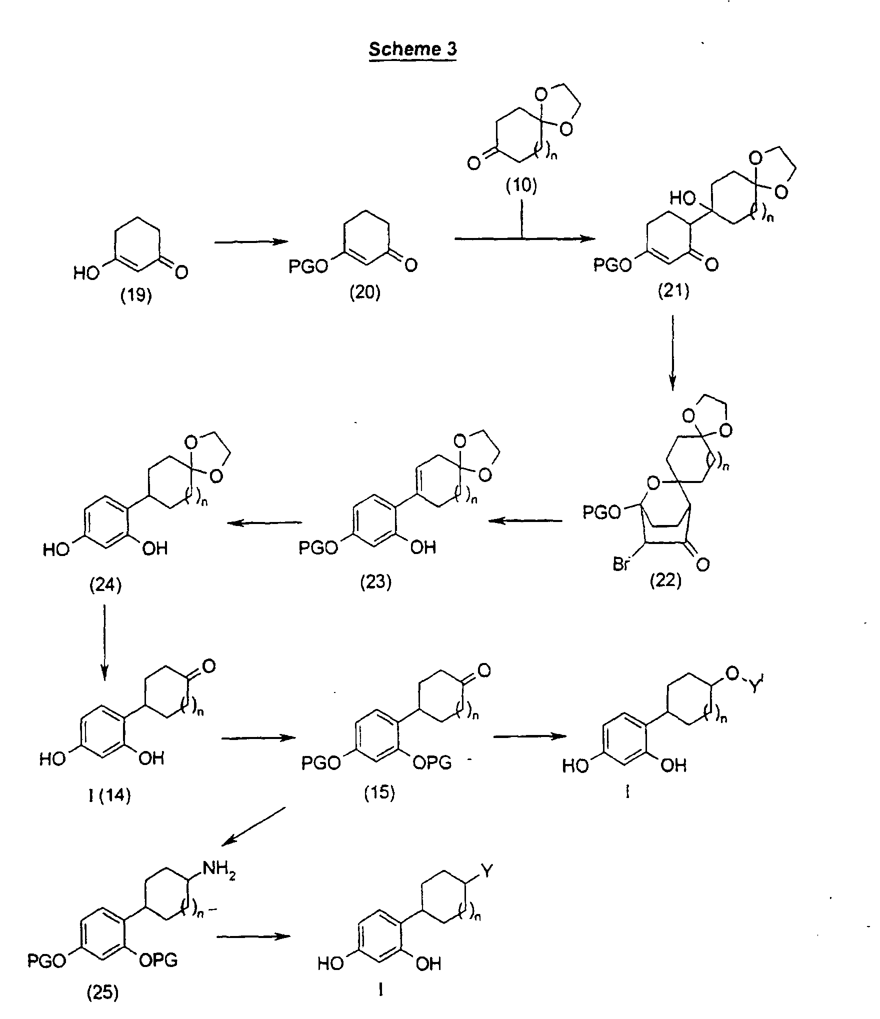

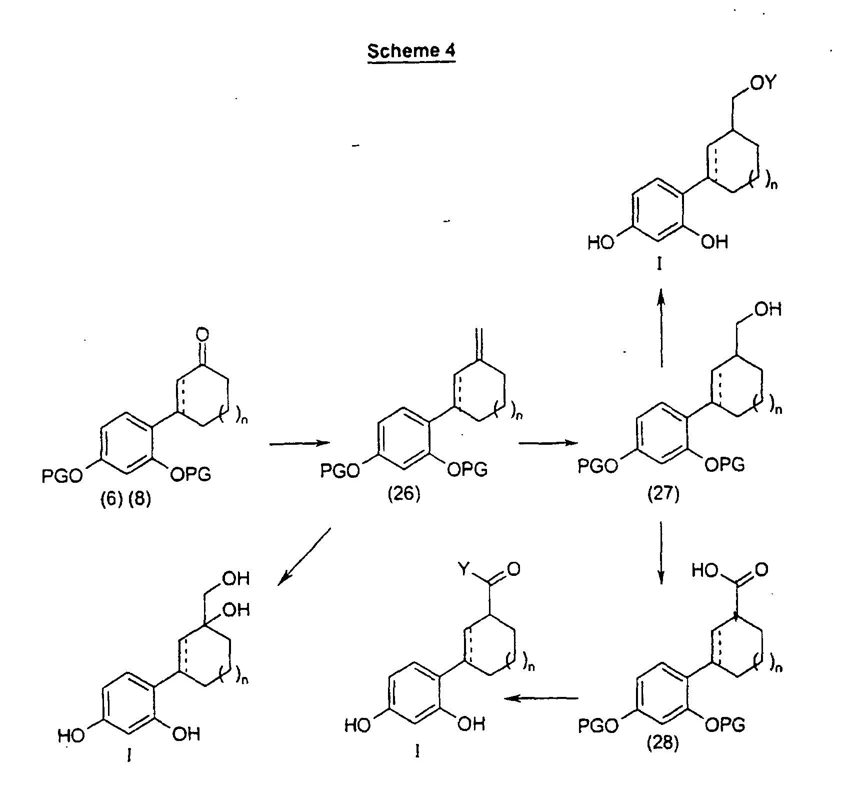

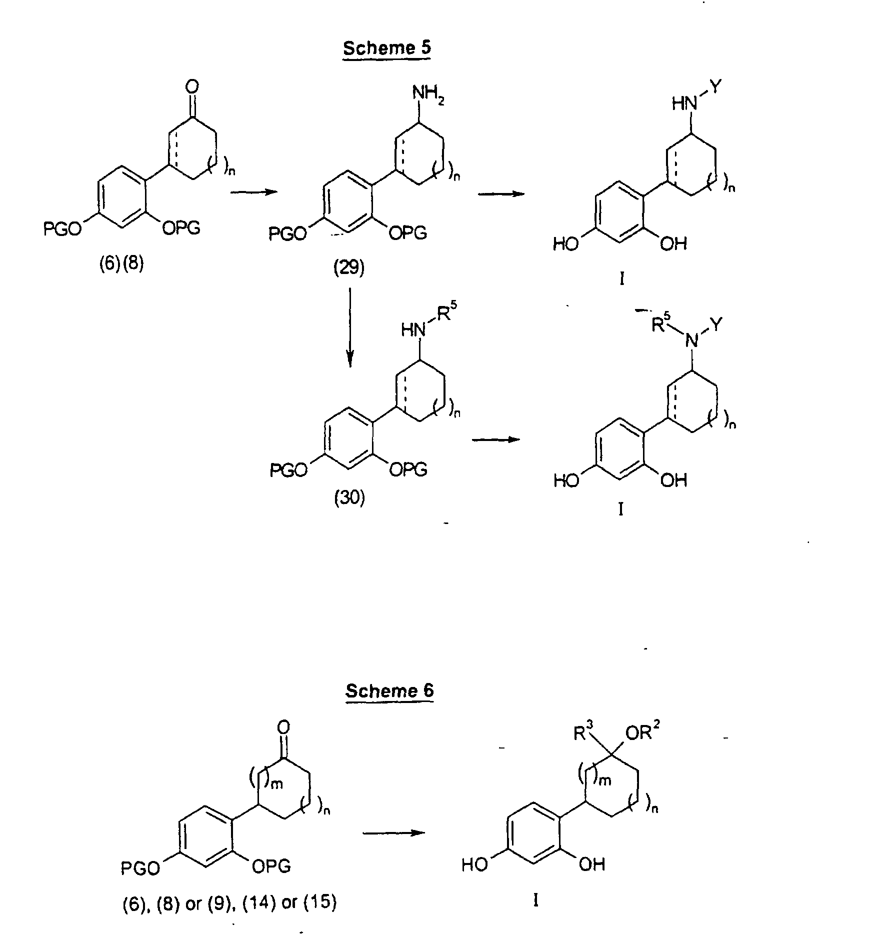

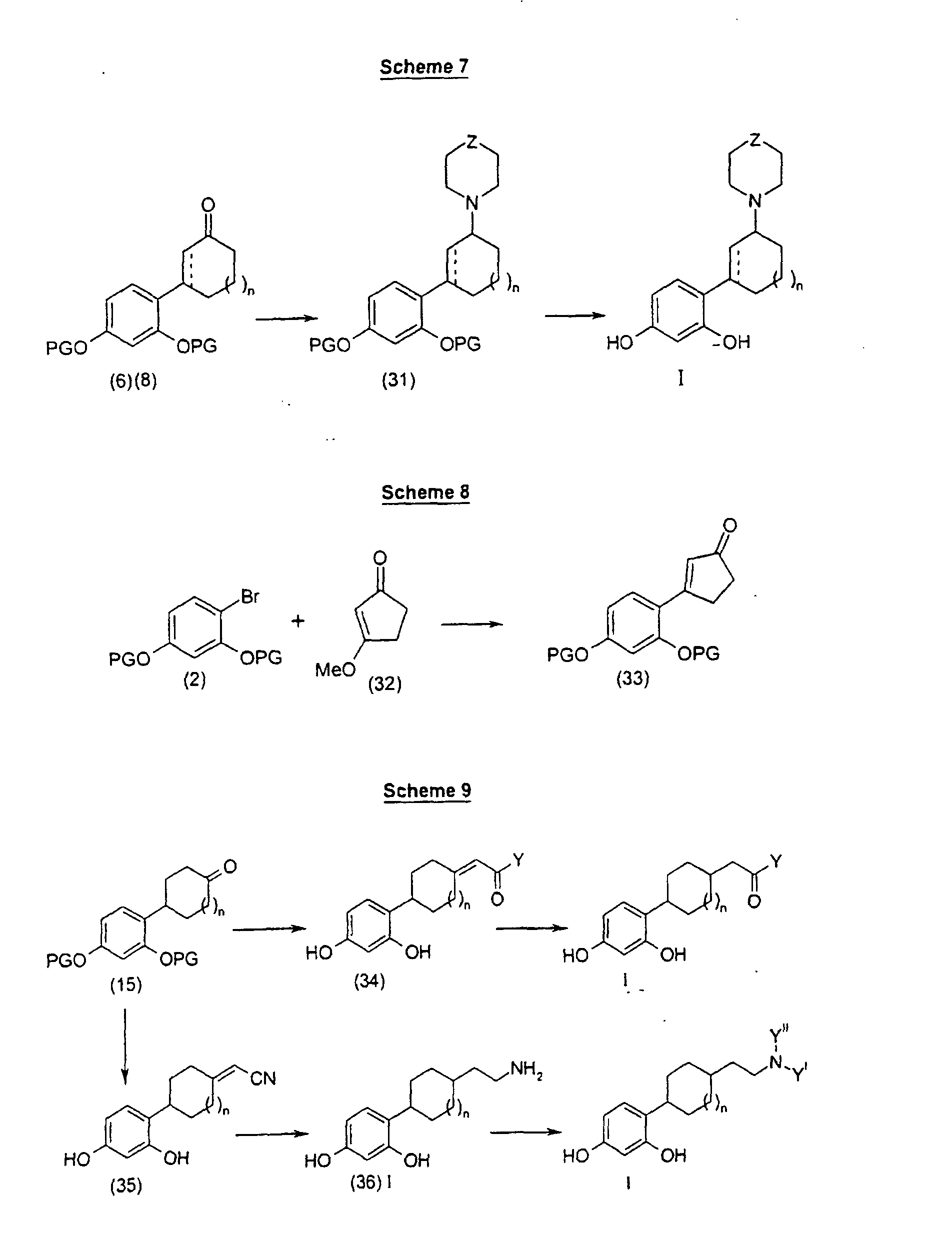

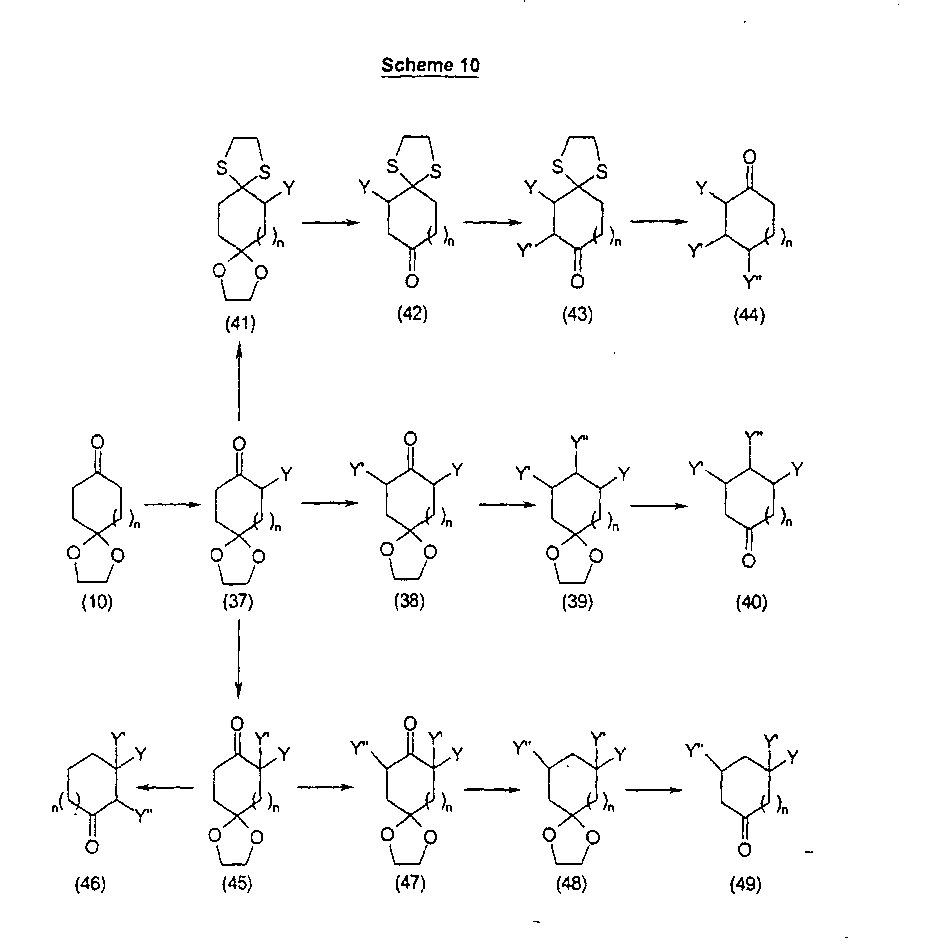

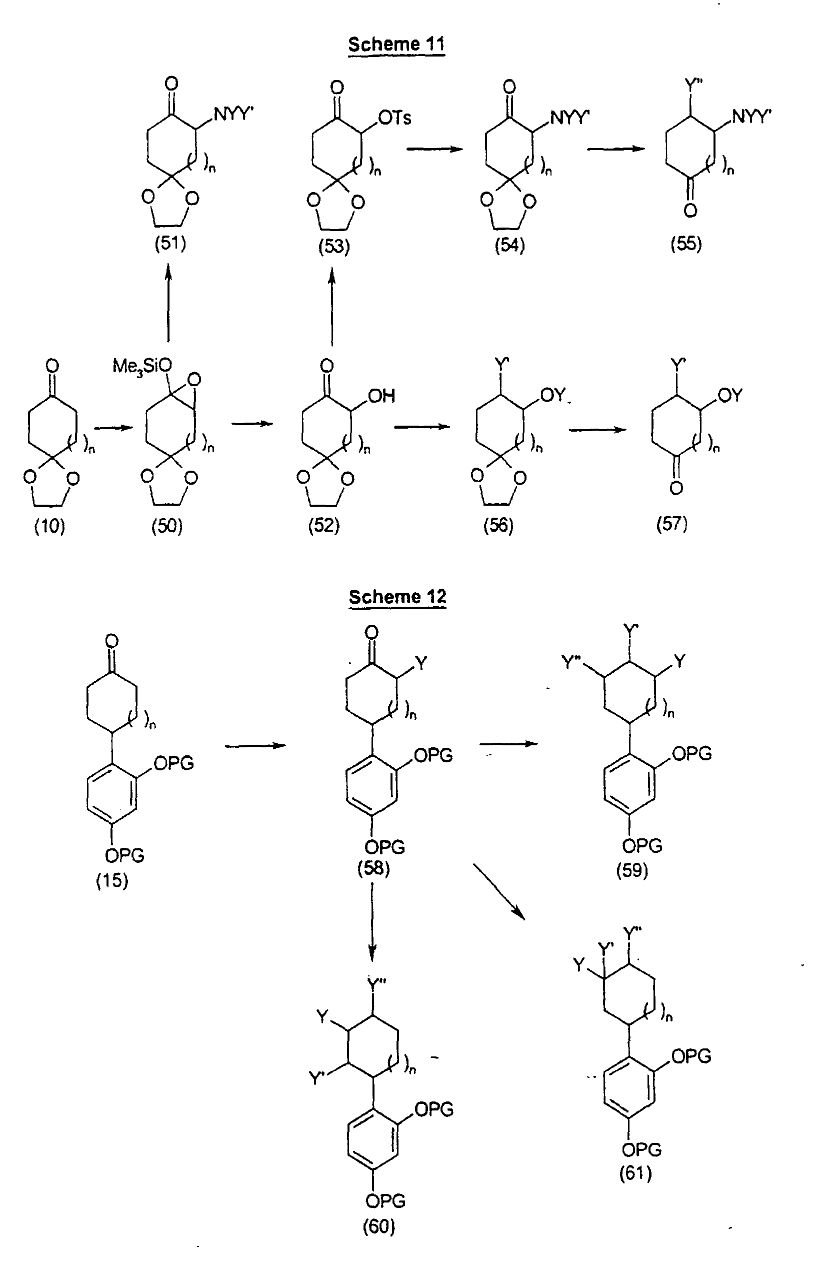

Y, Y^{I}, Y^{II} shown in the schemes above each independently represents any of the various substituents on R¹ as defined above, or hydrogen as appropriate.

Reaction Schemes 1 through 12 illustrate various methods of synthesizing compounds of formula I. PG refers to a protecting group.

Referring to Scheme 1, compounds of formula (**2**) can be formed by protecting commercially available 4-bromoresorcinol (**1**). A suitable protecting group such as methoxymethyl (MOM) can be introduced by conventional methods that are well known to those skilled in the art. For example, alkylation of 4-bromoresorcinol can occur with two equivalents of methoxymethyl chloride in the presence of diisopropylamine in a halogenated solvent at 0°C to room temperature.

Compounds of general formula (**3**) can be obtained using conventional methods. For example, the reaction of compounds of formula (**2**) with *n*-butyllithium in the presence of *N*,*N*,*N*',*N*'-tetramethyiethylenediamine in a suitable solvent such as tetrahydrofuran, followed by quenching with triisopropyl borate and hydrolysing with aqueous acid, can yield compounds of formula (**3**).

Compounds of general formula (**5**) can be obtained using conventional methods. For example, the treatment of compounds of formula (**4**) with triphenylphosphine and bromine in a chlorinated solvent will yield compounds of formula (**5**). Compound (4) where n=1 is commercially available (Aldrich, Milwaukee, WI, USA).

Compounds of formula (**6**) can be obtained by reacting compounds of formula (**3**) with compounds of formula (5) under Suzuki coupling conditions. For example, the Suzuki reaction can be carried out using palladium tetrakis (triphenylphosphine)palladium (five mole percent), sodium carbonate (two equivalents) and heating in a suitable solvent system (*e*.*g*., dimethoxyethane/water) at 80°C. Conversion of compounds of formula (**6**) to compounds of formula (**8**) can occur under standard reducing conditions, such as, *e*.*g*., hydrogen gas and a metal catalyst such as rhodium on alumina, at room temperature and atmospheric pressure. Deprotection of compounds of formula (**6**) or (**8**) under suitable conditions, *e*.*g*., where the protecting group is MOM, heating at 50°C in methanol with acidic Dowex, gives compounds of formula I where R¹ is substituted with =O (**7**), (**9**). Compounds of formula (**7**) or (**9**) can be further derivatised under standard conditions to yield resorcinols of formula I where R¹ is substituted with =NOR². For example, heating compounds of formula (**7**) in a suitable solvent (*e*.*g*., ethanol) at 80°C with the required hydroxylamine hydrochloride salt and triethylamine yields the analogues of formula I. Compounds of formula I where R¹ is substituted with NHOR² can be prepared from compounds of formula I where R¹ is substituted with =NOR² by a reduction under standard reaction conditions, *e*.*g*., sodium cyanoborohydride in acetic acid at or about room temperature.

Compounds of formula (8) can be further derivatised under standard conditions to yield resorcinols of formula I where R¹ is disubstituted with fluoro. For example, treating compounds of formula (**8**) with diethylaminosulfur trifluoride in a suitable solvent, *e.g*. dimethoxyethane, after suitable deprotection, would give analogues of formula I.

Referring to Scheme 2, compounds of the general formula (**10**) are well known and can be obtained using conventional methods (see, *e*.*g*., Crombie *et al*., 1982, J. Chem. Soc. Perkin Trans. I, 1485). Compounds of formula (**11**) can be obtained from the reaction of compounds of formula (**2**) with *n*-butyllithium in the presence of *N,N,N',N'*-tetramethylethylenediamine in an ethereal solvent, followed by the addition of a compound of formula (**10**). Dehydration of compounds of formula (**11**) under standard conditions, *e*.*g*., heating compounds of formula **(11)** at about 110°C in a Dean-Stark apparatus in the presence of camphor sulfonic acid in a suitable solvent (*e.g.*, toluene), yields compounds of formula (**12**). Hydrogenation under standard conditions, *e.g.*, using hydrogen gas and palladium on charcoal in ethanol, yields compounds of the general formula (**13**). Deprotection under suitable conditions yields resorcinols of formula I where R¹ is substituted with =O (**14**). Compounds of formula (**14**) can be further derivatised under standard conditions to yield resorcinols of formula I where R¹ is substituted with =NOR². For example, heating compounds of formula (**14**) in a suitable solvent (*e*.*g*., ethanol) at about 80°C with the required hydroxylamine hydrochloride salt and triethylamine yields analogues of formula I. Further reduction under standard conditions would yield compounds of formula I, where R¹ is substituted with NHOR². Compounds of formula (**14**) can also be reprotected with a suitable protecting group such as *tert*-butyldimethylsilyl under standard reaction conditions to yield compounds of formula (**15**).

Compounds of formula (**15**) can be further derivatised using standard reaction conditions. For example, methylenation using an appropriate Wittig will yield compounds of formula (**16**), *e*.*g*., treatment of methyltriphenylphosphonium bromide with potassium *t*-butoxide in a suitable solvent (*e*.*g*., tetrahydrofuran) at a temperature between -78°C and 0°C. followed by the addition of a compound of formula (**15**) will yield compounds of formula (**16**). Subsequent conversion to compounds of formula (**18**) under standard conditions, *e*.*g*. hydroboration, to give compounds of formula (**17**), and further oxidation using suitable conditions, such as pyridinium dichromate in dimethylformamide at room temperature, gives compounds of formula (**18**). Compounds of formula (**17**) can be treated with an alkyl bromide in a suitable solvent (*e.g.*, acetone) in the presence of potassium carbonate to yield compounds of formula I where R¹ is substituted with an ether group after suitable deprotection has taken place, *e.g*. when the protecting group is *tert*-butyldimethylsilyl, tetrabutylammonium fluoride in tetrahydrofuran can be used. Alternatively, compounds of formula (**17**) can also be esterified under standard conditions, e.g. treatment with an acid chloride in the presence of triethylamine in a chlorinated solvent at about room temperature. Compounds of formula (**18**) can be derivatised to form analogues such as esters and amides under conditions well known to those with skill in the art. For example, conditions to form amides may involve treating compounds of formula (**18)** with *iso*-butylchloroformate and triethylamine in a chlorinated solvent at about 0°C, followed by the addition of a suitable amine. Deprotection under suitable conditions will yield compounds of formula I where R¹ is substituted with an amide. Deprotection of compounds of formula (**16**), (**17**) and (**18**) under standard conditions also provides compounds of formula I where R' is substituted with methylene, hydroxylmethyl or a carboxylic acid, respectively.

Referring to Scheme 3, compounds of formula (**20**) can be prepared starting with compound (**19**), which is commercially available. Conversion to compounds of formula (**20**) can occur under standard conditions such as, for example, where the protecting group is benzyl, condensation can occur between compound (**19**) and benzyl alcohol with the removal of water using a Dean-Stark apparatus in conjunction with well known methodology. Condensation of compounds of formula (**20**) with compounds of formula (**10**) can occur using standard techniques, for example, treatment of compounds of formula (**20**) with a base such as lithium diisopropylamide in an ethereal solvent followed by the addition of a compound of formula (**10**) would give compounds of formula (**21**). Treatment of compounds of formula (**21**) with a suitable reagent such as N-bromosuccinimide in a chlorinated solvent at about room temperature can give compounds of formula (**22**). Compounds of formula (**23**) can then be generated from compounds of formula (**22**) under suitable conditions. Such conditions can involve treating compounds of formula (**22**) with a base such as 1,8-diazobicyclo[5.4.0]undec-7-ene in a suitable solvent such as *N,N*-dimethylformamide at 140°C. Treatment of compounds of formula (**23**) to standard hydrogenation conditions, *e*.*g*., hydrogen gas and palladium on charcoal in ethanol, yields compounds of the general formula (**24**) when the protecting group is benzyl. Compounds of formula (**14**) can then be obtained by treating compounds of formula (**24**) to acidic conditions.

Conversion of compounds of formula (**14**) to compounds of formula I may involve the need to use protecting groups that will be obvious to those of skill in the art. Some examples of such compounds of formula I are illustrated in Scheme 3. Conversion of compounds of formula (**15)** to compounds of formula I may involve the reduction of the ketone moiety under standard conditions, *e*.*g*., sodium borohydride in ethanol. Further derivitisation can occur, *e.g*. using chemistry described elsewhere in this document, to give compounds of formula I where Y^{I} may be alkyl, acyl or a carbonylamino. In examples where protecting groups have been used, suitable deprotection will be required to yield compounds of formula I.

Alternatively, compounds of formula (**15**) can be manipulated to give compounds of formula I where R¹ is substituted with an amide or sulfonamide. Treatment of compounds of formula (**15**) with benzylamine under reductive amination conditions, *e.g.*, one equivalent of sodium triacetoxyborohydride in a suitable solvent (dichloroethane) followed by hydrogenolysis under standard conditions, *e*.*g*., palladium on charcoal, hydrogen gas, ethanol, provides compounds of formula (**25**). Synthesis of compounds of formula I can be obtained using conventional methods. For example, compounds of formula (**25**) can react with sulfonyl chlorides and acid chlorides in a chlorinated solvent in the presence of a base (*e.g*. triethylamine) at room temperature. Deprotection using suitable reaction conditions provides compounds of formula I where Y is a sulfonamide or amide group.

Referring to Scheme 4, compounds of formula (**26**) can be synthesized using standard methods. For example, compounds of formula (**6**) or (**8**) can be homologated using a Wittig reaction and further manipulated as described above to yield compounds of formula I. Compounds of formula (**26**) or (**16**) from Scheme 2 can also undergo dihydroxylation under standard conditions, *e.g.* catalytic osmium tetroxide and N-methyl morpholine in an ethereal solvent, and after suitable deprotection yield compounds of formula I where R¹ is substituted with -(OH)(CH₂OH).

Referring to Scheme 5, compounds of formula **(29)** can be obtained by reacting compounds **(6)** or **(8)** as described above in Scheme 3. Compounds of formulae **(29)** and **(25)** can also be derivatised by treating with an alkylating agent, *e.g*., an alkyl iodide in a chlorinated solvent in the presence of triethylamine at room temperature, to give compound **(30)** prior to sulfonylation or amide bond formation and deprotection, to yield compounds of formula I wherein Y is -N(R⁵)SO₂R⁴ or equivalent amide where R⁵ is not hydrogen.

Referring to Scheme 6, compounds of formula (**6**), (**8**) or (**9**), (**14**) or (**15**) can be treated with a suitable organometallic reagent, such as a Grignard reagent, in an ethereal solvent at a temperature between -78°C and 0°C, followed by deprotection to yield compounds of formula I where R¹ is substituted with (R²)OR².

Referring to Scheme 7, compounds of formula (**31**) can be formed under conditions well known to those skilled in the art. Treatment of compounds (**6**) or (**8**) with an amine, such as piperidine, in a suitable solvent (*e*.*g*., dichloroethane) and a reducing agent such as sodium triacetoxyborohydride, followed by deprotection will yield compounds of formula I where R¹ is substituted with a nitrogen-containing heterocycle.

Referring to Scheme 8, compounds of general formula (**33**) can be obtained using conventional methods. For example, the reaction of compounds of formula (**2**) with *n*-butyllithium in the presence of *N,N,N',N'*-tetramethylethylenediamine in a suitable solvent such as tetrahydrofuran, followed by quenching with ketone (**32**) (commercially available from Aldrich), and hydrolysing with aqueous acid, can yield compounds of formula (**33**). Functional group manipulation as outlined in Schemes 1-7 and Scheme 8 then allows the synthesis of compounds of formula I.

Referring to scheme 9, compounds of formula (**15**), (**6**) or (**8**) can be further derivatised to yield compounds of formula (**34**) using standard Wittig or Wadworths-Emmons chemistry, followed by suitable deprotection. Compounds of formula (**34**) can be reduced using standard hydrogenation as described above to yield compounds where Y is OH, O-alkyl, or an aminoalkyl. Standard Wadworths-Emmons chemistry can also yield compounds of formula (**35**). Reduction under suitable conditions will yield compounds of formula (**36**), which can be further derivatised using standard chemistry described previously in this document to give compounds where NY^{I}Y^{II} is an amide, sulfonamide, or aminoalkyl.

Referring to Scheme 10, compounds of formula (**10**) can be converted into compounds of formula (**37**) using standard alkylation procedures. For example, compound (10) can be treated with a suitable base such as lithium diisopropyl amide in a suitable solvent such as tetrahydrofuran at a temperature between -78°C and 0°C, followed by the addition of a suitable alkylating agent. Such alkylating agents are well known to those of skill in the art, and can include chloro, bromo, or iodoalkyl compounds; epoxides; aldehydes; aziridines; α,β-unsaturated esters, ketones or amides; acyl chlorides; electrophilic sources of oxygen such as Mo(CO)₅.pyridine (Crimmons, M. T. *et al*., 1992, *J. Am. Chem. Soc*., 114:5445); or electrophilic sources of nitrogen such as 2,4,6-triisopropylbenzene sulfonyl azide (Evans. P. A. *et al*., 1992, Tetrahedron Lett. 33:6959). Such alkylating reagents are commercially available or can be prepared by standard procedures well known to those of skill in the art.

Compounds of formula (**37**) can be further manipulated using methodology similar to that described above. For instance, alkylation of compounds of formula (**37**) under conditions of kinetic deprotonation (see, *e*.*g*., Kopka, I. and Rathke, M. W., 1981, *J. Org. Chem.* 46:3771), followed by alkylation as previously described, would yield compounds of formula (**38**). Alternatively, alkylation of compounds of formula (**37**) under conditions of thermodynamic deprotonation (Kopka and Rathke, 1981, above), followed by alkylation as previously described, would yield compounds of formula (**45**). Further alkylation or functional group manipulation known to those of skill in the art and, as described elsewhere in this document, followed by removal of the carbonyl protecting group under standard conditions, *e*.*g*., aqueous hydrochloric acid at 0°C to 50°C, would yield compounds of formulae (**40**), (**46**), and (**49**).

Compounds of formula (**37**) can also be protected with a suitable protecting group, *e*.*g*., ethylenedithio ketal, and after removal of the ketal protecting group under standard reaction conditions, e.g., aqueous hydrochloric acid at 0°C to 50°C, would allow further functionalisation of the cycloalkyl ring using the methodology described above to yield compounds of formula (**44**).

Referring to Scheme 11, compounds of formula (**50**) can be formed from compounds of formula (**10**) using known methodology (see, *e*.*g*., Adam, W. *et al*., 1989, *Tetrahedron Lett.* 30:6497) (Ts = tosyl). Standard functional group manipulation yields compounds of formula (**55**) and (**57**). Oxirane ring cleavage by an amine yields compounds of formula (**51**). Alternatively, acid hydrolysis yields compounds of formula (**52**). Conversion of the corresponding alcohol to a leaving group such as para-toluenesulfonyl would allow the nucleophilic displacement with a range of nucleophiles under standard, well-known conditions. Such nucleophiles may include amines, thiolates, alkoxides, and carbon-based nucleophiles such as cyanide, which are commercially available or prepared by standard procedures well known to those of skill in the art.

Compounds of formula (**40**), (**44**) and (**49**) can be prepared using the above-described methods or other methods known in the art such that substituents on R¹ are as defined above.

Referring to Schemes 10 and 11, ketones of formulae (**37**), (**38**), (**40**), (**42**), (**43**), (**44**), (**45**), (**46**), (**47**), (**49**), (**51**), (**54**), (**55**), and (**57**) can be further manipulated as described in Schemes 1-9 above, and converted into compounds of formula I as described in Schemes 2 and 3.

Referring to Scheme 12, compounds of formula I can also be prepared using the methodology described above. Compounds of formula (**58**) can be converted into compounds of formula I using the chemistry as described in Schemes 1-11 above.

It will be appreciated by those of skill in the art that in the processes described above, the functional groups of intermediate compounds may need to be protected by protecting groups. The use of protecting groups is well-known in the art, and is fully described, among other places, in: Protecting Groups in Organic Chemistry, J. W. F. McOmie, (ed.), 1973, Plenum Press; and in: Protecting Groups in Organic Synthesis, 2^{nd} edition, T. W. Greene & P. G. M. Wutz, 1991, Wiley-Interscience.

The compounds of formula I that are basic in nature are capable of forming a wide variety of different salts with various inorganic and organic acids. Although such salts must be pharmaceutically acceptable for administration to animals, it is often desirable in practice to initially isolate a compound of formula I from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the active base compounds of this invention are readily prepared by treating the base compound with a substantially equivalent amount of the chosen mineral or organic acid in an aqueous solvent medium or in a suitable organic solvent, such as methanol or ethanol. Upon careful evaporation of the solvent, the desired solid salt is readily obtained.

Those compounds of formula I that are acidic in nature are capable of forming base salts with various pharmaceutically acceptable cations. Examples of such salts indude the alkali metal and alkaline earth metal salts and, particularly, the sodium and potassium salts. These salts can be prepared by conventional techniques. The chemical bases that are used as reagents to prepare the pharmaceutically acceptable base salts of this invention are those that form non-toxic base salts with the acidic compounds of formula I. Such non-toxic base salts include those derived from such pharmaceutically acceptable cations as sodium, potassium, calcium and magnesium, etc. These salts can easily be prepared by treating the corresponding acidic compounds with an aqueous solution containing the desired pharmaceutically acceptable cations, and then evaporating the resulting solution to dryness, preferably under reduced pressure. Alternatively, they can also be prepared by mixing lower alkanolic solutions of the acidic compounds and the desired alkali metal alkoxide together, and then evaporating the resulting solution to dryness, as described above. In either case, stoichiometric quantities of reagents are preferably employed in order to ensure completeness of reaction and maximum yields of the desired final products.

Compounds of formula I and their pharmaceutically acceptable salts (hereinafter "the active compounds used in this invention") are useful in the preparation of medicaments useful in the treatment of disorders of human pigmentation, including solar and simple lentigines (including age/liver spots), melasma/chloasma and postinflammatory hyperpigmentation. Such compounds reduce skin melanin levels by inhibiting the production of melanin, whether the latter is produced constitutively or in response to UV irradiation (such as sun exposure). Thus, the active compounds used in this invention can be used to reduce skin melanin content in non-pathological states so as to induce a lighter skin tone, as desired by the user, or to prevent melanin accumulation in skin that has been exposed to UV irradiation. They can also be used in combination with skin peeling agents (including glycolic acid or trichloroacetic acid face peels) to lighten skin tone and prevent repigmentation. The appropriate dose regimen, the amount of each dose administered, and specific intervals between doses of the active compound will depend upon the particular active compound employed, the condition of the patient being treated, and the nature and severity of the disorder or condition being treated. Preferably, the active compound is administered in an amount and at an interval that results in the desired treatment of or improvement in the disorder or condition being treated.

An active compound used in the present invention can also be used in combination with sun screens (UVA or UVB blockers) to prevent repigmentation, to protect against sun or UV-induced skin darkening or to enhance their ability to reduce skin melanin and their skin bleaching action. An active compound used in the present invention can also be used in combination with retinoic acid or its derivatives or any compounds that interact with retinoic acid receptors and accelerate or enhance the invention's ability to reduce skin melanin and skin bleaching action, or enhance the invention's ability to prevent the accumulation of skin melanin. An active compound used in the present invention can also be used in combination with 4-hydroxyanisole.

The active compounds used in this invention can also be used in combination with ascorbic acid, its derivatives and ascorbic-acid based products (such as magnesium ascorbate) or other products with an anti-oxidant mechanism (such as resveratrol) which accelerate or enhance their ability to reduce skin melanin and their skin bleaching action.

This invention relates both to cosmetic methods of lightening or reducing the pigmentation of skin in which the compound of formula I, or pharmaceutically acceptable salt thereof, and one or more of the other active ingredients referred to above are administered together, as part of the same pharmaceutical composition, as well as methods in which they are administered separately as part of an appropriate dose regimen designed to obtain the benefits of the combination therapy. The appropriate dose regimen, the amount of each dose administered, and specific intervals between doses of each active agent will depend upon the specific combination of active agents employed, the condition of the patient being treated, and the nature and severity of the disorder or condition being treated. Such additional active ingredients will generally be administered in amounts less than or equal to those for which they are effective as single topical therapeutic agents. The FDA approved dosages for such active agents that have received FDA approval for administration to humans are publicly available.

The active compounds of the present invention are generally administered in the form of pharmaceutical compositions comprising at least one of the compounds of formula (I), together with a pharmaceutically acceptable vehicle or diluent. Such compositions are generally formulated in a conventional manner utilizing solid or liquid vehicles or diluents as appropriate for topical administration, in the form of solutions, gels, creams, jellies, pastes, lotions, ointments, salves and aerosols.

Examples of vehicles for application of the active compounds of this invention include an aqueous or water-alcohol solution, an emulsion of the oil-in-water or water-in-oil type, an emulsified gel, or a two-phase system. Preferably, the compositions according to the invention are in the form of lotions, creams, milks, gels, masks, microspheres or nanospheres, or vesicular dispersions. In the case of vesicular dispersions, the lipids of which the vesicles are made can be of the ionic or nonionic type, or a mixture thereof.

As used herein, a "skin-lightening or pigmentation reducing amount of a compound of formula I", means an amount or concentration of the compound capable of detectably lightening skin or reducing pigmentation in a human, as determined by any standard assay. The active compound is typically administered in a pharmaceutical composition and for a standard course of treatment that produces the desired result of skin depigmentation.

As used herein, a "tyrosinase-inhibiting effective amount of a compound of formula I", means an amount or concentration of the compound capable of detectably inhibiting tyrosinase activity in a human, as determined by any standard assay.

As used herein, an "amount of a compound of formula I capable of treating an inflammatory disorder such as psoriasis, dermatitis or acne, or treating dandruff", means an amount or concentration of the compound capable of detectably ameliorating, reducing, eliminating, slowing, or preventing the progression of, any symptom or condition associated with or caused by such disorder or condition, in a human, as determined by any standard assay.

In the depigmenting compositions according to the present invention, the concentration of the active compound of the invention is generally between 0.01 and 10%. preferably between 0.1 and 10%, relative to the total weight of the composition.

The compositions of this invention can optionally also contain a moistener, a surfactant, keratolytic, an anti-inflammatory agent, a complexing agent, an antioxidant, a preservative, a fragrance, or a sunscreen.

The ability of compounds of formula I to inhibit tyrosinase may be determined using any of the following procedures.

### 1. Tyrosinase (DOPA oxidase) assay using cell lysate:

Human melanoma cell line, SKMEL 188 (licensed from Memorial Sloan-Kettering), is used in the cell lysate assay and the screen. In the assay, compounds and L-dihydroxyphenytalanine (L-DOPA) (100 µg/ml) are incubated with the cell lysates containing human tyrosinase for 8 hrs before the plates are read at 405 nm. Potency of the compounds in DOPA oxidase assay is correlated very well with that in tyrosine hydroxylase assay using ³H-tyrosine as a substrate. Most of the compounds of formula I that were tested in this assay exhibited an IC₅₀ of less than 10 µM. For example, the compound of Example 23, *i*.*e*., (±)-3-(2,4-Dihydroxyphenyl)cyclopentanone oxime, had an IC₅₀ in this assay of 2 µm.

### 2. Melanin assay in human primary melanocytes:

Compounds are incubated with human primary melanocytes in the presence of α-melanocyte stimulating hormone (α-MSH) for 2-3 days. Cells are then lysed with sodium hydroxide and sodium dodecyl sulfate (SDS) and melanin signals are read at 405 nm. Alternatively, ¹⁴C-DOPA is added to the cells in combination with tyrosinase inhibitors and acid-insoluble ¹⁴C-melanin is quantitated by a scintillation counter. IC₅₀'s reflect the inhibitory potency of the compounds in the new melanin synthesis that was stimulated by α-MSH.

### 3. Tyrosine kinase assay (TK):

TK assays can be performed using purified tyrosine kinase domains of c-met, erb-B2, or IGF-r. A specific antibody against phosphorylated tyrosine residue is used in the assay. Colorimetric signals are generated by horse radish peroxidase, which is conjugated to the antibody.

### 4. Human skin equivalent model:

A mixture of human melanocytes and keratinocytes is grown in an air-liquid interphase. This tissue culture forms a three dimensional structure that histologically and microscopically resembles the human skin epidermis. Test compounds are added on top of the cells to mimic topical drug application. After incubation with the compounds (10 µM) for 3 days, the cells are washed extensively and lysed for DOPA oxidase assay.

### 5. IL-1 assay (Interleukin-1 assay):

An IL-1α ELISA assay (R&D system) can be used to evaluate the effect of compounds on IL-1 secretion in a human skin equivalent model. IL-1α is a pro-inflammatory cytokine and plays a role in UV-induced skin inflammation.

### 6. In vivo study:

Black or dark brown guinea pigs with homogeneous skin color can be used in this study. A solution of the test compound of formula I (5% in ethanol:propylene glycol, 70:30) and the vehicle control are applied to the animals twice daily, 5 days per week for 4-8 weeks. Using this assay, depigmentation can be determined by subtracting the light reflectance of untreated skin from the light reflectance of treated skin.

The present invention is illustrated by the following examples. It will be understood, however, that the invention is not limited to the specific details of these examples. Melting points are uncorrected. Proton nuclear magnetic resonance spectra (400 MHz ¹H NMR) were measured for solutions in d₆-DMSO, CDCl₃, or d₄-MeOH, and peak positions are expressed in parts per million (ppm) downfield from tetramethylsilane (TMS). The peak shapes are denoted as follows: s, singlet; d, doublet; t, triplet; q, quartet, m, multiplet, b, broad.

The following examples are illustrative only, and are not intended to limit the scope of the present invention.

### EXAMPLES

### Intermediate 1

### 1-Bromo-2,4-bis(methoxymethoxy)benzene

An oven dried 250 ml round bottomed flask equipped with magnetic stirrer, under an argon atmosphere, was loaded with 4-bromoresorcinol (9.45 g, 50 mmol) and CH₂Cl₂ (50 ml). The stirred suspension was cooled to 0°C and diisopropylamine (19.1 ml, 110 mmol) was added in one portion via syringe. Stirring of the red solution was continued for a further ten minutes before methyl chloromethyl ether (10.7 ml, 120 mmol) was added dropwise, *via* syringe, ensuring the internal temperature did not exceed 10°C. The resulting yellow solution was then allowed to warm to room temperature overnight. Ammonium hydroxide solution (50 mL, 50%) was poured into the reaction vessel and stirring was continued for one hr. The mixture was poured into a separating funnel and the phases separated. The aqueous phase was then extracted with CH₂Cl₂ (3x30 ml) and the combined organics washed with brine (20 ml), dried over anhydrous magnesium sulphate, filtered and concentrated *in vacuo* affording an orange oil. Purification was achieved by flash column chromatography, (SiO₂, ethyl acetate/petroleum ether, 1:1, v/v), furnishing the title product (10.7 g, 77%) as a pale yellow oil. δ_{H}(CDCl₃) 7.42 (1H, d), 6.88 (1H, d), 6.64 (1H, dd), 5.24 (2H, s), 5.15 (2H, s), 3.53 (3H, s), 3.48 (3H, s).

### Intermediate 2

### 8-[2,4-Bis(methoxymethoxy)phenyl]-1,4-dioxaspiro[4.5]decan-8-ol

A round bottomed flask, equipped with magnetic stirrer, under an argon atmosphere was loaded with 1-bromo-2,4-*bis*(methoxymethoxy)benzene (2.00 g, 7.2 mmol) and THF (50 mL). *N, N, N', N'-* Tetramethylethylene diamine (2.3 ml, 15.2 mmol) was added in one portion *via* syringe and the stirred solution was cooled to -78°C. *n*-Butyl lithium (9.5 ml, 15.2 mmol, 1.6M in hexane) was added dropwise via syringe. The resulting yellow solution was stirred for 1 hr at -78°C and 1,4-cyclohexanedione monoethylene ketal (1.35 g, 8.7 mmol) was added as a solution in THF (25 ml) slowly, *via* syringe. The resulting solution was stirred at -78°C for 1 hr and then allowed to warm to room temperature overnight. Hydrochloric acid (20 ml, 2M) was added and the reaction mixture stirred vigorously for 15 min. Ethyl acetate (100 ml) was added and the mixture poured into a separating funnel. The phases were separated and the aqueous phase was extracted with ethyl acetate (3x20 ml). The combined organics were washed with brine (20 ml), dried over anhydrous magnesium sulphate, filtered and concentrated affording an orange oil which was purified by flash column chromatography (SiO₂, ethyl acetate/petroleum ether, 45:55, v/v). The title product (1.42 g, 56%) was isolated as a colourless oil. m/z (ES⁺) 337 (M - H₂O + H⁺); δ_{H} (CDCl₃) 1.61 - 1.64(2H, m), 2.00 - 2.18(6H, m), 3.44(3H, s), 3.48(3H, s), 3.90 - 3.97(4H, m), 5.11(2H, s), 5.24(2H, s), 6.64(1H, dd), 6.82(1H, d), 7.20(1H, d).

### Intermediate 3

### 8-[2,4-Bis(methoxymethoxy)phenyl]-1,4-dioxaspiro[4.5]dec-7-ene

8-[2,4-*Bis*(methoxymethoxy)phenyl]-1,4-dioxaspiro[4.5]decan-8-ol (1.40 g, 3.95 mmol) was placed in a 50ml round bottomed flask equipped with magnetic stirrer and Dean-Stark apparatus. Toluene (30 ml) was added, followed by camphor sulphonic acid (10 mg). The stirred solution was then heated under reflux for 1 hr, cooled and saturated aqueous sodium bicarbonate solution (10 ml) added. The mixture was poured into a separating funnel and the phases separated. The aqueous phase was extracted with ethyl acetate (2x15 ml) and the combined organics were washed with brine (15 ml), dried over anhydrous magnesium sulphate, filtered and then concentrated *in vacuo* yielding an orange oil which was purified by flash column chromatography (SiO₂, ethyl acetate/petroleum ether, 45:55, v/v) to afford the title product (0.94 g) as a colourless oil. δ_{H} (CDCl₃) 1.84 (2H, t), 2.41 - 2.43 (2H, m), 2.56 - 2.62 (2H, m), 3.47 (6H, s), 3.98 - 4.02 (4H, m), 5.13 (4H, s), 5.58 - 5.63 (1H, m), 6.64 (1H, dd), 6.78 (1 H, d), 7.08 (1H, d).

### Intermediate 4

### 8-[2,4-Bis(methoxymethoxy)phenyl]-1,4-dioxaspiro[4.5]decane

8-[2,4-*Bis*(methoxymethoxy)phenyl]-1,4-dioxaspiro[4.5]dec-7-ene (0.950 g, 2.83 mmol) and palladium (200 mg, 10% on carbon) were stirred under an atmosphere of hydrogen for 15hr. The mixture was then filtered through a plug of Celite, washing with ethyl acetate. The filtrate was then evaporated to dryness, affording the desired product (0.955 g, 100%) as a colourless oil. δ_{H} (CDCl₃) 1.67 - 1.87 (8H, m), 2.90 - 2.99 (1H, m), 3.46 (3H, s), 3.48 (3H, s), 3.97 (4H, s), 5.12 (2H, s), 5.18 (2H, s), 6.65 (1 H, dd), 6.78 (1H, d), 7.12 (1H, d).

### Intermediate 5

### 4 - [2, 4 - Bis(methoxymethoxy)phenyl]cyclohexanone

A round bottomed flask equipped with magnetic stirrer was charged with 8 - [2, 4 - *bis*(methoxymethoxy)phenyl] - 1, 4 - dioxaspiro[4.5]decane (3.20 g 9.47 mmol) and methanol (50 ml). Over a 20min period, aqueous hydrochloric acid (50 ml, 1.00M) was added to the stirred solution, at room temperature and the reaction mixture stirred for 1.5 hr. Solid sodium bicarbonate was added until the reaction mixture was neutralised and the solvent was removed under reduced pressure. The residue was partitioned between ethyl acetate (30 ml) and water (10 ml), and the aqueous layer was extracted with ethyl acetate (3x20 ml). The combined organic layers were washed with brine (10 ml), dried over magnesium sulfate, filtered and concentrated *in vacuo.* The crude product was purified *via* flash column chromatography (SiO₂, ethyl acetate / petroleum ether, 1:4, v/v), affording the title compound (2.20 g, 60%) as a white powder. δ_{H}(CDCl₃) 1.85 - 1.96 (2H, m), 2.14 - 2.22 (2H, m), 2.46 - 2.59 (4H, m), 3.39 (1H, tt), 3.49 (3H, s), 3.52 (3H, s), 5.16 (2H, s), 5.23 (2H, s), 6.67 - 6.71 (1H, m), 6.85 (1 H, m), 7.08 (1H, d).

### Intermediate 6

### 3-[2,4-bis(methoxymethoxy)phenyl]-2-cyclohexen-1-one

Aqueous sodium carbonate (2 ml of a 6M solution) and 2,4-bis(methoxymethoxy)phenylboronic acid (120 mg) in ethanol (2 ml) were added to a solution of palladium tetrakis(triphenylphosphine) (57 mg) and 3-bromo-2-cyclohexen-1-one (87 mg) in dimethoxyethane (3 ml) and the mixture was heated under reflux. After 6 hr, the mixture was partitioned between water (50 ml) and ethyl acetate (100 ml). The organic layer was dried over magnesium sulfate and evaporated *in vacuo* to furnish an oil that was purified by flash column chromatography (SiO₂, ethyl acetate/petroleum ether, 1:3, v/v) to furnish the title compound as an oil (120 mg, 83%). δ_{H} (CDCl₃) 2.10 (2H, quintet), 2.47 (2H, t), 2.74 (2H, m), 3.476 (3H, s), 3.484 (3H, s), 5.185 (2H, s), 5.190 (2H, s), 6.21 (1H, m), 6.71 (1H, dd), 6.85 (1H, d), 7.16 (1H, d).

### Intermediate 7

### (±)-3-[2,4-Bis(methoxymethoxy)phenyl]cyclohexanone

A suspension of 3-[2,4-*bis*(methoxymethoxy)phenyl]-2-cyclohexen-1-one (300 m g) and palladium catalyst (50 mg, 10% palladium on carbon) in ethanol was stirred at ambient temperature under 1 atmosphere of hydrogen. After 16 hr, the mixture was filtered through celite and the filtrate was evaporated *in vacuo.* The product was dissolved in dichloromethane (15 ml). Celite and pyridinium chlorochromate (430 mg) were added and the mixture was stirred at room temperature. After 3 hr, the mixture was filtered through a pad of silica and eluted with petroleum ether/ethyl acetate (10:3, v/v), then purified by flash column chromatography (SiO₂, petroleum ether/ethyl acetate 4:1 v/v) to furnish the title compound as an oil (200 mg, 70%). δ_{H} (CDCl₃) 1.7-1.9 (2H, overlapping m), 2.05 (1H, m), 2.15 (1H, m), 2.35-2.60 (4H, overlapping m), 3.37 (1H, m), 3.490 (3H,-s), 3.492 (3H, s), 5.15 (2H, s), 5.20 (2H, s), 6.70 (1H, dd), 6.82 (1H, d), 7.09 (1H, d).

### Intermediate 8

### 3-(2,4-Dimethoxymethoxyphenyl)-2-cyclohexen-1-one oxime

3-[2,4-*Bis*(methoxymethoxy)phenyl]-2-cyclohexen-1-one (200 mg), hydroxylamine hydrochloride (72 mg) and triethylamine (0.14 ml) were heated under reflux in ethanol (10 ml). After 3 hr, the cooled reaction mixture was partitioned between water and ethyl acetate. The organic layer was dried over magnesium sulfate and evaporated *in vacuo* to furnish the title compound as an oil (206 mg, 99%). m/z (ES⁺) 308 (M+H)⁺.

### Intermediate 9

### (±)-1-{3-[2,4-Bis(methoxymethoxy)phenyl]cyclohexyl}piperazine

(±)-3-[2,4-*Bis*(methoxymethoxy)phenyl]cyclohexanone (80 mg) and piperazine (24 mg) were dissolved in dichloroethane (5 ml) and stirred at ambient temperature for 1 hr under argon. Tetramethylammonium triacetoxyborohydride (79 mg) was added and stirring continued under argon. After 16 hr, additional portions of piperazine (24 mg) and tetramethylammonium triacetoxyborohydride (79 mg) were added and stirring continued. After a further 6 hr, glacial acetic acid was added dropwise until a solution was obtained, and stirring continued at ambient temperature. After a further 16 hr, the reaction mixture was partitioned between sodium hydrogen carbonate (20 ml of a saturated solution) and ethyl acetate. The aqueous layer was extracted with ethyl acetate (3x20 ml), and the combined organic extracts were dried over magnesium sulfate and evaporated *in vacuo.* The crude residue was purified by flash column chromatography chromatography (SiO₂, dichloromethane/methanol, 9:1 v/v) to furnish the title compound as an off white solid (52 mg, 53%) and mixture of diastereoisomers; m/z (ES⁺) 365 (M+H)⁺.

### Intermediate 10

### (±)-3-[2,4-Bis(methoxymethoxy)phenyl]cyclohexylamine

Nickel chloride hexahydrate (77 mg) and sodium borohydride (24 mg) were added to a stirred solution of (±)-3-[2,4-*bis*(methoxymethoxy)phenyl]cyclohexanone oxime (50 mg) in methanol (2 ml). After 0.5 hr, water was added until effervescence ceased, the reaction mixture was filtered, and the residue was washed thoroughly with methanol. The combined filtrate and washings were evaporated *in vacuo* and the crude residue was purified by flash column chromatography (SiO₂, dichloromethane/methanol, 9:1 v/v) to furnish the title compound as a colourless oil (31 mg, 65%) and a mixture of diastereoisomers; m/z (ES⁺) 295 (M+H)⁺.

### Intermediate 11

### (±)-N-{3-[2,4-Bis(methoxymethoxy)phenyl]cyclohexyl}methanesulfonamide

Triethylamine (0.014 ml) and methanesulfonyl chloride (8 µl) were added to a solution of (±)-3-[2,4-*bis*(methoxymethoxy)phenyl]cyclohexylamine (27 mg) in dichloromethane (1 ml), and the mixture was stirred under argon at ambient temperature. After 1 hr, the mixture was partitioned between ethyl acetate (20 ml) and sodium hydrogen carbonate (20 ml of a saturated aqueous solution). The aqueous layer was extracted with ethyl acetate (2x20 ml), and the combined organic extracts were dried over magnesium sulfate and evaporated in *vacuo* to furnish the title compound as an oil and a mixture of diastereoisomers; m/z (ES⁺) 374 (M+H)⁺.

### Intermediate 12

### (±)-2,4-Bis(methoxymethoxy)-1-(3-methylenecyclohexyl)benzene

Potassium *tert*-butoxide (50 mg) was added to a suspension of methyltriphenylphosphonium bromide in tetrahydrofuran (4 ml) at 0°C. After 0.5 hr, a solution of (±)-3-[2,4-*bis*(methoxymethoxy)phenyl]cyclohexanone (100 mg) in tetrahydrofuran (1 -ml) was added, and the mixture was allowed to warm to ambient temperature. After 16 hr, the reaction mixture was partitioned between ammonium chloride (30 ml of a saturated aqueous solution) and ethyl acetate. The aqueous layer was extracted with further ethyl acetate (2x 30 ml), and the combined organic extracts were washed with brine (30 ml), dried over magnesium sulfate, and evaporated *in vacuo.* The crude residue was purified by flash column chromatography (SiO₂, ethyl acetate/petrol, 4:1 v/v) to furnish the title compound as pale yellow oil (80 mg, 81%). δ_{H} (CDCl₃) 1.50 (2H, m), 1.90 (2H, m), 2.05 (1H, m), 2.16 (1H, m), 2.35 (1H, m), 2.44 (1H, m), 2.97 (1H, m), 3.44 (3H, s), 3.48 (3H, s), 4.64 (1H, s), 4.69 (1H, s), 5.13 (2H, s), 5.17 (2H, s), 6.68 (1H, m), 6.77 (1H, m), 7.12 (1H, d).

### Intermediate 13

### (±)-{3-[2,4-Bis(methoxymethoxy)phenyl]cyclohexyl}methanol

9-Borabicyclononane (2.7 ml of a 0.5M solution in tetrahydrofuran) was added to a stirred solution of (±)-2,4-*bis*(methoxymethoxy)-1-(3-methylenecyclohexyl)benzene (80 mg) in tetrahydrofuran (2 ml) at 0°C under argon. After 1 hr at 0°C, the reaction mixture was allowed to warm to ambient temperature and stirring continued. After 2 hr, the reaction mixture was cooled to 0°C and water (0.1 ml) was added. After the effervescence had subsided, hydrogen peroxide (1 ml of a 30% w/v solution) and sodium hydroxide (1 ml of a 2M solution) were added and the mixture was allowed to warm to ambient temperature. After a further 16 hr, the reaction mixture was cooled to 0°C. Saturated aqueous sodium metabisulfite was added until no oxidant could be detected by starch iodide paper, and the reaction mixture was extracted with ethyl acetate (3x30 ml). The combined organic extracts were washed with water (20 ml), dried over magnesium sulfate, and evaporated *in vacuo.* The crude residue was purified by flash column chromatography (SiO₂, ethyl acetate/petrol, 1:1 v/v) to furnish the title compound as a pale brown oil (46 mg, 54%); m/z (ES⁺) 311 (M+H)⁺.

### Intermediate 14

### (±)-3-[2,4-Bis(methoxymethoxy)phenyl]cyclohexanone oxime

Hydroxylamine hydrochloride (71 mg), triethylamine (0.17 ml) and (±)-3-[2,4-*bis*(methoxymethoxy)phenyl]cyclohexanone (200 mg) were heated under reflux in ethanol (8 ml). After 0.75 hr, the reaction mixture was evaporated *in vacuo* and the residue was partitioned between ethyl acetate (100 ml) and water (100 ml). The aqueous layer was extracted with ethyl acetate (2x100 ml), and the combined organic extracts were dried over magnesium sulfate and evaporated *in vacuo.* The crude residue was purified by flash column chromatography (SiO₂, ethyl acetate/petrol, 1:1 v/v) to furnish the title compound as a pale yellow oil (197 mg, 94%); m/z (ES⁺) 310 (M+H)⁺.

### Intermediate 15

### (±)-N-{3-[2,4-Bis(methoxymethoxy)phenyl]cyclohexyl}hydroxylamine

Borane (0.412 ml of a 1M solution in tetrahydrofuran) was added to a stirred solution of (±)-3-[2,4-*bis*(methoxymethoxy)phenyl]cyclohexanone oxime (85 mg) in tetrahydrofuran (2 ml) at 0°C under argon. After 2 hr, acetic acid (1 ml) was added and the mixture was allowed to warm to ambient temperature. After 16 hr, sodium hydrogen carbonate (20 ml of a saturated aqueous solution) was added, the mixture was extracted with ethyl acetate (3x20 ml) and the combined organic extracts were dried over magnesium sulfate and evaporated *in vacuo*. The crude residue was purified by flash column chromatography (SiO₂, dichloromethane/methanol, 9:1 v/v) to furnish two compounds as clear gums which were identified as diastereoisomers of the title compound; trans-isomer (12 mg, 14%) m/z (ES⁺) 312 (M+H)⁺; *cis*-isomer (0.017 g, 20%) m/z (ES⁺) 312 (M+H)⁺.

### Intermediate 16

### 4-(2,4-Bis{[tert-butyl(dimethyl)silyl]oxy}phenyl)cyclohexanone

4-(2,4-Dihydroxyphenyl)cyclohexanone (400 mg) was dissolved in dimethyl formamide (3ml) with stirring. *tert*-Butyldimethylsilyl chloride (704 mg), imidazole (660 mg) and 4-dimethylaminopyridine (3 mg) were added sequentially. After 4 hr, the solvent was removed *in vacuo* and the residue partitioned between ethyl acetate (20 ml) and water (5 ml). The aqueous phase was extracted with ethyl acetate (2x10 ml), and the combined organic phases were washed with brine (10 ml), dried over anhydrous magnesium sulphate, and concentrated under reduced pressure to give a brown oil. Purification via flash column chromatography (SiO₂ eluting with ethyl acetate/petroleum ether, 1:9 v/v) furnished the title compound as white flakes (750 mg, 89%). δ_{H} (CDCl₃): 0.18 (6H, s), 0.20 (6H, s), 0.97 (9H, s), 1.03 (9H, s), 1.72 - 1.87 (2H, m), 2.15 - 2.17 (2H, m), 2.42 - 2.48 (4H, m), 3.33 (1H, tt), 6.32 (1H, d), 6.39 (1H, dd), 6.94 (1H, d); m/z (ES⁺) 435 (M+1)⁺.

### Intermediate 17

### tert-Butyl[3-{[tert-butyl(dimethyl)silyl]oxy}-4-(4-methylenecyclohexyl)phenoxy]dimethyl silane

To a stirred suspension of methyltriphenylphosphonium bromide (329 mg) in anhydrous THF (10 ml) at 0°C was added potassium *tert*-butoxide (103 mg) in one portion. After stirring for 30 min, a solution of 4-(2,4-*bis*{[*tert*-butyl(dimethyl)silyl]oxy}phenyl) cyclohexanone (200 mg) in THF (5 ml) was added. The reaction mixture was stirred for a further 30 min at 0°C, and saturated aqueous ammonium chloride solution (20 ml) was added. The layers were separated and the aqueous layer extracted with ethyl acetate (3x20 ml). The combined organic phases were washed with brine (20 ml), dried over anhydrous magnesium sulphate, and concentrated *in vacuo*. Purification via flash column chromatography (SiO₂ eluting with diethyl ether:petroleum ether, 1:4 v/v) furnished the title compound as a colourless oil (135 mg, 68%). δ_{H} (CDCl₃): 0.19 (6H, s), 0.24 (6H, s), 0.97 (9H, s), 1.03 (9H, s), 1.41 (2H, dq), 1.84 - 1.93 (2H, m), 2.16 (2H, dt), 2.33 - 2.42 (2H, m), 3.01 (1H, tt), 4.66 (2H, s), 6.29 (1H, dd), 6.40 (1H, dd), 6.94 (1H, d).

### Intermediate 18

### trans / cis -[4-(2,4-Bis{[tert-butyl(dimethyl)silyl]oxy}phenyl)cyclohexyl]methanol

*tert*-Butyl[3-{[*tert*-butyl(dimethyl)silyl]oxy}-4-(4-methylenecyclohexyl)phenoxy]dimethyl silane (135 mg) was dissolved in anhydrous THF (5 ml) with stirring and cooled to -78°C. 9-Borabicyclo[3.3.1]nonane (3.13 ml, 0.5M in THF) was added to the stirred solution and the resulting reaction mixture allowed to warm to room temperature over 3 hr and then stirred for 3 days. Cooling to 0°C was followed by addition of hydrogen peroxide (1 ml, 30% aqueous solution) and sodium hydroxide (1 ml, 2M aqueous solution). The reaction mixture was allowed to warm to room temperature with stirring over 1 hr, cooled to 0°C, and saturated aqueous sodium metabisulphate solution (30 ml) was added. The layers were separated and the aqueous layer extracted with ethyl acetate (3x20 ml). The combined organic phases were washed with brine (20 ml), dried over anhydrous magnesium sulphate, and concentrated in *vacuo*. Purification *via* flash column chromatography (SiO₂ eluting with petroleum ether:diethyl ether, 3:17 v/v) afforded the desired product as a yellow oil (73 mg, 52%). δ_{H} (CDCl₃): 0.18 (6H, s), 0.22 (6H, s), 0.97 (9H, s), 1.00 (9H, s), 1.03 - 1.15 (0.5H, m), 1.22 - 1.33 (0.5H, m), 1.43 - 1.61 (4H, m), 1.77 - 1.94 (4H, m), 2.75 - 2.92 (1H, m), 3.47 (1H, d), 3.70 (1H, d), 6.26 - 6.29 (1H, m), 6.37 - 6.40 (1H, m), 6.93 - 6.97 (1H, m).

### Intermediate 19

### trans / cis -4-(2,4-Bis{[tert-butyl(dimethyl)silyl]oxy}phenyl)-1-methylcyclohexanol

4-(2,4-*Bis*(*tert*-butyldimethylsilyloxy))phenylcyclohexanone (50 mg) was dissolved in anhydrous THF (10 ml) and cooled to 0°C. Methylmagnesium chloride (59 ml, 22% w/v in THF) was added dropwise and the reaction mixture allowed to warm to room temperature with stirring over 2 days. The reaction mixture was partitioned between aqueous HCl (10 ml, 0.5M) and ethyl acetate (10 ml). The aqueous phase was extracted with ethyl acetate (2x10 ml) and the combined organic phases were washed with saturated aqueous sodium bicarbonate solution (10 ml), brine (10 ml), and then dried over anhydrous magnesium sulphate. Removal of the solvent under reduced pressure afforded an oil which was purified *via* flash column chromatography (SiO₂ eluting with ethyl acetate:petroleum ether, 1:4 v/v) affording the title compound as a white solid and a mixture of diastereoisomers (29 mg, 56%). δ_{H} (CDCl₃): 0.19 (6H, s), 0.23 (6H, s), 0.97 (9H, s), 1.02 (9H, s), 1.26 (1.5H, s), 1.32 (1.5H, s), 1.36 - 1.82 (8H, m), 2.73 - 2.91 (1 H, m), 6.29 - 6.31 (1 H, m), 6.39 - 6.42 (1 H, m), 6.80 (0.5H, d), 7.20 (0.5H, d).

### Intermediate 20

### trans / cis - 4-(2,4-Bis{[tert-butyl(dimethyl)silyl]oxy}phenyl)cyclohexylamine

4-[2,4-*Bis*(*tert*-butyldimethylsilyloxy)phenyl]cyclohexanone oxime (120 mg) was dissolved in anhydrous methanol (10 ml) with stirring. The solution was cooled to -40°C, and nickel chloride hexahydrate (133 mg) was added. Stirring was continued for 10 min before sodium borohydride (42 mg) was added in one portion. The reaction mixture was stirred at - 40°C for 20 min and water (0.5 ml) was added. The reaction mixture was allowed to warm to room temperature with stirring. Silica gel was added and the solvent removed *in vacuo*. Purification *via* flash column chromatography (SiO₂ eluting with CH₂Cl₂:MeOH:NH₄OH, 44:50:6 v/v) afforded the desired product as a pale brown oil (83 mg, 71%) and a mixture of diastereoisomers. δ_{H} (CD₃OD): 0.22 (6H, s), 0.27 (6H, s), 1.02 (9H, s), 1.08 (9H, s), 1.24 - 1.40 (1H, m), 1.42- 1.57 (1H, m), 1.57- 1.68 (1H, m), 1.71 - 1.90 (4H, m), 1.99 - 2.07 (1H, m), 2.84 - 2.98 (1H, m), 4.28 - 4.40 (1H, m), 6.34 (1H, d), 6.44 (1H, t), 7.04 (0.5H, d), 7.16 (0.5H, d); m/z (ES⁺) 436 (M+1)⁺.

### Intermediate 21

### trans / cis -N-[4-(2,4-Bis{[tert-butyl(dimethyl)silyl]oxy}phenyl)cyclohexyl]acetamide

*trans* / *cis -* 4-(2,4-*Bis*{[*tert*-butyl(dimethyl)silyl]oxy}phenyl)cyclohexylamine (27 mg) was dissolved in pyridine (0.5 ml). Acetyl chloride (6 µl) and 4-dimethylaminopyridine (2 mg) were added sequentially and the reaction mixture stirred for 24 hr. The solvent was removed *in vacuo* and the residue partitioned between ethyl acetate (10 ml) and water (2 ml). The aqueous phase was extracted with ethyl acetate (2x5 ml) and the combined organic phases were washed with brine (10 ml), dried over anhydrous magnesium sulphate, and concentrated to give a brown oil. Purification via flash column chromatography (SiO₂ eluting with ethyl acetate:petroleum ether, 7:3 v/v) afforded the title product as a white solid (15 mg, 50%) and a mixture of diastereoisomers. δ_{H} (CDCl₃): 0.17 (3H, s), 0.19 (3H, s), 0.21 (3H, s), 0.22 (3H, s), 0.96 (9H, s), 1.02 (9H, s), 1.06 - 1.20 (2H, m), 1.36 - 1.54 (2H, m), 1.60 - 1.75 (2H, m), 1.80 - 1.92 (2H, m), 1.95 (1.5H, s), 2.00 (1.5H, s), 2.75 - 2.90 (1H, m), 3.76 - 3.87 (1 H, m), 5.34 - 5.39 (0.5H, d), 5.72 - 5.77 (0.5H, d), 6.26 - 6.29 (1H, m), 6.38 - 6.41 (1H, m), 6.93 (0.5H, d), 6.95 (0.5H, d); m/z (ES⁺) 478 (M + 1)⁺.

### Intermediate 22

### 3-[2,4-Bis(methoxymethoxy)phenyl]-2-cyclopenten-1-one

1,3-*Bis*(methoxymethoxy)-4-bromobenzene (1.0 g) was dissolved in THF (20 ml) and cooled to -78°C under argon. *N,N,N',N'*-Tetramethylethylene diamine was added followed by dropwise addition of *n*-BuLi (3.4 ml of a 2.2M solution in hexanes) over 10 mins. After stirring at -78°C for 1 hr, a solution of 3-methoxy-2-cyclopentene-1-one (605 mg) in THF (5 ml) was added slowly. The reaction mixture was stirred at -78°C for 1 hr before warming to 0°C. 1M HCl (20 ml) was added, and after 10 min the mixture was extracted with ethyl acetate (2x50 ml). The combined organic phases were washed with brine (30 ml), dried over magnesium sulfate, and concentrated *in vacuo*. The crude residue was purified by flash column chromatography (SiO₂, ethyl acetate/petrol, 1:1 v/v) to furnish the title compound as yellow oil (128 mg, 13%); m/z (ES⁺) 279 (M + 1)⁺.

### Intermediate 23

### (±)-3-[2,4-Bis(methoxymethoxy)phenyl]cyclopentanone

3-[2,4-*Bis*(methoxymethoxy)phenyl]-2-cyclopenten-1-one (50 mg) and palladium (10 mg, 10% on carbon) were stirred under an atmosphere of hydrogen for 15 hr. The mixture was then filtered through a plug of Celite, washing with ethyl acetate, and the filtrate was concentrated *in vacuo.* The residue was purified by flash column chromatography (SiO₂, ethyl acetate/petrol, 1:1 v/v) to furnish the title compound as a colourless oil (8 mg, 16%); m/z (ES⁺) 583 (2M + Na)⁺.

### Intermediate 24

### 3 - (Benzyloxy) - 2 - cyclohexen - 1 - one

To a round bottomed flask equipped with magnetic stirrer and Dean - Stark apparatus was added 1, 3 - cyclohexanedione (60.0 g, 535 mmol), toluene (450 ml), *p* - toluenesulfonic acid monohydrate (1.35 g, 5.20 mmol) and benzyl alcohol (52.6 g, 487 mmol). The resulting solution was heated to reflux temperature for 12hr. The reaction mixture was cooled to room temperature and then washed with saturated aqueous sodium carbonate solution (2x100 ml). The organic layer was then washed with brine (100 ml), dried over magnesium sulfate, filtered and concentrated *in vacuo*, affording a brown oil (94.9 g) which crystallised upon standing for 17hr. The crude crystalline material was slurried in isopropyl ether (20 ml). The mixture was filtered and the crystalline material was washed with ice cold isopropyl ether (3x30 ml), then with cold petroleum ether (2x20 ml). The resulting peach coloured crystalline solid was dried overnight under reduced pressure, furnishing the desired product (74.4g, 76%). m/z (ES⁺) 203 (M+H⁺).

### Intermediate 25

### (±)-3 - (Benzyloxy) - 6 - (8 - hydroxy - 1, 4 - dioxaspiro[4.5]dec - 8 - yl) - 2 - cyclohexen - 1 - one

To a round bottomed flask equipped with magnetic stirrer was added anhydrous tetrahydrofuran (600 ml) and di*iso*propylamine (38.1 ml, 272 mmol). The stirred solution was cooled to -78°C and *n* - butyl lithium (113.4 ml, 272 mmol, 2.4M in cyclohexanes) was added dropwise via syringe in 20 ml portions. The resulting yellow solution was stirred for 35 min at -78°C, then 3 - (benzyloxy) - 2 - cyclohexen - 1 - one (50.0 g, 248 mmol) was added as a solution in anhydrous tetrahydrofuran (100 ml). The solution was stirred for 1 hr prior to the addition of cyclohexane - 1, 4 - dione monoethylene ketal (38.7 g, 248 mmol) as a solution in anhydrous tetrahydrofuran (100 ml). The solution was stirred for 2 hr at -78°C, then allowed to warm slowly to room temperature over 1 hr. Saturated aqueous ammonium chloride (80 ml) was added, followed by dichloromethane (700 ml), and the mixture was stirred until no solids remained. The layers were separated and the aqueous phase extracted with dichloromethane (2x100 ml). The combined organic layers were washed with brine (50 ml), dried over magnesium sulfate, then concentrated *in vacuo.* Trituration of the resulting solid with methanol afforded the title compound (78.4 g, 88%). m/z (ES⁺) 359 (M+H⁺).

### Intermediate 26

### (±)-1 - (Benzyloxy) - 6 - bromo - 3 - (1, 4 - dioxaspiro[4.5]dec - 8 - yl) - 2 - oxabicyclo [2.2.2]octan - 5 - one

A round bottomed flask equipped with magnetic stirrer was charged with (±)-3 - (benzyloxy) - 6 - (8 - hydroxy - 1,4 - dioxaspiro[4.5]dec - 8 - yl) - 2 - cyclohexen - 1 - one (78.4 g, 219 mmol) and dichloromethane (600 ml). To the stirred solution was added *N* - bromosuccinimide (40.9 g, 230 mmol) in one portion, followed by aqueous hydrobromic acid (3 drops, 48% solution) when no more solid remained. The resulting solution was stirred at room temperature for 2 hr then poured into a separating funnel containing aqueous sodium *meta*bisulfite solution (150 ml) and dichloromethane (200 ml), then the funnel was shaken vigorously. The layers were separated and the organic layer was washed with brine (200 ml), dried over magnesium sulfate, filtered, then concentrated *in vacuo* to give a solid. Trituration with methanol (500 ml) afforded the desired compound (82.8 g, 86%) as a white solid. m/z (ES⁺) 437 and 439 [(1:1), M+H⁺].

### Intermediate 27

### 5 - (Benzyloxy) - 2 - (1, 4 - dioxaspiro[4.5]dec - 7 - en - 8 - yl)phenol

A round bottomed flask was charged with (±)-1 - (benzyloxy) - 6 - bromo - 3 - (1, 4 - dioxaspiro[4.5]dec - 8 - yl) - 2 - oxabicyclo[2.2.2]octan - 5 - one (13.8 g, 31.6 mmol) and anhydrous *N, N* - dimethylformamide (140 ml). To the stirred solution was added 1, 8 - diazabicyclo[5.4.0]undec - 7 - ene (9.92 ml, 66.3 mmol) in one portion. The solution turned dark brown in colour immediately and was then heated to 140°C for 12 hr with vigorous stirring. The reaction mixture was allowed to cool to room temperature and most of the solvent was removed under reduced pressure. The remaining oil was partitioned between ethyl acetate (200 ml) and water (100 ml), then the layers were separated and the aqueous phase was extracted with ethyl acetate (3x50 ml). The combined organic layers were back extracted with water (3x30 ml) to remove any residual *N, N -* dimethylformamide. The organic phase was washed with brine (20 ml), dried over magnesium sulfate, filtered and concentrated *in vacuo* to afford a brown oily solid, which was adsorbed onto silica gel. Purification *via* flash column chromatography (SiO₂, ethyl acetate / petroleum ether, 1:1, v/v) furnished the desired product (7.1g, 66%) as a white solid. m/z (ES⁺) 339(M+H⁺).

### Intermediate 28

### 4 - (1, 4 - Dioxaspiro[4.5]dec - 8 - yl) - 1, 3 - benzenediol

A round bottomed flask equipped with magnetic stirrer was charged with 5 - (benzyloxy) - 2 - (1, 4 - dioxaspiro[4.5]dec - 7 - en - 8 - yl)phenol (6.90 g, 20.4 mmol), ethanol (300 ml) and palladium (2.00 g, 10% on activated carbon). The reaction vessel was then evacuated and placed under a hydrogen atmosphere. This process was repeated 15 times before stirring vigorously for 64 hr under a hydrogen atmosphere. The reaction mixture was filtered through a celite plug, washing with ethyl acetate. The filtrate was concentrated *in vacuo,* furnishing the title compound (5.10 g, 100%) as a solid. m/z(ES⁺) 251(M+H⁺).

### Intermediate 29

### cis - N - Benzyl - N - [4 - (2, 4 - bis{[tert - butyl(dimethyl)silyl]oxy}phenyl)cyclohexyl] amine

To a round bottomed flask equipped with magnetic stirrer was loaded 4-(2, 4-*bis*{[*tert*- butyl(dimethyl)silyl]oxy}phenyl)cyclohexanone (3.20 g, 7.36 mmol). Anhydrous 1, 2-dichloroethane (85 ml) was added, and to the stirred solution was added benzylamine (0.97 ml, 8.83 mmol) as a solution in 1, 2-dichloroethane (20 ml). Activated powdered 4A molecular sieves (5.80 g) were added and the reaction mixture stirred vigorously for 2.5 hr. Tetramethylammoniumtriacetoxyborohydride (2.90 g, 11.0 mmol) was added in one portion and the reaction mixture stirred for 64 hr at room temperature. Aqueous sodium hydroxide solution (30 ml, 0.4M) was added and vigorous stirring was continued for 0.5 hr. The reaction mixture was then filtered through celite, washing with dichloromethane (100 ml). The layers were separated and the aqueous layer was extracted with dichloromethane (2x50 ml). The combined organic phases were washed with brine (100 ml), dried over magnesium sulfate, filtered and concentrated *in vacuo* affording the crude product. Purification via flash column chromatography (SiO₂, ethyl acetate / petroleum ether, gradient elution using 1:9, 1:4, then 3:7 v/v) furnished the title product (2.69 g, 70%) as a pale yellow oil. δ_{H}(CDCl₃) 0.01 (6H, s), 0.05 (6H, s), 0.77 (9H, s), 0.83 (9H, s), 1.31 (1H, br), 1.39 (4H, m), 1.52 (2H, m), 1.70 (2H, m), 2.69 (1H, m), 2.75 (1H, m), 6.10 (1H, d), 6.23 (1H, dd), 6.84 (1H, d), 7.15 (5H, m).

### Intermediate 30

### N-Benzyl - N - [4 - (2, 4 - bis{[tert-butyl(dimethyl)silyl]oxy}phenyl) cyclohexylidene] amine

To a round bottomed flask equipped with magnetic stirrer was added 4-(2, 4-*bis*{[*tert*-butyl(dimethyl)silyl]oxy}phenyl)cyclohexanone (817 mg, 1.88 mmol). Anhydrous dichloromethane (50 ml) was added followed by benzylamine (0.82 ml, 7.52 mmol) and activated 4A molecular sieves (10.0 g). The reaction mixture was stirred vigorously for 15 hr, then dichloromethane (50 ml) was added and the reaction mixture filtered through celite, washing with dichloromethane (50 ml). The filtrate was concentrated *in vacuo* affording the desired product (1.00 g, 86%) as a yellow oil. δ_{H}(CDCl₃) 0.19 (6H, s), 0.26 (6H, s), 0.98 (9H. s), 1.03 (9H, s), 1.51 (1H, m), 1.72 (1H, m), 2.03 (2H, m), 2.45 (1H, m), 2.60 (1H, m), 3.04 (1H, m), 3.22 (1H, m), 4.55 (1H, d), 4.60 (1H, d), 6.31 (1H, d), 6.41 (1H, dd), 6.93 (1H, d), 7.33 (5H, m).

### Intermediate 31

### trans - N - Benzyl - N - [4 - (2, 4 - bis{[tert - butyl(dimethyl)silyl]oxy}phenyl) cyclohexyl]amine

To a round bottomed flask equipped with magnetic stirrer was added *N* - benzyl - *N*-[4 - (2, 4 - *bis*{[*tert* - butyl(dimethyl)silyl]oxy}phenyl)cyclohexylidene]amine (4.00 g, 7.63 mmol) and anhydrous tetrahydrofuran (480 ml) followed by anhydrous methanol (120 ml). To the solution was added sodium borohydride (1.16 g, 30.5 mmol) and the reaction mixture stirred for 17 hr. The reaction mixture was then diluted with diethyl ether (600 ml) and aqueous sodium hydroxide (400 ml, 0.4M) was added. After stirring for 10 min, the layers were separated and the aqueous layer extracted with dichloromethane (3x100 ml). The combined organic phases were washed with brine (50 ml), dried over magnesium sulfate and concentrated *in vacuo* to give a yellow oil. Purification via flash column chromatography (SiO₂, ethyl acetate / petroleum ether, gradient elution using 1:9, 1:4, then 3:7, v/v) furnished the desired product as a cream coloured solid (2.09 g, 54%). δ_{H}(CDCl₃) 0.01 (6H, s), 0.05 (6H, s), 0.80 (9H, s), 0.85 (9H, s), 1.18 (4H, m), 1.66 (2H, m), 1.87 (2H, m), 2.19 (1H, m), 2.68 (1 H, M), 6.12 (1H, d), 6.23 (1H, dd), 6.77 (1 H, d), 7.17 (5H, m).

### Intermediate 32

### trans-4 - (2,4 - Bis{[tert - butyl(dimethyl)silyl]oxy}phenyl)cyclohexylamine

To a round bottom flask was added *trans* - *N -* benzyl - *N* - [4 - (2, 4 - *bis*{[*tert*-butyl(dimethyl)silyl]oxy} phenyl) cyclohexyl]amine (500 mg, 0.95 mmol) and ethanol (20 ml). To the stirred solution was added palladium (10% w/w on activated carbon, 200 mg, 0.19 mmol) as a slurry in ethanol (5 ml). The reaction vessel was evacuated, then placed under hydrogen (10 cycles). The reaction mixture was stirred vigorously under an atmosphere of hydrogen for 18 hr, then filtered through a celite plug, washing with methanol (100 ml). The solvent was removed in vacuo affording the desired product (402 mg, 97%) as a colourless oil. δ_{H}(CDCl₃) 0.01 (6H, s), 0.05 (6H, s), 0.78 (9H, s), 0.82 (9H, s), 1.08 (2H, m), 1.21 (2H, m), 1.62 (2H, m), 1.78 (2H, m), 2.59 (2H, m), 6.11 (1H, d), 6.22 (1 H, dd), 6.78 (1 H, d).

### Intermediate 33

### cis - 4 - (2, 4 - bis{[tert-butyl(dimethyl)silyl]oxy}phenyl)cyclohexylamine

To a round bottom flask equipped with magnetic stirrer was added cis - *N* - benzyl-*N* - [4 - (2, 4 - *bis*{[*tert -* butyl(dimethyl)silyl]oxy}phenyl)cyclohexyl]amine (700 mg, 1.33 mmol) and ethanol (30 ml). To the stirred solution was added palladium (10% w/w on activated carbon, 283 mg, 0.27 mmol) as a slurry in ethanol (5 ml). The reaction vessel was evacuated then placed under hydrogen (10 cycles). The reaction mixture was stirred vigorously under an atmosphere of hydrogen for 18 hr then filtered through a celite plug, washing with methanol (100 ml). The solvent was removed *in vacuo* affording the desired product (561 mg, 97%) as a colourless oil. δ_{H}(CDCl₃) 0.01 (6H, s), 0.04 (6H, s), 0.78 (9H, s), 0.83 (9H, s), 1.21 - 1.55 (10H, m), 2.64 (1H, m), 3.05 (1H, m), 6.11 (1H, d), 6.22 (1H, dd), 6.84 (1H, d).

### Intermediate 34

### cis - N - [4 - (2, 4 - Bis{[tert-butyl(dimethyl)silyl]oxy}phenyl)cyclohexyl] methane sulfonamide

A round bottomed flask equipped with magnetic stirrer was charged with *cis* - 4 - (2, 4 - *bis*{[*tert*-butyl(dimethyl)silyl]oxy}phenyl)cyclohexylamine (47 mg, 0.108 mmol) and 1, 2-dichloroethane (4 ml). To the stirred solution was added methanesulfonyl chloride (10 µl, 0.12 mmol), triethylamine (30 µl, 0.22 mmol) and 4-dimethylaminopyridine (catalytic amount). The solution was stirred for 17 hr then partitioned between aqueous sodium hydroxide (5 ml, 0.2M) and dichloromethane (5 ml). The aqueous phase was extracted with dichloromethane (2x5 ml) and the combined organic layers were washed with brine (8 ml), dried over magnesium sulfate and concentrated *in vacuo*. The crude product was adsorbed onto silica gel and purified *via* flash column chromatography (SiO₂, ethyl acetate / petroleum ether, gradient elution using 1:9, 1:4, then 1:3, v/v) to give the title compound (39 mg, 70%) which solidified on standing. δ_{H}(CDCl₃) 0.17 (6H, s), 0.23 (6H, s), 0.97 (9H, s), 1.00 (9H, s), 1.53 (2H, m), 1.71 (4H, m), 1.94 (2H, m), 2.85 (1H, m), 2.99 (3H, s), 3.78 (1H, m), 4.83 (1H, d), 6.28 (1H, d), 6.42 (1 H, dd), 6.97 (1H, d).

### Intermediate 35

### trans - N - [4 - (2, 4 - Bis{[tert-butyl(dimethyl)silyl]oxy}phenyl)cyclohexyl] methane sulfonamide

To a round bottomed flask equipped with magnetic stirrer was added *trans* - 4 - (2, 4-*bis*{[*tert* - butyl(dimethyl)silyl]oxy}phenyl)cyclohexylamine (248 mg, 0.57 mmol) and 1, 2-dichloroethane (25 ml). To the stirred solution was added triethylamine (191 µl, 1.37 mmol) followed by methanesulfonyl chloride (53 µl, 0.68 mmol) and three crystals of 4-dimethylaminopyridine. The resulting solution was stirred at room temperature for 18 hr and then poured into a separating funnel containing dichloromethane (100 ml) and water (20 ml). The layers were separated and the aqueous phase extracted with dichloromethane (1x50 ml). The combined organic phases were washed with brine (50 ml), dried over magnesium sulfate, filtered and concentrated in vacuo affording the title compound as a pale yellow oil (320 mg, 100%). δ_{H}(CDCl₃) 0.02 (6H, s), 0.05 (6H, s), 0.78 (9H, s), 0.82 (9H, s), 1.22 (4H, m), 1.70 (2H, m), 1.99 (2H, m), 2.61 (1 H, m), 2.81 (3H, s), 3.19 (1 H, m), 3.92 (1 H, d), 6.13 (1 H, d), 6.22 (1 H, dd), 6.66 (1 H, d).

### Intermediate 36

### 4 - (4 - {[tert - Butyl(dimethyl)silyl]oxy}phenyl)cyclohexanone

To a round bottomed flask equipped with magnetic stirrer was added 4-(4-hydroxy)phenylcyclohexanone (1.00 g, 5.26 mmol) (commercially available from Aldrich) and anhydrous *N,N*-dimethylformamids (5 ml). To the stirred solution was added imidazole (0.90 g, 13.20 mmol), *tert*-butyl(dimethyl)silyl chloride (1.19 g, 7.89 mmol) and 4-dimethylaminopyridine (catalytic amount). The reaction mixture was stirred for 17 hr at room temperature and then the *N,N*-dimethylformamide removed *in vacuo*. The residue was partitioned between ethyl acetate (100 ml) and water (5 ml). The layers were separated and the aqueous phase was extracted with ethyl acetate (3x20 ml). The combined organic layers were washed with brine, dried over magnesium sulfate, filtered and concentrated *in vacuo*. Purification via flash column chromatography (SiO₂, ethyl acetate / petroleum ether, 2:3, v/v) afforded the title compound (1.39 g, 87%) as a pale yellow solid. δ_{H}(CDCl₃) 0.19 (6H, s), 0.98 (9H, s), 1.87 (2H, m), 2.20 (2H, m), 2.46 (4H, m), 2.99 (1 H, m), 6.77 (2H, d), 7.07 (2H, d).

### Intermediate 37

### 4 - (4 - {[tert - Butyl(dimethyl)silyl]oxy}phenyl) - 1 - [2, 4 - bis(methoxymethoxy) phenyl] cyclohexanol

A round bottomed flask equipped with magnetic stirrer was charged with 1-bromo-2,4-bis(methoxymethoxy)benzene (277 mg, 1.00 mmol) and anhydrous tetrahydrofuran (5 ml). The stirred solution was cooled to -78°C and *N, N, N', N'*-tetramethylethylene diamine (0.32 ml, 2.10 mmol) was added followed by dropwise addition of *n*-butyl lithium (0.88 ml, 2.10 mmol, 2.40M solution in cyclohexanes). The resulting solution was stirred for 40 min at -78°C, then 4 - (4 - {[*tert* - butyl(dimethyl)silyl]oxy}phenyl) cyclohexanone (304 mg, 1.00 mmol) was added via syringe as a solution in anhydrous tetrahydrofuran (2 ml) and the reaction mixture stirred for 30 min at -78 °C, then allowed to warm to room temperature over 3 hr. The reaction was quenched with aqueous hydrochloric acid (5 ml, 0.10M), then poured into a separating funnel containing ethyl acetate (50 ml) and water (10 ml). The layers were separated and the aqueous phase extracted with ethyl acetate (3x5 ml). The combined organic layers were washed with brine (10 ml), dried over magnesium sulfate, filtered and concentrated *in vacuo.* The resulting oil was purified via flash column chromatography (SiO₂, ethyl acetate / petroleum ether, 3:7, v/v) furnishing the title compound as a white solid (127 mg, 25%) and a mixture of diastereoisomers. δ_{H}(CDCl₃) 0.19 (6H, s), 0.98 (6H, s), 1.63 (2H, m), 1.84 (2H, m), 1.96 (2H, m), 2.56 (2H, m), 2.70 (1H, m), 3.48 (3H, s), 3.49 (3H, s), 3.85 (1H, s), 5.15 (2H, s), 5.24 (2H, s), 6.68 (1H, dd), 6.73 (2H, d), 6.85 (1H, d), 7.05 (2H, d), 7.33 (1H, d).

### Intermediate 38

### tert - Butyl (4 - {4 - [2, 4-bis(methoxymethoxy)phenyl]-3-cyclohexen-1 -yl}phenoxy) dimethylsilane

A round bottomed flask equipped with magnetic stirrer was charged with 4 - (4 - {[*tert* - butyl(dimethyl)silyl]oxy}phenyl) - 1 - [2, 4 - bis(methoxymethoxy) phenyl]cyclohexanol (125 mg, 0.25 mmol), toluene (10 ml) and *p*-toluene sulfonic acid monohydrate (3 crystals). The reaction mixture was heated to reflux temperature for 30 min, then cooled to room temperature before adding saturated aqueous sodium bicarbonate (5 ml). The layers were separated and the aqueous layer was extracted with ethyl acetate (3x 10ml). The combined organic layers were washed with brine (10 ml), dried over magnesium sulfate, filtered and concentrated in vacuo. The title compound was isolated without further purification as an oil (118 mg, 98%) and a mixture of diastereoisomers. δ_{H}(CDCl₃) 0.19 (6H, s), 0.98 (9H, s), 1.84 (1H, m), 2.00 (1H, m), 2.27 (1H, m), 2.44 (2H, m), 2.56 (1H, m), 3.48 (3H, s), 3.49 (3H, s), 5.15 (2x2H, s), 5.79 (1H, m), 6.67 (1H, dd), 6.77 (2H, d), 6.78 (1H, d), 7.07 (2H, d), 7.11 (1H, d).

### Intermediate 39

### tert - Butyl (4 - {4 - [2, 4 - bis(methoxymethoxy)phenyl]cyclohexyl}phenoxy) dimethyl silane

To a round bottomed flask equipped with magnetic stirrer was added tert - butyl (4-{4 - [2, 4-*bis*(methoxymethoxy)phenyl]-3-cyclohexen-1-yl}phenoxy) dimethylsilane (118 mg, 0.24 mmol) and ethanol (15 ml). To the stirred solution, palladium (catalytic amount, 10% on activated carbon) was added in one portion. The reaction vessel was then evacuated and placed under an atmosphere of hydrogen. This process was repeated for 10 cycles before leaving under a hydrogen atmosphere. The reaction mixture was stirred vigorously for 17 hr then filtered through celite, washing with ethyl acetate. The filtrate was concentrated in vacuo affording the title compound as a colourless oil (118 mg, 100%) and a mixture of diastereoisomers. δ_{H}(CDCl₃) 0.15 (6H, s), 0.92 (9H, s), 1.51 (2H, m), 1.63 (2H, m), 1.81 (2H, m), 1.94 (2H, m), 2.43 and 2.90 (1H, m), 2.84 and 3.02 (1H, m), 3.38 and 3.39 (3H, s), 3.40 (2x1.5H, s), 5.04 and 5.06 (2H, s), 5.08 and 5.10 (2H, s), 6.56 and 6.61 (1H, m), 6.70 (3H, m), 7.03 (2H, m), 7.11 (1H, d).

### Intermediate 40

### (±)-Methyl {4 - [2, 4 - bis(methoxymethoxy)phenyl]cyclohexylidene}acetate

A round bottomed flask equipped with magnetic stirrer was loaded with sodium hydride (0.20 g, 5.10 mmol, 60% dispersion in mineral oil) which was washed with petroleum ether (4x20 ml). The excess petroleum ether was removed under reduced pressure. Anhydrous tetrahydrofuran (120 ml) was added and the stirred solution was cooled to 0°C. Trimethylphosphonoacetate (756 µl, 5.10 mmol) was added dropwise *via* syringe and the stirred mixture was allowed to warm to room temperature over 1 hr. The mixture was cooled to 0°C prior to the addition of 4 - [2, 4 - *bis*(methoxymethoxy)phenyl] cyclohexanone (1.00 g, 3.40 mmol) as a solution in tetrahydrofuran (30 ml). The pale yellow mixture was heated to reflux temperature for 0.75 hr, then cooled to room temperature and partitioned between ethyl acetate (100 ml) and saturated aqueous ammonium chloride solution (30 ml). The layers were separated and the aqueous layer was extracted with ethyl acetate (3x30 ml). The combined organic layers were washed with brine (30 ml), dried over magnesium sulfate, filtered and concentrated *in vacuo*, affording a yellow oil. Purification *via* flash column chromatography (SiO₂, ethyl acetate / petroleum ether, 1:2, v/v) furnished the title compound as a yellow oil (1.13 g, 95%). δ_{H}(CD₃OD) 1.53 - 1.70 (2H, m), 2.00 - 2.13 (4H, m), 2.45 (2H, m), 3.26 (1H, m), 3.48 (3H, s), 3.53 (3H, s), 3.71 (3H, s), 5.17 (2H, s), 5.24 (2H, s), 5.73 (1 H, s), 6.67 and 6.68 (1H, d), 6.83 (1H, d), 7.08 (1H, d).

### Intermediate 41

### cis / trans - Methyl (4 - [2, 4 - bis(methoxymethoxy)phenyl]cyclohexyl} acetate

To a round bottomed flask equipped with magnetic stirrer was added (±)-methyl {4-[2, 4 - *bis*(methoxymethoxy)phenyl]cyclohexylidene}acetate (1.13 g, 3.23 mmol) and ethanol (50 ml). To the stirred solution was added palladium (catalytic amount, 10% on activated carbon) in one portion. The reaction vessel was evacuated and placed under an atmosphere of hydrogen. This process was repeated for 10 cycles before leaving under a hydrogen atmosphere and then stirred vigorously for 17 hr. The reaction mixture was filtered through celite, washing with ethanol and the filtrate was evaporated to dryness. furnishing the title compound (1.13 g, 99%) as a colourless oil and a mixture of diastereoisomers. δ_{H}(CD₃OD) 1.53 (2H, m), 1.62 - 1.78 (2H, m), 1.87 (4H, m), 2.93 (1H, m), 3.47 (3H, s), 3.51 (3H, s), 3.71 and 3.72 (3H, s), 5.16 and 5.17 (2H, s), 5.22 and 5.23 (2H, s), 6.66 and 6.68 (1 H, d), 6.79 and 6.80 (1H, s), 7.12 and 7.15 (1H, d).

### Intermediate 42

### (±)-{4 - [2,4 - Bis(methoxymethoxy)phenyl]cyclohexylidene}acetic acid

To a round bottomed flask equipped with magnetic stirrer was added trimethylsilyldiethylphosphonoacetate (1.08 ml, 3.83 mmol) and anhydrous tetrahydrofuran (25 ml). The stirred solution was cooled to 0°C and *n*-butyl lithium (1.80 ml, 3.83 mmol, 2.2M in cyclohexanes) was added dropwise, *via* syringe over 5 min. The reaction mixture was allowed to warm slowly to room temperature and stirred for 17 hr. 4 - [(2, 4-*Bis*(methoxymethoxy)phenyl)]cyclohexanone (750 mg, 2.55 mmol) was added *via* syringe as a solution in tetrahydrofuran (25 ml). After 2 hr stirring at room temperature, the reaction mixture was poured into a separating funnel containing aqueous sodium hydroxide solution (10 ml, 10% w/v). After extracting once with diethyl ether (10 ml), the aqueous layer was acidified by adding concentrated hydrochloric acid (10 ml), then extracted with diethyl ether (3x20 ml). The combined organic layers were washed with water (10 ml), dried over magnesium sulfate, filtered and concentrated *in vacuo* affording the title compound (422 mg, 52%) as an oil. δ_{H}(CDCl₃) 1.86 (2H, m), 2.00 - 2.13 (4H, m), 2.42 (2H, m), 3.19 (1H, m), 3.48 (3H, s), 3.51 (3H, s), 5.14 (2H, s), 5.21 (2H, s), 5.71 (1H, s), 6.67 (1H, dd), 6.81 (1H, d), 7.05 (1H, d).

### Intermediate 43

### (±)-{4 - [2,4 - Dihydroxyphenyl]cyclohexylidene}acetic acid

A round bottomed flask equipped with magnetic stirrer was loaded with (±)-{4 - [2, 4-*bis*(methoxymethoxy)phenyl]cyclohexylidene}acetic acid (25 mg, 74 µmol), acidic dowex resin (75 mg) and methanol (15 ml) then stirred at 60°C for 3 hr. The reaction mixture was filtered through a celite plug, washing with methanol. The solvent was removed under reduced pressure to give a yellow oil (15 mg) which was purified by preparative TLC (ethyl acetate /petroleum ether, 3:1, v/v), furnishing the title compound (6.5mg, 35%) as an oil. m/z(ES⁺) 339 (M+H⁺).

### Intermediate 44

### (±)-{4-[2, 4-Bis(methoxymethoxy)phenyl]cyclohexylidene}acetonitrile

To a round bottomed flask equipped with magnetic stirrer was added sodium hydride (40 mg, 0.95 mmol, 60% dispersion in mineral oil). After washing the sodium hydride with petroleum ether (2x20 ml), the excess solvent was removed under reduced pressure. 1, 2-Dimethoxyethane (10 ml) was added and the stirred suspension cooled to 0°C. Diethyl cyanomethylphosphonate (102 µl, 0.95 mmol) was added dropwise. The reaction mixture was allowed to warm slowly to room temperature over 2 hr. 4-[(2, 4-*Bis*(methoxymethoxy)phenyl)]cyclohexanone (200 mg, 0.68 mmol) was then added as a solution in 1, 2-dimethoxyethane (10 ml) and the reaction mixture stirred for 17 hr at room temperature. The reaction mixture was poured into a separating funnel containing water (50 ml) and diethyl ether (50 ml). The layers were separated and the aqueous phase extracted with diethyl ether (2x20 ml). The combined organic layers were washed with brine (20 ml), dried over magnesium sulfate, filtered and concentrated *in vacuo*, to give an oil. Purification via flash column chromatography (SiO₂, ethyl acetate / petroleum ether, 1:2, v/v) afforded the title compound (141 mg, 66%) as a pale yellow oil. δ_{H}(CD₃OD) 1.57 - 1.70 (2H, m), 2.02 - 2.25 (2H, m), 2.34 -2.49 (2H, m), 2.60 (1H, m), 3.03 (1H, m), 3.24 (1H, m), 3.47 (3H, s), 3.52 (3H, s), 5.17 (2H, s), 5.23(2H, s), 5.33 (1H, s), 6.66 and 6.69 (1H, d), 6.83 (1H, d), 7.09 (1H, d

### Intermediate 45

### (±)-[4-(2, 4-Dihydroxyphenyl)cyclohexylidene]acetonitrile

To a round bottomed flask equipped with magnetic stirrer was added (±)-{4-[2, 4-*bis*(methoxymethoxy)phenyl]cyclohexylidene}acetonitrile (141 mg, 0.45 mmol) and methanol (5 ml). The reaction mixture was stirred, the solution was heated to reflux temperature and aqueous hydrochloric acid (5 ml, 1.0M) was added slowly. Heating was continued for 1 hr and the reaction mixture was allowed to cool to room temperature prior to the addition of saturated aqueous sodium bicarbonate solution (12 ml). The reaction mixture was then partitioned between ethyl acetate (30 ml) and water (10 ml). The aqueous layer was extracted with ethyl acetate (3x15 ml) and the combined organic layers washed with brine (20 ml), dried over magnesium sulfate, filtered and concentrated *in vacuo.* The residue was purified via flash column chromatography (SiO₂, ethyl acetate / petroleum ether, 1:1, v/v) to afford the title compound as a white solid (90 mg, 88%). m/z (ES⁻) 228 (M-H⁺); δ_{H}(CD₃OD) 1.56 - 1.68 (2H, m), 2.02 - 2.14 (2H, m), 2.33 - 2.48 (2H, m), 2.57 (1 H, m), 3.01 (1 H, m), 3.14 (1H, m), 5.31 (1H, s), 6.26 and 6.28 (1H, d), 6.32 (1H, d), 6.80 (1H, d).

### Intermediate 46

### (±)-1-(3,3-Difluorocyclohexyl)-2,4-bis(methoxymethoxy)benzene

Diethylaminosulfur trifluoride (34 µl) was added to a stirred solution of (±)-3-[2,4-*bis*(methoxymethoxy)phenyl]cyclohexanone (40 mg) in anhydrous 1,2-dimethoxyethane at room temperature under argon. After 1 hr further diethylaminosulfur trifluoride (170 µl) was added. After 48 hr, the reaction mixture was partitioned between water (20 ml) and ethyl acetate (20 ml). The aqueous layer was extracted with ethyl acetate (2 x 20 ml) and the combined organic extracts were dried over magnesium sulfate and evaporated *in vacuo.* The crude residue was purified by flash column chromatography (SiO₂, ethyl acetate/petrol, 1:1 v/v) to give the title compound as a gum (31 mg); m/z (ES⁺) 317 (M+H)⁺, R_{f} (ethyl acetate/petrol, 1:1 v/v) 0.5.

### Intermediate 47

### (±)-3-[2,4-Bis(methoxymethoxy)phenyl]cyclohexanecarboxylic acid

A solution of (±)-{3-[2,4-*bis*(methoxymethoxy)phenyl]cyclohexyl}methanol (50 mg) in acetone (1 ml) was added to a stirred solution of chromium (VI) oxide (64 mg) in 2M sulfuric acid (0.64 ml) at 0°C over 3 hr. After 3 hr at 0°C and then 16 hr at room temperature, the reaction mixture was partitioned between water (10ml) and ethyl acetate (20 ml). The layers were separated and the aqueous layer was extracted with ethyl acetate (2 x 20 ml). The combined organic extracts were washed with water (20 ml), dried over magnesium sulfate and evaporated *in vacuo.* The residue was purified by flash column chromatography (SiO₂, ethyl acetate/petrol, 4:1 v/v) to give the title compound as a colourless oil (15 mg, 29%). R_{f} (ethyl acetate/petrol, 4:1 v/v) 0.5.

### Intermediate 48

### (±)-3-[2,4-Bis(methoxymethoxy)phenyl]cyclohexanecarboxamide

Triethylamine (16 µl) and isobutylchloroformate (14 µl) were added to a stirred solution of (±)-3-[2,4-*bis*(methoxymethoxy)phenyl]cyclohexanecarboxylic acid in anhydrous tetrahydrofuran (2 ml) at 0°C under argon. After 30 min, aqueous ammonia solution (0.5 ml, 28% w/w) was added. The reaction mixture was partitioned between water (20 ml) and ethyl acetate (50 ml). The aqueous layer was extracted with ethyl acetate (2 x 20 ml) and the combined organic extracts were dried over magnesium sulfate and evaporated *in vacuo* to give the title compound (26 mg, 87%) as a mixture of diastereoisomers; m/z (ES⁺) 324 (M+H)⁺.

### Intermediate 49

### (±)-3-[2,4-Bis(methoxymethoxy)phenyl]-N-hydroxycyclohexanecarboxamide

Triethylamine (11 µl) and isobutylchloroformate (10 µl) were added to a stirred solution of (±)-3-[2,4-*bis*(methoxymethoxy)phenyl]cyclohexanecarboxylic acid in anhydrous tetrahydrofuran (2 ml) at 0°C under argon. After 30 min, aqueous hydroxylamine solution (0.5 ml, 50 wt%) was added. The reaction mixture was partitioned between water (20 ml) and ethyl acetate (50 ml). The aqueous layer was extracted with ethyl acetate (2 x 20 ml) and the combined organic extracts were dried over magnesium sulfate and evaporated *in vacuo* to give the title compound as a solid (26 mg, quant.) and a mixture of diastereoisomers; m/z (ES⁺) 340 (M+H)⁺.

### Intermediate 50

### (±)-3-[2,4-Bis(methoxymethoxy)phenyl]-N-ethylcyclohexanecarboxamide

Triethylamine (11 µl) and isobutylchloroformate (10 µl) were added to a stirred solution of (±)-3-[2,4-*bis*(methoxymethoxy)phenyl]cyclohexanecarboxylic acid in anhydrous tetrahydrofuran (2 ml) under argon at 0°C. After 30 min, ethylamine (0.5 ml, 2M solution in tetrahydrofuran) was added. The reaction mixture was partitioned between water (20 ml) and ethyl acetate (50 ml). The aqueous layer was extracted with ethyl acetate (2 x 20 ml) and the combined organic extracts were dried over magnesium sulfate and evaporated *in vacuo* to give the title compound as a solid (24 mg, quant.) and a mixture of diastereoisomers; m/z (ES⁺) 352 (M+H)⁺.

### Intermediate 51

### (±)-3-[2,4-Bis(methoxymethoxy)phenyl]-1-(hydroxymethyl)cyclohexanol

*N*-Methyl morpholine *N*-oxide (120 mg) and osmium tetroxide (100 µl, 2.5 wt% in *tert*-butanol) were added to a solution of (±)-2,4-*bis*(methoxymethoxy)-1-(3-methylenecyclohexyl) benzene (30 mg) in tetrahydrofuran (0.7 ml) and water (0.3 ml) at room temperature. After 16 hr, celite was added to the reaction mixture followed by water (5 ml) and sodium persulfite (20 mg). The reaction mixture was filtered, the filtrate was adjusted to pH 4 with 2M hydrochloric acid solution and the aqueous volume was made up to 20 ml. The aqueous layer was extracted with ethyl acetate (3x 20 ml), the combined organic extracts were washed with brine (20 ml), dried over magnesium sulfate and evaporated *in vacuo.* The residue was purified by flash column chromatography (SiO₂, ethyl acetate) to give the title compound as a solid (30 mg, 89%) and a mixture of diastereoisomers; m/z (ES⁺) 327 (M+H)⁺.

### Intermediate 52

### (±)-N-{3-[2,4-bis(methoxymethoxy)phenyl]cyclohexyl}acetamide

Triethylamine (15 µl) and acetic anhydride (10 ml) were added to a stirred solution of (±)-3-[2,4-*bis*(methoxymethoxy)phenyl]cyclohexylamine (29 mg) in anhydrous 1,2-dichloroethane (2 ml) at room temperature. After 0.5 hr, the reaction mixture was partitioned between water (20 ml) and ethyl acetate (50 ml) and the aqueous layer was extracted with ethyl acetate (3 x 20 ml). The combined organic extracts were dried over magnesium sulfate and evaporated *in vacuo* to give the title compound as a solid (22 mg, 66%) and a mixture of diastereoisomers: m/z (ES⁺) 338 (M+H)⁺.

### Intermediates 53 and 54

### trans-4-(2,4-Bis{[tert-butyl(dimethyl)silyl]oxy}phenyl)cyclohexanol cis-4-(2,4-Bis{[tert-butyl(dimethyl)silyl]oxy}phenyl)cyclohexanol

Sodium borohydride (164 mg) was added to a stirred solution of 4-(2,4-*bis*{(*tert*-butyl(dimethyl)silyl]oxy}phenyl)cyclohexanone (1.57 g) in ethanol (50 ml) at 0°C. After 2 hr at 0°C and then 18 hr at room temperature, the reaction mixture was partitioned between 2M HCl (20 ml), water (40 ml) and ethyl acetate (50 ml) and the aqueous layer was re-extracted with ethyl acetate (2 x 50ml). The combined organic extracts were washed with brine (40 ml), dried over magnesium sulfate and evaporated *in vacuo.* The residue was purified using flash column chromatography (SiO₂, ethyl acetate/petrol, 3:17 v/v) to give the trans-title compound as a white solid (546 mg, 35%), and the cis-title compounds as a white solid (83 mg, 5%).
*trans* - δ_{H} (CDCl₃) 0.18 (6H, s), 0.22 (6H, s), 0.98 (9H, s), 1.02 (9H, s), 1.18 - 1.22 (4H, m), 1.80 - 1.84 (3H, m), 2.00 - 2.05 (2H, m), 2.78 - 2.86 (1H, m), 3.60 - 3.70 (1H, m), 6.28 (1 H, d), 6.39 (1H, dd), 6.94 (1 H, d).
*cis* - δ_{H} (CDCl₃) 0.18 (6H, s), 0.22 (6H, s), 0.98 (9H, s), 1.02 (9H, s), 1.58 - 1.78 (6H, m), 1.84 - 1.92 (2H, m), 2.70 - 2.80 (1H, m), 4.12 (1H, bs), 6.28 (1H, d), 6.40 (1H, dd), 7.02 (1H, d).

### Intermediate 55

### trans-4-(2,4-Bis{[tert-butyl(dimethyl)silyl]oxy}phenyl)cyclohexyl 4-(dimethylamino) benzoate

Triethylamine (20 µl), 4-dimethylaminopyridine (catalytic amount) and 4-dimethylaminobenzoyl chloride (26 mg) were added to a stirred solution of *trans*-4-(2,4-*bis*{[*tert*-butyl(dimethyl)silyl]oxy}phenyl)cyclohexanol (30 mg) in dry dichloromethane (2 ml) at room temperature under argon. After 24 hr, the solvent was removed under reduced pressure and the residue was partitioned between water (10 ml) and ethyl acetate (20 ml). The aqueous layer was extracted with ethyl acetate (2x 20 ml) and the combined organic extracts were washed with brine (10 ml), dried over magnesium sulfate and evaporated *in vacuo*. The residue was purified by flash column chromatography (SiO₂, ethyl acetate/petrol, 1:24 v/v) to give the title compound as a white solid (18 mg, 45%); m/z (ES⁺) 585 (M+H)⁺.

### Intermediate 56

### cis / trans - 4-(2,4-Bis{[tert-butyl(dimethyl)silyl]oxy}phenyl)cyclohexanecarboxylic acid

Pyridinium dichromate (146 mg) was added to a stirred solution of *cis* / *trans* - [4-(2,4-*bis*{[*tert*-butyl(dimethyl)silyl]oxy}phenyl)cyclohexyl]methanol (50 mg) in *N,N*-dimethylformamide (1 ml) at room temperature under argon. After 24 hr, the reaction mixture was partitioned between water (20 ml) and diethyl ether (30 ml). The layers were separated and the aqueous layer was extracted with diethyl ether (2 x 30 ml). The combined organic extracts were dried over magnesium sulfate and evaporated *in vacuo*. The residue was purified by flash column chromatography (SiO₂, ethyl acetate/petrol, 1:9 v/v) to give the title compound as a cream solid (23 mg, 44%); m/z (ES⁺) 465 (M+H)⁺.

### Intermediate 57

### trans-4-(2,4-Bis{[tert-butyl(dimethyl)silyl]oxy}phenyl)cyclohexyl ethylcarbamate

*N,N*-Di*is*opropylethylamine (199 µl) and ethyl isocyanate (90 µl) were added to a stirred solution of *trans*-4-(2,4-*bis*{[*tert*-butyl(dimethyl)silyl]oxy}phenyl)cyclohexanol (50 mg) in dry dichloroethane (1 ml) at room temperature under argon. The reaction mixture was heated to 40°C for 120h, and was partitioned between water (50 ml) and ethyl acetate (50 ml). The layers were separated and the aqueous layer was extracted with ethyl acetate (2 x 50 ml). The combined organic extracts were dried over magnesium sulfate and evaporated *in vacuo.* The residue was purified by flash column chromatography (SiO₂, ethyl acetate/petrol, 1:2 v/v) to give the title compound as a solid (55 mg, 95%); R_{f} (ethyl acetate/petrol, 1:2 v/v) 0.65.

### Intermediate 58

### trans-4-(2,4-Bis{[tert-butyl(dimethyl)silyl]oxy}phenyl)cyclohexyl cyclohexylcarbamate

*N,N*-Diisopropylethylamine (199 µl) and cyclohexyl isocyanate (128 µl) were added to a stirred solution of 4-(2,4-*bis*([*tert*-butyl(dimethyl)silyl]oxy}phenyl)cyclohexanol (50mg) in dry dichloroethane (1 ml) at room temperature under argon. The reaction mixture was heated to 40°C for 120 h, and was partitioned between water (50 ml) and ethyl acetate (50 ml). The layers were separated and the aqueous layer was extracted with ethyl acetate (2x50 ml). The combined organic extracts were dried over magnesium sulfate and evaporated *in vacuo*. The residue was purified by flash column chromatography (SiO₂, ethyl acetate/petrol, 1:2 v/v) to give the title compound as a pale yellow solid (30 mg, 47%); δ_{H} (CDCl₃) 0.12 (6H, s), 0.18 (6H, s), 0.90 (9H, s), 0.95 (9H, s). 1.00 - 2.10 (18H, m), 2.70 - 2.80 (1H, m), 3.36 - 3.50 (1 H, m), 4.40 - 4.48 (1H, m), 4.50 - 4.62 (1H, m), 6.20 (1 H, d), 6.32 (1 H, dd), 6.86 (1 H, d).

### Intermediate 59

### trans-4-(2,4-Dihydroxyphenyl)cyclohexanol

4-(2,4-Dihydroxyphenyl)cyclohexanone (18 mg) was placed in a round-bottomed flask equipped with magnetic stirrer. Ethanol (5 ml) was added, followed by sodium borohydride (3.3 mg), and the reaction mixture was stirred for 16 hr. Aqueous HCl (20 ml, 1M), followed by ethyl acetate (20 ml), was added, and the organic phase removed and washed with brine (15 ml), dried over anhydrous magnesium sulphate, filtered, and then concentrated *in vacuo.* The residue was purified by flash column chromatography (SiO₂, ethyl acetate/petroleum ether, 60:40, v/v) to afford the title compound (14 mg, 78%) as a white solid. δ_{H} (CD₃OD) 1.38-1.56 (4H, m), 1.85-1.88 (2H, m), 2.04 - 2.07 (2H, m), 2.80 (1 H, tt), 3.58 - 3.65 (1 H, m), 6.24-6.29 (2H, m), 6.90 (1H, d); m/z (ES⁺) 267 ((M+AcOH) - 1).

### Intermediate 60

### cis - 4-(2,4-Bis{[tert-Butyl(dimethyl)silyl]oxy}phenyl)cyclohexyl methanesulfonate

To a round bottom flask containing *cis*-4-(2,4-*bis*{[*tert*-butyl(dimethyl)silyl]oxy}phenyl) cyclohexanol (200 mg, 0.46 mmol) was added dichloromethane (10 ml) followed by triethylamine (96 µl, 0.69 mmol) and dimethylaminopyridine (catalytic amount). The flask was purged with argon and methane sulfonyl chloride (53 µl, 0.69 mmol) was added with stirring. Stirring was continued for a further 24 hr and the reaction mixture was poured into water and extracted with dichloromethane (4 x 20 ml). The combined organic extracts were washed with brine, dried over magnesium sulfate and concentrated *in vacuo*. Purification via flash column chromatography (SiO₂, ethyl acetate/petrol, 1:9) afforded the title compound as an oil (194 mg, 82%). δ_{H} (CDCl₃) 0.19 (6H, s), 0.22 (6H, s), 0.97 (9H, s), 1.01 (9H, s), 1.20 - 1.35 (2H, m), 1.65 - 1.78 (4H, m), 2.15 - 2.22 (2H, m), 2.85 - 2.95 (1H, m), 3.02 (3H, s), 5.06 (1H, s), 6.30 (1H, d), 6.43 (1H, dd), 7.00 (1H, d).

### Intermediate 61

### [4-(2,4-Bis{[tert-butyl(dimethyl)silyl]oxy}phenyl)cyclohexyl]methyl propionate

4-Dimethylaminopyridine (catalytic amount), triethylamine (68 µl) and propionyl chloride (42 µl) were added to a stirred solution of [4-(2,4-*bis*{[*tert*-butyl(dimethyl)silyl]oxy} phenyl) cyclohexyl]methanol in anhydrous dichloromethane (3 ml) at room temperature under argon. After 16 hr, the reaction mixture was partitioned between water (10 ml) and ethyl acetate (20 ml). The layers were separated and the aqueous layer was extracted with ethyl acetate (2 x 20 ml). The combined organic extracts were dried over magnesium sulfate and evaporated *in vacuo*. The residue was purified by flash column chromatography (SiO₂, ethyl acetate/petrol, 1:19 v/v) to give the title compound as a yellow oil and a mixture of diastereoisomers (114 mg, 92%); δ_{H} (CDCl₃) -0.04 (6H, s), 0.02 (6H, s), 0.96 (9H, s), 1.00 (9H, s), 1.08 - 1.18 (3H, m), 1.20 - 2.10 ( 9H, m), 2.24 - 2.40 (2H, m), 2.76 - 2.92 (1H, m), 3.94 (0.6H, d), 4.18 (0.4H, d), 6.26 (1 H, m), 6.40 (1H, dd), 6.94 - 6.98 (1 H, m).

### Intermediates 62 and 63

### Diastereoisomers of ethyl 4 - (2, 4 - bis{[tert - butyl(dimethyl)silyl]oxy}phenyl) - 1-hydroxycyclohexanecarboxylate

A round bottom flask equipped with stirrer bar was charged with tetrahydrofuran (6 ml), ethyl vinyl ether (0.28 ml, 2.92 mmol) and cooled to -78°C. A solution of *tert*-butyl lithium (1 ml, 1.7 M, 1.7 mmol) was added dropwise and the flask allowed to reach 0°C. The flask was maintained at this temperature until the bright yellow colour had been discharged before being re-cooled to -78 °C. A solution of 4 - (4 - {[*tert* - butyl(dimethyl)silyl]oxy}phenyl) cyclohexanone (250 mg, 0.58 mmol) in THF (4 ml) was added dropwise. After 30 min the reaction was quenched with wet THF (0.5 ml water in 5 ml of THF), poured into water (10 ml) and extracted with diethyl ether (3 x 10 ml). The combined organic extracts were washed with brine, dried over magnesium sulfate and concentrated *in vacuo*. This residue was dissolved in methanol (40 ml), cooled to -78 °C and ozonised oxygen passed through the solution for 10 min. After this time the excess ozone was removed by passing oxygen through the solution. The reaction mixture was allowed to warm to room temperature and concentrated *in vacuo.* Purification via flash column chromatography (SiO₂, ethyl acetate/petrol, 1:6) afforded the title compounds. First eluted diastereoisomer (31 mg, 11%); δ_{H} (CDCl₃) 0.18 (6H, s), 0.25 (6H, s), 0.97 (9H, s), 1.02 (9H, s), 1.30 (3H, t), 1.80 - 1.95 (8H, m), 2.82 - 2.95 (2H, m), 4.23 (2H, q), 6.28 (1H, d), 6.42 (1H, dd), 7.05 (1H, d). Second eluted diastereoisomer (32 mg, 12%); δ_{H} (CDCl₃) 0.20 (6H, s), 0.25 (6H, s), 0.95 (9H, s), 1.00 (9H, s), 1.36 (3H, t), 1.62 - 1.85 (6H, m), 2.10 - 2.22 (2H, m), 2.87 - 2.98 (2H, m), 4.28 (2H, q), 6.28 (1H, d), 6.42 (1H, dd), 6.97 (1H, d).

### Intermediate 64

### 4-{2, 4-Bis [tert-butyl(dimethyl)silyloxy]phenyl cyclohexanone oxime

To a round bottomed flask equipped with magnetic stirrer was added 4-(2, 4-*bis* [*tert*-butyl(dimethyl)silyloxy]phenyl cyclohexanone (100 mg, 0.23 mmol) and ethanol (5 ml). To the stirred suspension was added hydroxylamine hydrochloride (32 mg, 0.46 mmol) and triethylamine (103 µl, 0.74 mmol), then the solution was heated under reflux temperature for 2.5 hr. The solution was allowed to cool to room temperature then evaporated to dryness in vacuo. The residue was partitioned between ethyl acetate (20 ml) and water (10 ml) and the layers separated. The aqueous layer was extracted with ethyl acetate (3x20 ml) and the combined organic layers washed with brine (10 ml), dried over magnesium sulfate, filtered and concentrated *in vacuo.* The title compound was isolated (103 mg, 100 %) as a white. δ_{H} (CDCl₃) 0.19 (6H, s), 0.24 (6H, s), 0.95 (9H, s), 1.02 (9H, s), 1.43-1.62 (2H, m), 1.77-1.87 (1H, m), 1.93-2.04 (2H, m), 2.18-2.24 (1H, m), 2.46-2.53 (1H, m), 3.05-3.15 (1H, m), 3.41-3.50 (1H, m), 6.30 (1H, d), 6.40 (1H, dd), 6.79 (1H, br), 6.92 (1H, d).

### Example 1

### 4 - (2, 4 - Dihydroxyphenyl) - 3 - cyclohexen - 1 - one

A round bottomed flask equipped with magnetic stirrer was charged with 8 - [2, 4 - *bis*(methoxymethoxy)phenyl] - 1, 4 - dioxaspiro[4.5]dec - 7- ene (1.50 g, 4.24 mmol) and methanol (30 ml). To the stirred solution was added aqueous hydrochloric acid (30 ml, 1.0M) and the solution was heated to reflux temperature for 1.5 hr. After cooling to room temperature, saturated aqueous sodium bicarbonate (20 ml) was added and the layers separated. The aqueous layer was extracted with ethyl acetate (4x20 ml) and the combined organic layers washed with brine (20 ml), dried over magnesium sulfate, filtered and concentrated *in vacuo*. The resulting oil was purified *via* flash column chromatography (SiO₂, ethyl acetate/ petroleum ether, 1:3, v/v) to afford the title compound as a yellow solid (323 mg, 37%). m/z (ES⁻) 203 (M-H⁺); δ_{H}(CD₃OD) 2.62 (2H, t), 2.86 (2H, t), 3.04 (2H, m), 5.78 (1H, m), 6.28 (1H, m), 6.32 (1H, m), 6.96 (1H, d).

### Example 2a

### 4-(2, 4-Dihydroxyphenyl)cyclohexanone

A round bottomed flask equipped with magnetic stirrer was loaded 8-[2,4-*bis*(methoxymethoxy)phenyl]-1,4-dioxaspiro[4.5]decane (1.30 g, 3.9 mmol) and methanol (15 ml). To the resulting stirred solution was added aqueous HCl (15 ml, 1M) in one portion. After stirring for one hr at room temperature the acid was quenched by adding saturated aqueous sodium bicarbonate solution (10 ml). After stirring vigorously for 10 min, the reaction mixture was transferred to a separating funnel and the phases separated and the aqueous phase was extracted with ethyl acetate (3x20 ml). The combined organic phases were washed with brine and the solvent evaporated. To the slightly wet crude product was added methanol (30 ml) and acidic ion exchange resin (4 g). The resulting mixture was heated under reflux, with stirring, for 5 hr. Filtering through a plug of Celite, washing with ethyl acetate, followed by removal of solvent *in vacuo* afforded an orange oil. Purification by flash column chromatography, (SiO₂, ethyl acetate/petroleum ether, 1:1, v/v) furnished the title compound as a white powder (0.54 g, 68%). m/z (ES⁻) 411 (2M - 1); δ_{H} (CD₃OD) 1.94 (2H, ddd), 2.16 - 2.23 (2H, m), 2.41 (2H, dt), 2.62 (1H, t), 2.63 (1H, t), 6.24 (1H, dd), 6.31 (1H, d), 6.92 (1H, d).

### Example 2b

### 4 - (2, 4 - Dihydroxyphenyl)cyclohexanone

A round bottomed flask equipped with magnetic stirrer was charged with 4 - (1, 4-dioxaspiro[4.5]dec - 8 - yl) - 1, 3 - benzenediol (11.3 g, 45.2 mmol), acetone (250 ml) and water (50 ml). To the stirred solution was added pyridinium *p* - toluenesulfonate (1.14 g, 4.52 mmol) in one portion and the reaction mixture was then heated to reflux temperature for 8 hr. After allowing the reaction mixture to coot to room temperature, most of the acetone was removed *in vacuo* and the remaining mixture was partitioned between ethyl acetate (200 ml) and water (50 ml). The aqueous layer was extracted with ethyl acetate (3x50 ml) and the combined organic layers were washed with brine (30 ml), dried over magnesium sulfate, filtered and concentrated under reduced pressure to afford an off white powder. After washing the powder with dichloromethane (100 ml) and removal of excess solvent under reduced pressure, the desired product (9.30 g, 100%) was obtained as an off-white white powder. m/z (ES⁺) 207 (M+H⁺); δ_{H}(CD₃OD) 1.84 - 1.97 (2H, m), 2.15 - 2.23 (2H, m), 2.36 - 2.45 (2H, m), 2.58 - 2.68 (2H, m), 3.39 (1H, tt), 6.26 (1H, dd), 6.34 (1H, d), 6.96 (1H, d).

### Example 3

### 4-(2, 4-Dihydroxyphenyl)cyclohexanone oxime

To a round bottomed flask, equipped with magnetic stirrer was added 4-(2, 4-dihydroxyphenyl)cyclohexanone (100 mg, 0.49 mmol), anhydrous ethanol (5 ml), triethylamine (102 ml) and hydroxylamine hydrochloride (51 mg, 0.73 mmol). The reaction mixture was heated under reflux for 3 hr, then evaporated *in vacuo.* The resulting solid was purified by flash column chromatography, (SiO₂, ethyl acetate/petroleum ether, 35:65, v/v), furnishing the title compound as short white needles (107 mg, 100%). δ_{H} (CD₃OD) 1.44 - 1.61 (2H, m), 1.81 - 1.88 (1H, m), 1.94 - 2.00 (2H, m), 2.19 - 2.27 (1H, m), 2.43 (1H, d), 3.04 - 3.10 (1 H, m), 3.38 (1H, m), 6.22 - 6.25 (1H, m), 6.28 (1H, d), 6.84 - 6.86 (1H, m); m/z (El⁻) 220.

### Example 4

### O-Methyl-4-(2, 4-dihydroxyphenyl)cyclohexanone oxime

To a round bottomed flask, equipped with magnetic stirrer was added 4-(2, 4-dihydroxyphenyl)cyclohexanone (21 mg, 0.10 mmol), anhydrous ethanol (3 ml), sodium acetate (16 mg, 0.20 mmol) and *O*-methythydroxylamine hydrochloride (9 mg, 0.22 mmol). The reaction mixture was heated under reflux for 6 hr, then partitioned between ethyl acetate (10 ml) and water (10 ml). The organic layer was dried over anhydrous calcium sulphate, filtered and evaporated *in vacuo* furnishing a colourless oil. Purification via flash column chromatography (SiO₂, ethyl acetate/petroleum ether, 1:1, v/v), afforded the title compound as a white solid (11 mg, 47%). δ_{H} (CD₃OD) 1.43 - 1.63 (2H, m), 1.81 - 1.92 (2H, m), 1.93 - 2.20 (2H, m), 2.24 (1H, dt), 2.38 - 2.44 (1H, m), 3.07 (1H, tt), 3.78 (3H, s), 6.20 - 6.23 (1H, m), 6.26 (1H, d), 6.85 (1H, d); m/z (ES⁻) 234.

### Example 5

### O-Benzyl-4-(2, 4-dihydroxyphenyl)cyclohexanone oxime

To a round bottomed flask, equipped with magnetic stirrer, was added 4-(2,4-dihydroxyphenyl)cyclohexanone (21 mg, 0.10 mmol), anhydrous ethanol (3 ml), sodium acetate (17 mg, 0.21 mmol) and O-benzylhydroxylamine hydrochloride (18 mg, 0.21 mmol). The reaction mixture was heated under reflux for 6 hr, then partitioned between ethyl acetate (10 ml) and water (10 ml). The organic layer was dried over anhydrous calcium sulphate, filtered and evaporated *in vacuo* furnishing a pale pink oil which was purified by flash column chromatography (SiO₂, ethyl acetate/petroleum ether, 1:1, v/v) to afford the title compound as a colourless oil (11 mg, 32%). δ_{H} (400, CDCl₃) 1.47 - 1.68 (2H, m), 1.86 - 1.95 (1H, m), 1.97 - 2.08 (2H, m), 2.25 (1H, dt), 2.49 - 2.57 (1 H, m), 3.02 (1H, tt), 3.42 - 3.50 (1H, m), 4.78 (1H, s), 4.89 (1H, s), 6.25 - 6.29 (1H, m), 6.33 - 6.37 (1H, m), 6.94 (1H, d), 7.26 - 7.39 (5H, m); m/z (ES⁻) 310.

### Example 6

### 3-(2,4-dihydroxyphenyl)-2-cyclohexen-1-one

3-[2,4-*Bis*(methoxymethoxy)phenyl]-2-cyclohexen-1-one (50 m g) was heated to 50°C in methanol (4 ml) containing acidic ion exchange resin (500 mg). After 2 hr, the mixture was filtered and the filtrate was evaporated *in vacuo* and purified by flash column chromatography (SiO₂, ether/petroleum ether, 9:1, v/v) to furnish the title compound as a yellow solid (31 mg, 76%). δ_{H} (DMSO) 1.95 (2H, quintet), 2.30 (2H, t), 2.69 (2H, t), 6.26 (2H, overlapping m), 6.35 (1H, m), 7.10 (1H, d), 9.67 (1H, bs), 9.86 (1H, bs); m/z (ES⁻) 407 (2M-H)⁻.

### Example 7

### (±)-3-(2,4-Dihydroxyphenyl)cyclohexanone

(±)-3-[2,4-*Bis*(methoxymethoxy)phenyl]cyclohexanone (35 mg) in methanol (4 ml) containing acidic ion exchange resin (300 mg) was stirred at 50°C for 6 hr, and then at ambient temperature for 16 hr. The mixture was evaporated *in vacuo* and the residue was redissolved in acetone (4 ml) containing water (2 drops) and stirred at 50°C for 8 hr, then at ambient temperature for 64 hr. The mixture was filtered through celite and purified by flash column chromatography (SiO₂, petroleum ether/ethyl acetate 1:1 v/v) to furnish the title compound as a white solid containing an equilibrium mixture of cyclized and uncyclized forms (31 mg, 76%). δ_{H} (DMSO) 1.1 - 2.3 (8H, overlapping m), 3.0 (1H, m), 6.08 (0.5H, bs), 6.16 (1H, bd), 6.25 (0.5H, bs), 6.53 (0.5H, bs), 6.75 (0.5H, d), 6.89 (0.5H, b), 8.98 (0.5H, bs), 9.02 (0.5H, bs), 9.17 (0.5H, bs); m/z (ES⁻) 205 (M-H)⁻.

### Example 8

### 3-(2,4-Dihydroxyphenyl)-2-cyclohexen-1-one oxime

3-[2,4-*Bis*(methoxymethoxy)phenyl]-2-cyclohexen-1-one oxime (0.1 g) was heated to 50°C in methanol (5 ml) containing acidic ion exchange resin (0.3 g). After 4hr, the mixture was filtered and the resin was washed with ammonia solution (50 ml). Filtrate and washings were combined, evaporated *in vacuo* and purified by flash column chromatography (SiO₂, ethyl acetate/petroleum ether, 3:2, v/v) to furnish the title compound as a yellow solid (0.058 g, 81%). δ_{H} (DMSO) (mixture of stereoisomers) 1.68 (2H, m, major), 1.76 (2H, m, minor), 2.26 (2H, m, minor), 2.40-2.54 (4H major + 2H minor, overlapping m), 6.15-6.30 (3H major + 2H minor, overlapping m), 6.85-6.95 (1H major + 2H minor, overlapping m), 9.26 (1H, bs, major), 9.32 (1H, bs, minor), 9.35 (1H, bs, major), 9.40 (1H, bs, minor), 10.17 (1H, s, minor), 10.49(1H, s, major); m/z (ES⁻) 437 (2M-H)⁻.

### Example 9

### (±)-3-(2,4-Dihydroxyphenyl)cyclohexanone oxime

(±)-3-(2,4-Dihydroxyphenyl)cyclohexanone (13 mg), hydroxylamine hydrochloride (0.0065 g), and triethylamine (16 µl) were heated at 80°C in DMF (3 ml). After 3 hr, the reaction mixture was partitioned between ethyl acetate and water. The organic layer was washed with brine, dried over magnesium sulfate, and evaporated *in vacuo*. The residue was purified by flash column chromatography (SiO₂, petroleum ether/ethyl acetate 3:2 v/v) to furnish the title compound as a white solid (12 mg, 86%). δ_{H} (CD₃OD) 1.4- 2.0 (6H, overlapping m), 2.10 (0.5H, m), 2.20 (0.5H, m), 2.35 (0.5H, m), 2.46 (0.5H, m), 2.94 (1 H, m), 6.22-6.26 (2H, overlapping m), 6.92-6.95 (1 H, overlapping m); m/z (ES⁺) 222 (M+H)⁻.

### Example 10

### (±)-4-[3-(1-Piperazinyl)cyclohexyl]-1,3-benzenediol trifluoroacetic acid salt

(±)-1-{3-[2,4-*Bis*(methoxymethoxy)phenyl]cyclohexyl}piperazine (35 mg) was heated under reflux in methanol containing acidic ion exchange resin (0.3 g) for 5 hr. The reaction mixture was filtered, and the resin was washed with methanol and aqueous ammonia, and the combined filtrate and washings were concentrated *in vacuo.* The crude residue was purified by preparative HPLC to furnish the title compound as an off white solid (14 mg, 38%). δ_{H} (d₄-MeOH) 1.7 - 1.9 (6H, overlapping m), 2.05 (1H, m), 2.20 (1H, m), 3.1 - 3.5 (10H, overlapping m), 6.26 (2H, overlapping m), 6.94 (1H, d); m/z (ES⁺) 277 (M+H)⁺.

### Example 11

### (±)-N-[3-(2,4-Dihydroxyphenyl)cyclohexyl]methanesulfonamide

(±)-*N*-{3-[2,4-*Bis*(methoxymethoxy)phenyl]cyclohexyl}methanesulfonamide (34 mg) was heated under reflux in methanol (2 ml) containing acidic ion exchange resin (0.3 g) for 5 hr. The reaction mixture was filtered and the resin was washed with methanol. The combined filtrate and washings were evaporated *in vacuo* and the crude residue was purified by preparative HPLC to furnish a mixture of diastereoisomers of the title compound as a pale pink solid (10 mg, 38%). δ_{H} (d₄-MeOH) 1.20 - 2.00 (7H, overlapping m), 2.07 (1H, m), 2.90 (0.5H, m), 2.93 (1.5H, s), 2.97 (1.5H, s), 3.13 (0.5H, m), 3.30 (0.5H, m), 3.80(0.5H, m), 6.21 - 6.26 (1H, dd), 6.87 (0.5H, d), 6.89 (0.5H, d); m/z (ES⁻) 284 (M-H)⁻.

### Example 12

### (±)-4-[3-(Hydroxymethyl)cyclohexyl]-1,3-benzenediol

(±)-{3-[2,4-*Bis*(methoxymethoxy)phenyl]cyclohexyl}methanol (35 mg) was heated at 50°C in methanol (3.5 ml) containing acidic ion exchange resin (350 mg). After 6 hr, the mixture was filtered, the resin was washed with ethyl acetate, and the combined filtrate and washings were evaporated *in vacuo.* The crude residue was purified by flash column chromatography (SiO₂, ethyl acetate/petrol, 1:1 v/v) to furnish a mixture of diastereoisomers of the title compound as a white solid (2 mg, 8%). δ_{H} (d₄-MeOH) 0.9 - 1.9 (9H, overlapping m), 2.80 (0.5H, m), 2.90 (0.5H, m), 3.20 - 3.40 (2H, overlapping m), 6.15 (2H, m), 6.80 (0.5H, d), 6.85 (0.5H, d); m/z (ES⁻) 221 (M-H)⁻.

### Example 13

### (±)-4-[3-(Hydroxyamino)cyclohexyl]-1,3-benzenediol trifluoroacetate salt

*Cis*-*N*-{3-[2,4-*bis*(methoxymethoxy)phenyl]cyclohexyl}hydroxylamine (0.015 g) was heated under reflux in methanol (2 ml) containing acidic ion exchange resin (0.3 g) for 5 hr. The reaction mixture was filtered, and the resin was washed with aqueous ammonia and methanol and the combined filtrate and washings were concentrated *in vacuo*. The crude residue was purified by preparative HPLC to furnish the title compound as an off white solid (0.005 g, 46%). δ_{H} (d₄-MeOH) 1.25 - 1.60 (4H, overlapping m), 1.82 (1H, bd), 2.02 (1H, m), 2.17 (2H, overlapping m), 2.93 (1H, m), 3.36 (1H, m) 6.24 (1H, dd), 6.27 (1 H, d), 6.88 (1 H, d); m/z (ES⁺) 224 (M+H)⁺.

### Example 14

### 4-(4-Methylenecyclohexyl)-1,3-benzenediol

Tetrabutylammonium fluoride (230 µl) was added to a stirred solution of tert-butyl-[3-{[*tert*-butyl(dimethyl)silyl]oxy}-2-(4-methylenecyclohexyl)phenoxy](isopropyl)dimethylsilane (40 mg) in tetrahydrofuran (2 ml) at room temperature. After 24 hr, further tetrabutylammonium fluoride (50 µl) was added, and after 2 hr the solvent was removed under reduced pressure. The residue was partitioned between water (20 ml) and ethyl acetate (20 ml). The layers were separated and the aqueous layer was extracted with ethyl acetate (2x 20 ml). The combined organic extracts were washed with brine, dried over magnesium sulfate and evaporated *in vacuo*. The residue was purified by flash column chromatography (SiO₂, ethyl acetate/petrol, 2:3 v/v) to give the title compound as a white solid (17 mg, 90%). δ_{H} (CD₃OD) 1.25 - 1.40 (2H, m), 1:75 - 1.82 (2H, m), 2.04 - 2.15 (2H, m), 2.22 - 2.30 (2H, m), 2.86 (1H, tt), 3.20 (1H, m), 4.50 (2H, s), 6.10 - 6.16 (2H, m), 6.72 (1H, d); m/z (ES⁺) 205 (M+H)⁺.

### Example 15

### cis / trans-4-[4-(Hydroxymethyl)cyclohexyl]-1,3-benzenediol

*cis* / *trans*-[4-(2,4-*Bis*{[*tert*-butyl(dimethyl)silyl]oxy}phenyl)cyclohexyl]methanol (24 mg) was dissolved in THF (5 ml), and tetrabutylammonium fluoride (0.12 ml, 1.0M in THF) was added. The resulting solution was stirred at room temperature for 15 hr and then partitioned between ethyl acetate (20 ml) and water (2 ml). The aqueous phase was extracted with ethyl acetate (3x20 ml) and the combined organic phases were washed with brine (20 ml), dried over anhydrous magnesium sulphate, and concentrated *in vacuo*. Purification *via* flash column chromatography (SiO₂ eluting with ethyl acetate:petroleum ether, 1:1 v/v) furnished the desired compound (7 mg, 59%) as a white solid. δ_{H} (CD₃OD): 0.95 - 1.06 (0.5H, m), 1.24 - 1.38 (0.5H, m), 1.43 - 1.60 (4H, m), 1.69 - 1.83 (4H, m), 2.62 - 2.77 (1 H, m), 3.30 (1H, d), 3.56 (1H, d), 6.11 - 6.17 (2H, m), 6.76 - 7.01 (1H, m); m/z (ES⁻) 281 (M - 1 + 60)⁻.

### Example 16

### cis / trans-4-(4-Hydroxy-4-methylcyclohexyl)-1,3-benzenediol

*cis*/*trans*-4-(2,4-*Bis*{[*tert*-butyl(dimethyl)silyl]oxy}phenyl)-1-methylcyclohexanol (29 mg) was dissolved in THF (8 ml), and tetrabutylammonium fluoride (0.14 ml, 1.0M in THF) was added in one portion with stirring. The resulting solution was stirred for 17 hr and then partitioned between ethyl acetate (30 ml) and water (5 ml). The aqueous phase was extracted with ethyl acetate (2x10 ml), and the combined organic phases washed with brine (20 ml), dried over anhydrous magnesium sulphate, and concentrated *in vacuo*. Purification *via* flash column chromatography (SiO₂ eluting with ethyl acetate:petroleum ether, 1:19 v/v gradually increasing polarity to 3:7 v/v) afforded the title product as the individual 1,4-*cis* (5 mg, 36%) and 1,4-*trans* (9 mg, 64%) diastereoisomers (white solids). *Cis* isomer: δ_{H} (CD₃OD): 1.30 (3H, s), 1.50 - 1.66 (4H, m), 1.69 - 1.77 (4H, m), 2.74 - 2.82 (1H, m), 6.20 - 6.26 (2H, m). 6.89 (1H, d); m/z (ES⁻) 281 (M - 1 +60). Trans isomer: δ_{H} (CD₃OD): 1.22 (3H, s), 1.46 - 1.60 (4H, m), 1.69 - 1.82 (4H, m), 2.75 (1H, tt), 6.22 - 6.26 (2H, m), 6.93 (1 H, d); m/z (ES⁻) 281 (M - 1 + 60).

### Example 17

### cis / trans - N - [4 - (2, 4 - Dihydroxyphenyl)cyclohexyl]acetamide

To a round bottomed flask equipped with magnetic stirrer was added *cis* / *trans* - *N*-[4 - (2, 4 - *bis*{[*tert*-butyl(dimethyl)silyl]oxy}phenyl)cyclohexyl] acetamide (15 mg, 31 µmol), tetrahydrofuran (3 ml) and tetra-*n*-butylammonium fluoride (93 µl, 93 µmol, 1.0M solution in tetrahydrofuran). The resulting solution was stirred for 3 hr. Tetra-*n*-butylammonium fluoride (90 µl, 90 µmol, 1.0M solution in tetrahydrofuran) was added and the solution stirred for a further 64 hr. Saturated aqueous sodium bicarbonate solution (3 ml) was added and the solvents removed *in vacuo*. The residue was partitioned between ethyl acetate (20 ml) and water (5 ml) and the aqueous layer extracted with ethyl acetate (3x10 ml). The combined organic layers were washed with brine (10 ml), dried over magnesium sulfate, filtered and concentrated *in vacuo*. Purification via flash column chromatography (SiO₂, methanol /dichloromethane, 1:9, v/v) afforded an oily solid (6 mg) which was a mixture of isomers by NMR. Further purification *via* HPLC afforded the title compound (0.5 mg, 6%) as a mixture of diastereoisomers and a colourless oil, m/z (ES⁻) 308 (M-1+60{acetate}); δ_{H}(CD₃OD) 1.41 (1H, m), 1.57 (1H, m), 1.70 (2H, m), 1.83 (1H, m), 1.87 (1H, m), 1.93 (1H, m), 1.96 (1.5H, m), 2.04 (1.5H, m), 2.05 (1H, m), 2.82 (1H, m), 3.72 (0.5H, m), 4.14 (0.5H, m), 6.28 (2H, m), 6.92 (0.5H, dd), 6.97 (0.5H, dd).

### Example 18

### (±)-O-Methyl-3-(2,4-dihydroxyphenyl)cyclohexanone oxime

(±)-3-(2,4-Dihydroxyphenyl)cyclohexanone (22 mg), methoxylamine hydrochloride (18 mg) and sodium acetate (18 mg) were heated under reflux in ethanol. After 6 hr, further methoxylamine (36 mg) and sodium acetate (36 mg) were added and the mixture heated under reflux for a further 1 hr. The reaction mixture was evaporated *in vacuo* and the residue was partitioned between ethyl acetate (20 ml) and water (20 ml). The aqueous layer was extracted with ethyl acetate (2x20 ml), and the combined organic extracts were dried over magnesium sulfate and evaporated *in vacuo*. The crude residue was purified by flash column chromatography (SiO₂, ethyl acetate/petrol, 1:1 v/v) to furnish the title compound as a mixture of isomers (18 mg, 72%). δ_{H} (CDCl₃) 1.5 - 2.2 (6H, overlapping m), 2.42 (0.5H, bd), 2.69 (0.5H, bd), 2.92 (0.5H, m), 3.06 (0.5H, m), 3.24 - 3.38 (1H, m), 3.81 (1.5H, s), 3.88 (1.5H, s), 5.42 (0.5H, bs), 5.47 (0.5H, bs), 6.28 - 6.42 (2H, overlapping m), 6.86 (0.5H, bs), 6.98 (1H, m), 7.06 (0.5H, bs); m/z (ES-) 469 (2M-1)⁻.

### Example 19

### (±)-3-(2,4-Dihydroxyphenyl)-1-methylcyclohexanol

Methyl magnesium chloride (0.132 ml of a 22% w/w solution in tetrahydrofuran) was added to a solution of (±)-3-(2,4-dihydroxyphenyl)cyclohexanone (20 mg) in tetrahydrofuran (3 ml) at 0°C under argon. After 16 hr, dilute hydrochloric acid (1 ml) was added dropwise and the reaction mixture was partitioned between ethyl acetate (50 ml) and brine (50 ml). The aqueous layer was extracted with ethyl acetate (2x50 ml), and the combined organic extracts were dried over magnesium sulfate and evaporated *in vacuo.* The crude residue was purified by preparative HPLC to furnish the title compound as a white solid (6 mg, 28%). δ_{H} (d₄-MeOH) 1.20 (3H, s), 1.22 - 1.39 (2H, overlapping m), 1.46 (1H, t), 1.54 - 1.87 (5H, overlapping m), 3.22 (1H, m), 6.19 - 6.25 (2H, overlapping m), 6.86 91 H, d); m/z (ES⁻) 281 (M+60-H)⁻.

### Example 20

### (±)-O-Benzyl-3-(2,4-dihydroxyphenyl)cyclohexanone oxime

(±)-3-(2,4-Dihydroxyphenyl)cyclohexanone (30 mg), O-benzylhydroxylamine hydrochloride (46 mg) and sodium acetate (24 mg) were heated under reflux in ethanol (3 ml). After 16 hr, the reaction mixture was evaporated *in vacuo* and the residue was partitioned between ethyl acetate (50 ml) and brine (50 ml). The aqueous layer was extracted with ethyl acetate (2x50 ml), and the combined organic extracts were dried over magnesium sulfate and evaporated *in vacuo.* The crude residue was purified by preparative HPLC to furnish a mixture of geometric isomers of the title compound as an off white solid (12 mg, 26%). δ_{H} (d₄-MeOH) 1.42 - 2.06 (6H, overlapping m), 2.12 (0.5H, dt), 2.22 (0.5H, t), 2.34 (0.5H, m), 2.46 (0.5H, m), 2.96 (1H, m), 5.02 (2H, s), 6.20 - 6.27 (2H, overlapping m), 6.92 (1H, m), 7.22-7.34 (5H, overlapping m); m/z (ES⁻) 310 (M-H)⁻.

### Example 21

### 3-(2,4-Dihydroxyphenyl)-2-cyclopentenone oxime

3-[2,4-*Bis*(methoxymethoxy)phenyl]-2-cyclopenten-1-one (20 mg) was heated at 50°C in MeOH (4 ml) containing acidic ion exchange resin (100 mg) for 3 hr. The reaction mixture was filtered and the resin washed with ethyl acetate (20 ml). The filtrate was concentrated in *vacuo* and the residue purified by flash column chromatography (SiO₂, ethyl acetate/petrol, 7:3 v/v) to furnish 3-(2,4-dihydroxyphenyl)-2-cyclopenten-1-one (11 mg, 79%). 3-(2,4-Dihydroxyphenyl)-2-cyclopenten-1-one (6 mg), hydroxylamine hydrochloride (3.3 mg) and triethylamine (6.6 µl) were heated in ethanol (3 ml) under reflux for 3 hr. The reaction mixture was partitioned between ethyl acetate (20 ml) and water (20 ml). The aqueous phase was extracted with ethyl acetate (10 ml), and the combined organic phases were washed with brine (10 ml), dried over magnesium sulfate, and concentrated *in vacuo.* The crude residue was purified by flash column chromatography (SiO₂, ethyl acetate/petrol, 1:1 v/v) to furnish the title compound as a yellow solid and as one major isomer (4 mg, 62%). Data reported for the major isomer: δ_{H} (d⁴-MeOH) 2.74-2.77 (2H, m), 2.96-2.99 (2H, m), 6.34 (1H, dd), 6.38 (1H, d), 6.96 (1H, t), 7.22 (1H, d); m/z (ES⁻) 204 (M-H)⁻.

### Example 22

### (±)-3-(2,4-Dihydroxyphenyl)cyclopentanone

(±)-3-[2,4-*Bis*(methoxymethoxy)phenyl]cyclopentanone (8 mg) was heated at 50°C in MeOH (3 ml) containing acidic ion exchange resin (0.1 g) for 3 hr. The reaction mixture was filtered and the resin washed with ethyl acetate (20 ml). The filtrate was concentrated in-*vacuo* and the residue purified by flash column chromatography (SiO₂, ethyl acetate) to furnish the title compound as a white solid (3.8 mg, 70%). δ_{H} (d⁴-MeOH) 2.52-2.13 (1H, m), 2.26-2.48 (4H, overlapping m), 2.53-2.60 (1H, m), 3.55-3.61 (1H, m), 6.29 (1 H, dd), 6.33 (1H, d), 6.96 (1H, d); m/z (ES⁻) 251 ((M + 60)-1)⁻.

### Example 23

### (±)-3-(2,4-Dihydroxyphenyl)cyclopentanone oxime

(±)-3-(2,4-Dihydroxyphenyl)cyclopentanone (5 mg), hydroxylamine hydrochloride (2.7 mg) and triethylamine (5.4µM) were heated in EtOH (4 ml) under reflux for 3 hr. The reaction mixture was partitioned between ethyl acetate (20 ml) and water (20 ml). The aqueous phase was extracted with ethyl acetate (10 ml), and the combined organic phases were washed with brine (10 ml), dried over magnesium sulfate, and concentrated *in vacuo*. The crude residue was purified by flash column chromatography (SiO₂, ethyl acetate/petrol, 4:1 v/v) to furnish the title compound as a white solid and a mixture of isomers (3.8 mg, 71%). δ_{H} (d⁴-MeOH) 1.80-1.93 (1 H, overlapping m), 2.05-2.20 (0.5H, m), 2.39-2.55 (3H, overlapping m), 2.68-2.74 (1H, overlapping m), 2.94 (0.5H, br dd), 6.26-6.33 (2H, overlapping m), 6.94 (0.5H, d), 6.96 (0.5H, d); m/z (ES⁻) 266 ((M + 60)-1)⁻.

### Example 24

### cis - N - [4 - (2, 4 - Dihydroxyphenyl)cyclohexyl] - 1 - butanesulfonamide

To a round bottomed flask equipped with magnetic stirrer charged with *cis-*4-(2, 4 - *bis*{[*tert*-butyl(dimethyl)silyl]oxy}phenyl)cyclohexylamine (150 mg, 0.34 mmol) and 1, 2-dichloroethane (8 ml), was added triethylamine (96 µl, 0.70 mmol) and *n*-butylsulfonyl chloride (55 µl, 0.40 mmol) at room temperature. 4-Dimethylaminopyridine (3 crystals) was added and the mixture stirred for 17 hr. Aqueous sodium hydroxide solution (15 ml, 0.40M) was added and the mixture stirred for 10 min. The layers were partitioned and the aqueous layer was extracted with dichloromethane (15 ml). The combined organic layers were washed with brine (15 ml), dried over magnesium sulfate, filtered and concentrated *in vacuo.* The resulting solid was then dissolved in tetrahydrofuran (10 ml) and acetic acid (0.15 ml), then tetra-n-butylammonium fluoride hydrate (360 mg, 1.4 mmol) was added. The mixture was stirred at room temperature for 1 hr, then ethyl acetate (10 ml) and water (15 ml) were added. The layers were partitioned and the aqueous layer was extracted with ethyl acetate (10 ml). The combined organic layers were washed with brine (10 ml), dried over magnesium sulfate, filtered and concentrated *in vacuo* to leave an oil. Purification via flash column chromatography (SiO₂, ethyl acetate / petroleum ether, 1:10 then 1:1, v/v) furnished the title compound (40 mg, 35%) as a white solid, m/z (ES⁻) 326 (M - H⁺); δ_{H}(CD₃OD) 0.95 (3H, t), 1.45 - 1.55 (2H, m), 1.55 - 1.95 (10H, m), 2.80 - 2.90 (1 H, m), 3.00 - 3.20 (2H, m), 3.65 (1 H, m), 6.22 - 6.26 (2H, m), 6.96 (1 H, d).

### Example 25

### trans - N - [4 - (2, 4 - Dihydroxyphenyl)cyclohexyl]methanesulfonamide

To a round bottomed flask equipped with magnetic stirrer was added *trans* - *N -* [4 - (2, 4 - *bis*{[*tert*-butyl(dimethyl)silyl]oxy}phenyl)cyclohexyl] methanesulfonamide (320 mg, 0.57 mmol) and 1, 2-dichloroethane (50 ml). To the stirred solution was added trifluoroacetic acid (20 ml) and water (20 ml). The stirred reaction mixture was then heated under reflux for 18 hr and then cooled to room temperature. Toluene (70 ml) was added and the solvent removed in vacuo. Methanol (50 ml) was then added to the residue and the solvent removed under reduced pressure. The resulting oil was purified *via* flash column chromatography (SiO₂, ethyl acetate / petroleum ether, 1:3, 1 :2, then 1:1 v/v) to furnish the title product (115 mg, 71 %) as a white solid. m/z (ES⁺) 286 (M+H⁺); δ_{H}(CD₃OD) 1.52 (4H, m), 1.89 (2H, m), 2.13 (2H, m), 2.80 (1H, m), 3.00 (3H, s), 3.28 (1H, m), 6.27 (1H, d), 6.29 (1H, dd), 6.92 (1H, d).

### Example 26

### cis - N - [4 - (2, 4 - Dihydroxyphenyl)cyclohexyl]methanesulfonamide

To a round bottomed flask equipped with magnetic stirrer was added *cis - N -* [4 - (2, 4 - *bis*{[*tert*-butyl(dimethyl)silyl]oxy}phenyl)cyclohexyl]methanesulfonamide (44 mg, 100 µmol) and 1, 2-dichloroethane (4 ml). To the stirred solution was added methanesulfonyl chloride (10 µl, 120 µmol), triethylamine (28 µl, 200 µmol) and three crystals of 4-dimethylaminopyridine. The reaction mixture was then left stirring for 17 hr. The reaction mixture was then partitioned between aqueous sodium hydroxide (5 ml, 0.2M), and dichloromethane (5 ml). The aqueous phase was extracted with dichloromethane (2x5 ml) and the combined organic phases were washed with brine (7 ml), dried over magnesium sulfate, filtered and concentrated *in vacuo* to give a gum. This gum was dissolved in dichloromethane (6 ml), then water (3 ml) and trifluoroacetic acid (3 ml) were added and the mixture left to stir for 17 hr. The reaction mixture was diluted with toluene (15 ml) and the solvents were removed *in vacuo.* More toluene (15 ml) was added and evaporated under reduced pressure. Azeotropic removal of residual trifluoroacetic acid was effected with methanol to give a gum (38 mg). The residue was dissolved in dichloromethane (4.5 ml) and methanol (4.5 ml), then water (3 ml) and trifluoroacetic acid (3 ml) were added. The reaction mixture was stirred at room temperature for 64 hr. The reaction mixture was diluted with toluene (15 ml) and the solvents were removed *in vacuo.* More toluene (15 ml) was added and evaporated under reduced pressure. Azeotropic removal of residual trifluoroacetic acid was effected with methanol to give an oil (28 mg) which was purified via flash column chromatography (SiO₂, ethyl acetate / petroleum ether, gradient elution using 1:3, 1:2, then 1:1 v/v) to afford the title compound (13 mg, 81%) as a white solid. m/z (ES⁺) 286 (M+H⁺); δ_{H}(CD₃OD) 1.71 (6H, m), 1.90 (2H, m), 2.87 (1H, m), 3.00 (3H, s), 3.72 (1H, m), 6.28 (1H, d), 6.30 (1 H, dd), 7.01 (1H, d).

### Example 27

### 4 - [4 - (4 - Hydroxyphenyl)cyclohexyl] - 1, 3 - benzenediol

To a round bottomed flask equipped with magnetic stirrer was added *tert*-butyl(4 - {4-[2, 4 - *bis*(methoxymethoxy)phenyl]cyclohexyl}phenoxy)dimethylsilane (118 mg, 0.24 mmol), methanol (10 ml) and acidic Dowex® resin (500 mg). The reaction mixture was heated to reflux temperature for 5 hr then cooled to room temperature and filtered through a celite plug, washing with ethyl acetate. The filtrate was adsorbed onto silica gel and purified *via* flash column chromatography (SiO₂, ethyl acetate / petroleum ether, 2:3, v/v) to afford a white solid (44 mg) which was purified further by HPLC. The title compound was isolated as a white solid (12 mg, 17%). m/z (ES⁻) 283 (M-H⁺); δ_{H}(CD₃OD) 1.52 - 1.96 (6H, m), 2.11 (2H, m), 2.53 and 3.07 (1H, m), 2.90 (1H, m), 6.28 (2H, m), 6.75 (2H, m), 6.95 (1H, m), 7.10 (1H, m), 7.19 (1H, m).

### Example 28

### cis / trans - Methyl [4 - (2, 4 - dihydroxyphenyl)cyclohexyl]acetate

To a round bottomed flask equipped with magnetic stirrer was added *cis* / *trans -* methyl {4 - [2, 4 - *bis*(methoxymethoxy)phenyl]cyclohexyl} acetate (1.00 g, 2.84 mmol) and methanol (20 ml). The stirred solution was heated to reflux temperature and aqueous hydrochloric acid (20 ml, 1M) was added in aliquots (4x5ml) at 10 min intervals. After 2 hr, the reaction mixture was cooled to room temperature and saturated aqueous sodium bicarbonate (50 ml) added. The reaction mixture was poured into a separating funnel containing ethyl acetate (100 ml) and water (30 ml). The layers were separated and the aqueous layer was extracted with ethyl acetate (3x30 ml). The combined organic layers were washed with brine (20 ml), dried over magnesium sulfate, filtered and concentrated *in vacuo* to give a white solid. Purification *via* flash column chromatography (SiO₂, ethyl acetate /petroleum ether, 1:2, v/v) afforded the title compound (0.51 g, 69%) as a mixture of diastereoisomers. m/z (ES⁺) 265 (M+H⁺); δ_{H}(CD₃OD) 1.33-1.91 (9H, m), 2.30 (2H, m), 2.79 (1H, m), 3.72 (3H, s), 6.28 (2H, m), 6.95 (1 H, m).

### Example 29

### trans - Methyl [4 - (2, 4 - dihydroxyphenyl)cyclohexyl]acetate

*cis* / *trans -* Methyl [4 - (2, 4 - dihydroxyphenyl)cyclohexyl]acetate (25 mg) was purified via HPLC (acetonitrile / water, 30:70 - 80:20, 20 min isocratic) to afford the title compound as a white solid. m/z(ES⁺) 265 (M+H⁺); δ_{H}(CD₃OD) 1.21 (2H, m), 1.47 (2H, m), 1.64 (1 H, m), 1.88 (4H, m), 2.29 (2H, d), 2.79 (1 H, m), 3.70 (3H, s), 6.28 (2H, m), 6.92 (1 H, d).

### Example 30

### cis - Methyl [4 - (2, 4 - dihydroxyphenyl)cyclohexyl]acetate

*cis* / *trans -* Methyl [4 - (2, 4 - dihydroxyphenyl)cyclohexyl]acetate (25 mg) was purified via HPLC (acetonitrile / water, 30:70 - 80:20, 20 min isocratic) to afford the title compound as a white solid. m/z(ES⁺) 265 (M+H⁺); δ_{H}(CD₃OD) 1.60 -1.79 (8H, m), 2.31 (1H, m), 2.54 (2H, d), 2.84 (1H, m), 3.71 (3H, s), 6.27 (2H, m), 6.95 (1H, d).

### Example 31

### trans - [4 - (2, 4 - Dihydroxyphenyl)cyclohexyl]acetic acid

To a round 25ml bottomed flask containing trans - methyl [4 - (2, 4-dihydroxyphenyl)cyclohexyl]acetate (60 mg, 0.23 mmol) and water (4 ml) was added sodium hydroxide (32 mg, 0.78 mmol) and the solution heated to 40°C for 1 hr. The solution was poured into a separating funnel containing ethyl acetate (15 ml) and water (10 ml). To the aqueous layer was then added aqueous hydrochloric acid (10 ml, 1.0M) and ethyl acetate (20 ml). The layers were separated and the aqueous layer extracted with ethyl acetate (3x10 ml). The combined organic extracts were washed with brine (15 ml), dried over magnesium sulfate, filtered and concentrated *in vacuo* affording the title compound (34 mg, 60%) as a solid. m/z (ES⁺) 251 (M+H⁺); δ_{H}(CD₃OD) 1.20 (2H, m), 1.48 (2H, m), 1.89 (4H, m), 2.25 (2H, d), 2.81 (1H, m), 6.27 (2H, m), 6.91 (1H, m).

### Example 32

### cis - [4 - (2, 4 - Dihydroxyphenyl)cyclohexyl]acetic acid

To a round 25ml bottomed flask containing cis - methyl [4 - (2, 4-dihydroxyphenyl)cyclohexyl]acetate (10 mg, 0.038 mmol) and water (4 ml) was added sodium hydroxide (5 mg, 0.13 mmol) and the solution heated to 40°C for 1 hr. The solution was poured into a separating funnel containing ethyl acetate (15 ml) and water (10 ml). To the aqueous layer was then added aqueous hydrochloric acid (10 ml, 1.0M) and ethyl acetate (20 ml). The layers were separated and the aqueous layer extracted with ethyl acetate (3x10 ml). The combined organic extracts were washed with brine (15 ml), dried over magnesium sulfate, filtered and concentrated *in vacuo* affording the title compound (5 mg, 55%) as a solid. m/z (ES⁺) 251 (M+H⁺); δ_{H}(CD₃OD) 1.61 - 1.77 (8H, m), 2.30 (1 H, m), 2.49 (2H, d), 2.84 (1H, m), 6.27 (2H, m), 6.96 (1H, d).

### Example 33

### cis / trans - [4 - (2, 4 - Dihydroxyphenyl)cyclohexyl]acetic acid

To a round bottomed flask equipped with magnetic stirrer was added {4 - [2, 4-dihydroxyphenyl]cyclohexylidene}acetic acid (50 mg, 0.20 mmol) and ethanol (15 ml). To the stirred solution was added palladium (catalytic amount, 10% on activated carbon) in one portion. The reaction vessel was evacuated and then placed under an atmosphere of hydrogen. This was repeated ten times and then stirred for 17 hr under a hydrogen atmosphere at room temperature. The reaction mixture was filtered through a celite plug, washing with ethanol. The solvent was removed in vacuo to give the title compound (50mg, 100%) as a pale yellow oil. m/z (ES⁺) 251 (M+H⁺); δ_{H}(CD₃OD) 1.33 - 1.91 (9H, m), 2.30 (2H, m), 2.81 (1 H, m), 6.28 (2H, m), 6.94 (1 H, m).

### Example 34

### cis / trans - [4-(2, 4-Dihydroxyphenyl)cyclohexyl]acetonitrile

To a round bottomed flask equipped with magnetic stirrer was added {4-[2, 4-*bis*(methoxymethoxy)phenyl]cyclohexylidene}acetonitrile (408 mg, 1.3 mmol) and methanol (20 ml). The resulting solution was heated to reflux temperature and aqueous hydrochloric acid (20 ml, 1.0M) was added. The solution was heated for 1 hr then cooled and saturated aqueous sodium bicarbonate solution (50ml) added. The mixture was partitioned between ethyl acetate (100 ml) and water (20 ml) and the aqueous layer was extracted with ethyl acetate (2x20 ml). The combined organic layers were washed with brine (20 ml), dried over magnesium sulfate, filtered and concentrated *in vacuo* affording an oil. To a 50 ml round bottomed flask equipped with magnetic stirrer was added crude [4-(2, 4-dihydroxyphenyl)cyclohexylidene]acetonitrile (ca. 224 mg, 0.98 mmol) and ethanol (15 ml). To the stirred solution was added palladium (catalytic amount, 10% on activated carbon) in one portion. The reaction vessel was evacuated and then placed under a hydrogen atmosphere. This process was repeated 10 times before leaving the reaction mixture under a hydrogen atmosphere. Vigorous stirring was continued for 17 hr, then the reaction mixture was filtered through celite washing with methanol. The solvent was removed under reduced pressure and the residue was purified *via* flash column chromatography (SiO₂, ethyl acetate /petroleum ether, 1:1, v/v), furnishing the title compound (226 mg, 80% over 2 steps) as a colourless oil. m/z (ES⁺) 232 (M+H⁺); δ_{H}(CD₃OD) 1.31 (1H, m), 1.52 (1H, m), 1.67 (1H, m), 1.77 (0.5H, m), 1.83 (1H, m), 1.92 (2H, m), 1.98 (2H, m), 2.22 (0.5H), 2.44 and 2.67 (2H, d), 2.84 (1H, m), 6.28 (2H, m), 6.96 (1 H, m).

### Example 35

### cis / trans - 4 - [4-(2-Aminoethyl)cyclohexyl]-1,3-benzenediol hydrochloride

*(Cis* / *trans*) - [4-(2, 4-dihydroxyphenyl)cyclohexyl]acetonitrile (214 mg, 0.95 mmol), ethanol (25 ml) and chloroform (1 ml) were placed in a bomb and platinum (IV) oxide (25 mg, 0.11 mmol) was added. The bomb was placed in a high pressure hydrogenation apparatus and shaken for 4 hr at ca. 50 psi under a hydrogen atmosphere. The reaction mixture was filtered through a celite plug, washing with methanol (30 ml). The solvents were removed *in vacuo* and the residue was washed with ethyl acetate (3x10 ml) to yield the title compound (161 mg, 64%) as a yellow oil. m/z (ES⁺) 236 (M+H⁺); δ_{H}(CD₃OD) 1.08 - 1.93 (11 H, m), 2.79 and 2.97 (1H, m), 2.99 (2H, m), 6.22 (2H, m), 6.88 (1H, m).

### Example 36

### (±)-4-(3,3-Difluorocyclohexyl)-1,3-benzenediol

A mixture of (±)-1-(3,3-difluorocyclohexyl)-2,4-*bis*(methoxymethoxy)benzene (30 mg), methanol (2 ml) and acidic ion exchange resin (200 mg) was heated under reflux for 4h. The reaction mixture was filtered and the resin was washed with methanol. The combined filtrate and washings were evaporated *in vacuo* and the crude residue was purified by preparative HPLC to give the title compound as a solid (5 mg, 23%). δ_{H} (CD₃OD) 1.2 - 2.2 (8H, m), 3.08 (1H, m), 6.23 - 6.27 (2H, m), 6.87 (1H, d); m/z (ES⁻) 287 (M-1+AcOH)⁻.

### Example 37

### (±)-3-(2,4-Dihydroxyphenyl)cyclohexanecarboxamide

(±)-3-[2,4-*Bis*(methoxymethoxy)phenyl]cyclohexanecarboxamide (22 mg), methanol (2 ml) and acidic ion exchange resin (300 mg) were heated to reflux for 5 hr. The reaction mixture was filtered and the resin was washed with methanol. The combined filtrate and washings were evaporated *in vacuo* and the crude residue was purified by preparative HPLC to give the title compound as a white solid (5mg, 31%). δ_{H} (CD₃OD) 1.45 - 1.82 (6H, m), 2.07-2.19 (2H, m), 2.62 - 2.69 (1H, m), 3.00 - 3.09 (1H, m), 6.20 - 6.28 (2H, m), 6.89 (1H, d); m/z (ES⁺) 236 (M+H)⁺.

### Example 38

### (±)-3-(2,4-Dihydroxyphenyl)-N-hydroxycyclohexanecarboxamide

(±)-3-[2,4-*Bis*(methoxymethoxy)phenyl]cyclohexyl}methanol (25 mg), methanol (2 ml) and acidic ion exchange resin (300 mg) were heated under reflux for 4 hr. The reaction mixture was filtered and the resin was washed with methanol. The combined filtrate and washings were evaporated in vacuo and the crude residue was purified by preparative HPLC to give the title compound as a solid (5 mg, 27%). δ_{H} (CD₃OD) 1.28 - 2.40 (8H, m), 2.08 - 2.20 (0.5H, m), 2.78 - 2.94 (1 H, m), 3.60 - 3.72 (2H, m), 6.20 - 6.28 (2H, m), 6.80 - 6.90 (1H, m); m/z (ES⁻) 250 (M-H)⁻.

### Example 39

### (±)-3-(2,4-Dihydroxyphenyl)-N-ethylcyclohexanecarboxamide

(±)-3-[2,4-*Bis*(methoxymethoxy)phenyl]-*N*-ethylcyclohexanecarboxamide (25 mg), methanol (2 ml) and acidic ion exchange resin (300 mg) were heated to reflux for 4 hr. The reaction mixture was filtered and the resin was washed with methanol. The combined filtrate and washings were evaporated *in vacuo* and the crude residue was purified by preparative HPLC to furnish the title compound as a solid (2 mg, 12%). δ_{H} (CD₃OD) 1.80 (3H, t), 1.30-1.60 (4H, m), 1.74 - 1.94 (4H, m), 2.26 - 2.36 (1H, m), 2.82 - 2.92 (1 H, m), 3.16 (2H, q), 6.21 - 6.26 (2H, m), 6.88 (1 H, d); m/z (ES⁺) 264 (M+H)⁺.

### Example 40

### (±)-4-[3-Hydroxy-3-(hydroxymethyl)cyclohexyl]-1,3-benzenediol

(±)-3-[2,4-*Bis*(methoxymethoxy)phenyl]-1-(hydroxymethyl)cyclohexanol (29 mg), methanol (2 ml) and acidic ion exchange resin (300 mg) were heated under reflux for 3 hr. The reaction mixture was filtered and the resin was washed with methanol. The combined filtrate and washings were evaporated *in vacuo* and the crude residue was purified by preparative HPLC to furnish the title compound as a cream solid (5 mg. 24%). δ_{H} (CD₃OD) 1.20-2.00 (8H, m), 2.89 (1H, tt), 3.61 (1H, d), 3.69 (1H, d), 6.20 - 6.26 (2H, m), 6.90 (1H, d); m/z (ES⁻) 237 (M-H)⁻.

### Example 41

### (±)-N-[3-(2,4-dihydroxyphenyl)cyclohexyl]acetamide

(±)-*N*-{3-[2,4-*bis*(methoxymethoxy)phenyl]cyclohexyl}acetamide (20 mg), methanol (2 ml) and acidic ion exchange resin (300 mg) were heated under reflux for 3 hr. The reaction mixture was filtered and the resin was washed with methanol. The combined filtrate and washings were evaporated *in vacuo* and the crude residue was purified by preparative HPLC to furnish the title compound as a solid (5 mg, 34%). δ_{H} (CD₃OD) 1.10 - 2.00 (11H, m), 2.90 (0.3H, tt), 3.08 (0.7H, tt), 3.70 - 3.80 (0.3H, m), 4.16 (0.7H, m), 6.20 - 6.26 (2H, m), 6.84-6.90 (1H, m); mlz (ES⁺) 250 (M+H)⁺.

### Example 42

### cis / trans - 4-(2,4-Dihydroxyphenyl)cyclohexanecarboxylic acid

Tetrabutylammonium fluoride (0.12 ml) was added to a stirred solution of 4-(2,4-*bis*{[*tert*-butyl(dimethyl)silyl]oxy}phenyl)cyclohexanecarboxylic acid (22 mg) in tetrahydrofuran (1 ml) at room temperature under argon. After 24 hr, the reaction mixture was partitioned between ethyl acetate (30 ml) and water (30 ml). The layers were separated and the aqueous layer was extracted with ethyl acetate (2 x 30 ml). The combined organic extracts were dried over magnesium sulfate and evaporated *in vacuo*. The residue was purified by flash column chromatography (SiO₂, ethyl acetate/petrol, 7:1 v/v, <1% acetic acid) to give the title compound as an orange solid (10 mg, 89%). δ_{H} (CD₃OD) 1.40 - 1.65 (4H, m), 1.88 - 1.95 (2H, m), 2.06 - 2.14 (2H, m), 2.35 (1H, tt), 2.82 (1H, tt), 6.25 - 6.30 (2H, m), 6.90 (1H, d); *m*/*z* (ES⁺) 235 (M-H)⁻.

### Example 43

### trans-4-(2,4-Dihydroxyphenyl)cyclohexyl ethylcarbamate

A mixture of *trans*-4-(2,4-*bis*{[*tert*-butyl(dimethyl)silyl]oxy}phenyl)cyclohexyl ethylcarbamate (18 mg), methanol (10ml) and Amberlyst fluoride resin (0.3 g) were stirred at room temperature for 24 hr. The reaction mixture was filtered, and the filtrate was evaporated *in vacuo*. The residue was purified by flash column chromatography (SiO₂, ethyl acetate/petrol, 1:3 v/v) to give the title compound as a white solid-(24 mg, 87%). δ_{H} (CD₃OD) 1.10 (3H, t), 1.40 - 1.60 (4H, m), 1.80 - 1.90 (2H, m), 2.00 - 2.10 (2H, m), 2.72 - 2.80 (1H, m), 3.10 (2H, t), 4.50 - 4.60 (1H, m), 6.20 - 6.26 (2H, m), 6.88 (1H, d); m/z (ES⁺) 280 (M+H)⁺.

### Example 44

### trans-4-(2,4-Dihydroxyphenyl)cyclohexyl cyclohexylcarbamate

A mixture of *trans*-4-(2,4-*bis*{[*tert*-butyl(dimethyl)silyl]oxy}phenyl)cyclohexyl cyclohexylcarbamate (28 mg), methanol (10 ml) and Amberlyst fluoride resin (0.3 g) were stirred at room temperature for 72 hr. The reaction mixture was filtered and the filtrate was evaporated *in vacuo*. The residue was purified by flash column chromatography (SiO₂, ethyl acetate/petrol, 1:3 v/v) to give the title compound as a white solid (6 mg, 36%); δ_{H} (CD₃OD) 1.10 - 2.12 (18H, m), 2.70 - 2.82 (1H, m), 4.46 - 4.60 (1H, m), 6.18 - 6.30 (2H, m), 6.80-6.92 (1H, m); m/z (ES⁺) 334 (M+H)⁺.

### Example 45

### trans-4-[4-(Phenylsulfanyl)cyclohexyl]-1,3-benzenediol

A round bottom flask containing thiophenol (30 µl, 0.29 mmol), cesium fluoride (44 mg, 0.29 mmol) and *N,N*-dimethylformamide (3 ml) was warmed at 40 °C for 1 hr. To this was added *cis* - 4-(2,4-*bis*{[*tert*-butyl(dimethyl)silyl]oxy}phenyl)cyclohexyl methanesulfonate (100 mg, 0.19 mmol) in *N*, *N*-dimethylformamide (1 ml) and the reaction mixture stirred at 50 °C for 18 hr. The reaction mixture was poured into saturated aqueous sodium hydrogen carbonate (10 ml) and extracted with ethyl acetate (3x 20 ml). The combined organic extracts were washed with brine (10 ml), dried over magnesium sulfate and concentrated *in vacuo*. Purification *via* flash column chromatography (SiO₂, ethyl acetate/petrol, 1:3) afforded the title compound (23 mg, 40%) as an off-white solid. δ_{H}(d⁴-MeOH) 1.40 - 1.57 (4H, m), 1.82 - 1.90 (2H, m), 2.04 - 2.16 (2H, m), 2.80 (1H, m), 3.13 (1 H, m), 6.19 - 6.28 (2H, m), 6.88 (1 H, d), 7.23 (1H, m), 7.30 (2H, m), 7.40 (2H, m); m/z (ES⁺) 301 (M+H)⁺.

### Example 46

### trans - 4-[4 - (Phenylsulfonyl)cyclohexyl] - 1, 3 - benzenediol

A round bottom flask containing *trans*-4-[4-(phenylsulfanyl)cyclohexyl]-1,3-benzenediol (18 mg, 0.06 mmol) and dichloromethane (2 ml) was cooled to 0 °C and *meta*-chloroperbenzoic acid (50-60%. 41 mg, 0.24 mmol) was added with stirring. After 30 min at this temperature the reaction mixture was poured into saturated sodium thiosulfate solution (5 ml) and partitioned between saturated aqueous sodium hydrogen carbonate (10 ml) and dichloromethane (10 ml). The aqueous phase was further extracted with dichloromethane (2 x 10 ml) and the combined organic extracts were washed with brine, dried over magnesium sulfate and concentrated *in vacuo*. Purification via flash column chromatography (SiO₂, ethyl acetate/petrol, 30:70) afforded the title compound (6 mg, 30%) as an off-white solid. δ_{H} (d⁴-MeOH) 1.40 - 1.62 (4H, m), 1.88 - 1.95 (2H, m), 2.05 - 2.13 (2H, m), 2.72 (1H, m), 3.18 (1H, m), 6.08 - 6.13 (2H, m), 6.82 (1H, d), 7.64 (2H, m), 7.75 (1H, m), 7.90 (2H, m); m/z (ES⁺) 333 (M+H)⁺.

### Examples 47 and 48

### Diastereolsomers of ethyl 4 - (2, 4 - dihydroxyphenyl) - 1 - hydroxycyclohexane carboxylate

A solution of one isomer of ethyl 4 - (2, 4 - bis{[*tert* - butyl(dimethyl)silyl]oxy}phenyl) - 1 - hydroxycyclohexanecarboxylate (25 mg, 0.05 mmol) in methanol (2 ml) was stirred rapidly with Amberlyst A-26 (100 mg) for 18 hr. After this time the reaction mixture was filtered. The resin was then stirred rapidly for 1 hr in a solution of methanol (2 ml) and glacial acetic acid (5 drops). The reaction mixture was filtered and the combined filtrates concentrated *in vacuo*. Purification via flash column chromatography (SiO₂, ethyl acetate/petrol, 2:1) afforded the title compound (3 mg, 21%) as a white solid. δ_{H} (d⁴-MeOH) 1.29 (3H, t), 1.61 - 1.69 (2H, m), 1.73 - 1.98 (6H, m), 2.81 -2.90 (1H, m), 4.18 (2H, q), 6.21-6.27 (2H, m), 6.91 (1H, d); m/z (ES⁻) 279 (M-H)⁻.

A solution of the other isomer of ethyl 4 - (2, 4 - bis{[*tert* - butyl(dimethyl)silyl] oxy}phenyl) - 1 - hydroxycyclohexanecarboxylate (25 mg, 0.05 mmol) in methanol (2 ml) was stirred rapidly with Amberlyst A-26 (100 mg) for 18 hr. After this time the reaction mixture was filtered. The resin was then stirred rapidly for 1 hr in a solution of methanol (2 ml) and glacial acetic acid (5 drops). The reaction mixture was filtered and the combined filtrates concentrated *in vacuo.* Purification via flash column chromatography (SiO₂, ethyl acetate/petrol, 2:1) afforded the title compound (5 mg, 34%) as a white solid. δ_{H} (d⁴-MeOH) 1.22 (3H, t). 1.40 - 1.54 (4H, m), 1.63 - 1.74 (2H, m), 2.18 -2.25 (2H, m), 2.68 - 2.78 (1H, m), 4.14 (2H, q), 6.11 (1H, dd), 6.15 (1H, d), 6.71 (1H, d); m/z (ES⁻) 279 (M-H)⁻.

### Example 49

### Cis / trans - 4 - [4 - (hydroxyamino)cyclohexyl]-1, 3, benzenediol

To a stirred solution of 4 - (2, 4 - dihydroxyphenyl)cyclohexanone oxime (90 mg, 0.41 mmol) in acetic acid (3 ml) was added sodium cyanoborohydride (28 mg, 0.45 mmol) in one portion. After stirring for 16 hr, a further portion of sodium cyanoborohydride (28 mg) was added and stirring continued for a further 48 hr. The reaction mixture was poured into a mixture of water (3 ml) and ethyl acetate (25 ml) and stirred for 30 min. The solution was partitioned, and the aqueous layer further extracted with ethyl acetate (5 x 10 ml). The combined organics were washed with brine (15 ml), dried over magnesium sulfate and concentrated *in vacuo*. Purification by HPLC afforded the title compound as a pale pink solid (62 mg, 68%). δ_{H} (CD₃OD) 1.57 -1.77 (3H, m), 1.78-1.84 (1 H, m), 1.90 - 2.09 (2H, m), 2.27 - 2.12 (2H, m), 2.87 (0.6 H, m), 3.03 (0.4 H, m), 3.58 (0.4 H, m), 3.68 (0.6 H, m), 6.25 - 6.32 (2H, m), 6.92 (0.6H, d), 6.99 (0.4H, d), m/z (ES⁻) 222 (M-H)⁻.

### Example 50

### Trans - 4 - [4 - (methoxyamino)cyclohexyl]-1, 3, benzenediol

To a stirred solution of O-methyl-4 -(2, 4 - dihydroxyphenyl)cyclohexanone oxime (18 mg, 0.076 mmol) in acetic acid (1 ml) was added sodium cyanoborohydride (25 mg, 0.4 mmol) in one portion. After stirring overnight the reaction mixture was partitioned between water (10 ml) and ethyl acetate (10 ml). The aqueous layer was further extracted with ethyl acetate (10 ml) and the combined organic phases were washed with saturated sodium hydrogen carbonate solution (10 ml), dried over magnesium sulfate and concentrated *in vacuo.* Purification by flash column chromatography (SiO₂, ethyl acetate/petrol 2:3) afforded the title compound as a solid (12 mg, 66%). δ_{H} (CDCl₃) 1.13 - 1.26 (2H, m), 1.30 - 1.45 (2H, m), 1.80 - 1.89 (2H, m), 1.90 - 2.00 (2H, m), 2.68 - 2.78 (1H, m), 2.80 - 2.90 (1H, m), 3.49 (3H, s), 6.19 (1H, d), 6.24 (1H, dd), 6.86 (1H, d); m/z (ES⁺) 279 (MH+CH₃CN)⁺.

## Claims

1. A compound of formula I: 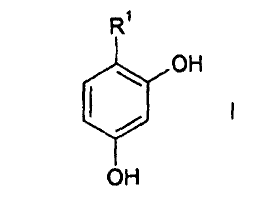 or a pharmaceutically acceptable salt thereof, wherein:
R¹ is a (C₃-C₈)cycloalkyl ring or (C₅-C₈)cycloalkenyl ring, wherein either the cycloalkyl ring or cycloalkenyl ring is substituted by one to three substituents independently selected from the group consisting of cyano; halo; (C₁-C₆)alkyl; aryl; (C₂-C₉)heterocycloalkyl; (C₂-C₉)heteroaryl; aryl(C₁-C₆)alkyl-; =O; =CHO(C₁-C₆)alkyl; amino; hydroxy; (C₁-C₆)alkoxy; aryl(C₁-C₆)alkoxy-; (C₁-C₆)acyl; (C₁-C₆)alkylamino-; aryl(C₁-C₆)alkylamino-; amino(C₁-C₆)alkyl-; (C₁-C₆)alkoxy-CO-NH-; (C₁-C₆)alkylamino-CO-; (C₂-C₆)alkenyl; (C₂-C₆)alkynyl; hydroxy(C₁-C₆)alkyl-; (C₁-C₆)alkoxy(C₁-C₆)alkyl-; (C₁-C₆)acyloxy(C₁-C₆)alkyl-; nitro; cyano(C₁-C₆)alkyl-; halo(C₁-C₆)alkyl-; nitro(C₁-C₆)alkyl-; trifluoromethyl; trifluoromethyl(C₁-C₆)alkyl-; (C₁-C₆)acylamino-; (C₁-C₆)acylamino(C₁-C₆)alkyl-; (C₁-C₆)alkoxy(C₁-C₆)acylamino-; amino(C₁-C₆)acyl-; amino(C₁-C₆)acyl(C₁-C₆)alkyl-; (C₁-C₆)alkylamino(C₁-C₆)acyl-; ((C₁-C₆) alkyl)₂amino(C₁-C₆)acyl-; -CO₂R²; -(C₁-C₆)alkyl-CO₂R²; -C(O)N(R²)₂; -(C₁-C₆)alkyl-C(O)N(R²)₂; R²ON=; R²ON=(C₁-C₆)alkyl-; R²ON=CR²(C₁-C₆)alkyl-; -NR²(OR²); -(C₁-C₆)alkyl-NR²(OR²);-C(O)(NR²OR²); -(C₁-C₆)alkyl-C(O)(NR²OR²); -S(O)ₘR²; wherein each R² is independently selected from hydrogen, (C₁-C₆)alkyl, aryl, or aryl(C₁-C₆)alkyl-; R³C(O)O-, wherein R³ is (C₁-C₆)alkyl, aryl, or aryl(C₁-C₆)alkyl-; R³C(O)O-(C₁-C₆)alkyl-; R⁴R⁵N-C(O)-O-; R⁴R⁵NS(O)₂-; R⁴R⁵NS(O)₂(C₁-C₆)alkyl-; R⁴S(O)₂R⁵N-; R⁴S(O)₂R⁵N(C₁-C₆)alkyl-; wherein m is 0, 1 or 2, and R⁴ and R⁵ are each independently selected from hydrogen or (C₁-C₆)alkyl; -C(=NR⁶)(N(R⁴)₂); or -(C₁-C₆)alkyl-C(=NR⁶)(N(R⁴)₂) wherein R⁶ represents OR² or R² wherein R² is defined as above;
with the proviso that the cycloalkenyl ring is not aromatic;
with the proviso that R¹ must be substituted by at least one of R³C(O)O- (C₁-C₆)alkyl-, R²ON=, R²ON=(C₁-C₆)alkyl-, R²ON=CR²(C₁-C₆)alkyl-, -NR²(OR²), R⁴R⁵NS(O)₂-, R⁴R⁵NS(O)₂(C₁-C₆)alkyl-, R⁴S(O)₂R⁵N-, or R⁴S(O)₂R⁵N(C₁-C₆)alkyl-;
with the proviso that when R¹ is only substituted by one of R²ON=, then R² cannot be hydrogen.

2. The compound of claim 1, wherein R¹ is a cyclohexyl or cyclohexenyl ring substituted at the 3- or 4-position, or a cyclopentyl or cyclopentenyl ring substituted at the 3-position.

3. The compound of claim 1, wherein R¹ is monosubstituted.

4. The compound of claim 1, wherein R¹ is disubstituted.

5. The compound of claim 1, wherein R¹ is substituted by at least one of R³C(O)O- or R³C(O)O-(C₁-C₆)alkyl-.

6. The compound of claim 1, wherein R¹ is substituted by at least one of R²ON=, R²ON=(C₁-C₆)alkyl-, or R²ON=CR²(C₁-C₆)alkyl-.

7. The compound of claim 1, wherein R¹ is substituted by at least one of-NR²(OR²).

8. The compound of claim 1, wherein R¹ is substituted by at least one of R⁴R⁵NS(O)₂-, R⁴R⁵NS(O)₂(C₁-C₆)alkyl-, R⁴S(O)₂R⁵N-, or R⁴S(O)₂R⁵N(C₁-C₆)alkyl-.

9. The compound of claim 1, wherein R¹ is substituted by at least one of R⁴S(O)₂R⁵N- or R⁴S(O)₂R⁵N(C₁-C₆)alkyl-.

10. The compound of claim 1, wherein R¹ is a (C₃-C₈)cycloalkyl ring or (C₅-C₈)cycloalkenyl ring, wherein either the cycloalkyl ring or cycloalkenyl ring is substituted by one of R³C(O)O-(C₁-C₆)alkyl-, R²ON=, R²ON=(C₁-C₆)alkyl-, R²ON=CR²(C₁-C₆)alkyl-, -NR²(OR²), R⁴R⁵NS(O)₂-, R⁴R⁵NS(O)₂(C₁-C₆)alkyl-, R⁴S(O)₂R⁵N-, or R⁴S(O)₂R⁵N(C₁-C₆)alkyl-.

11. The compound of claim 10, wherein R¹ is a (C₃-C₈)cycloalkyl ring or (C₅-C₈)cycloalkenyl ring, wherein either the cycloalkyl ring or cycloalkenyl ring is substituted by one of R³C(O)O-(C₁-C₆)alkyl-, R²ON=, or R⁴S(O)₂R⁵N-.

12. The compound of claim 10, wherein R¹ is substituted by R³C(O)O-(C₁-C₆)alkyl-.

13. The compound of claim 10, wherein R¹ is substituted by R²ON=, R²ON=(C₁-C₆)alkyl-, or R²ON=CR²(C₁-C₆)alkyl-.

14. The compound of claim 10, wherein R¹ is substituted by R²ON=.

15. The compound of claim 10, wherein R¹ is substituted by -NR²(OR²).

16. The compound of claim 10, wherein R¹ is substituted by R⁴R⁵NS(O)₂-, R⁴R⁵NS(O)₂(C₁-C₆)alkyl-, R⁴S(O)₂R⁵N- or R⁴S(O)₂R⁵N(C₁-C₆)alkyl-.

17. The compound of claim 1, wherein R¹ is substituted by R⁴S(O)₂R⁵N- or R⁴S(O)₂R⁵N(C₁-C₆)alkyl-.

18. The compound of claim 1, wherein the (C₂-C₉)heterocycloalkyl substituent is a group of the formula: 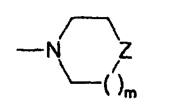 wherein m is 0, 1 or 2, and
Z is CH₂, NR², O, S, SO, or SO₂.

19. The compound of claim 1 selected from the group consisting of:
*O*-Benzyl-4-(2, 4-dihydroxyphenyl)cyclohexanone oxime;
(±)-*N*-[3-(2,4-Dihydroxyphenyl)cyclohexyl]methanesulfonamide;
(±)-*O*-Methyl-3-(2,4-dihydroxyphenyl)cyclohexanone oxime;
(±)-*O*-Benzyl-3-(2,4-dihydroxyphenyl)cyclohexanone oxime;
and a pharmaceutically acceptable salt thereof.

20. The compound of claim 1 selected from the group consisting of:
*O*-Methyl-4-(2, 4-dihydroxyphenyl)cyclohexanone oxime;
(±)-4-[3-(Hydroxyamino)cyclohexyl]-1,3-benzenediol;
cis - *N* - [4 - (2, 4 - Dihydroxyphenyl)cyclohexyl] - 1 - butanesulfonamide;
trans - *N* - [4 - (2, 4 - Dihydroxyphenyl)cyclohexyl]methanesulfonamide;
*cis - N -* [4 - (2, 4 - Dihydroxyphenyl)cyclohexyl]methanesulfonamide;
*cis* / *trans -* 4- [4-(hydroxyamino)cyclohexyl]-1,3 - benzenediol;
*trans* - 4-[4-(methoxyamino)cyclohexyl]-1,3 - benzenediol;
and a pharmaceutically acceptable salt thereof.

21. A topical pharmaceutical composition for lightening skin or reducing the pigmentation of skin in a human, comprising a pharmaceutically acceptable carrier, and a skin-lightening or pigmentation-reducing amount of a compound of formula I, as defined in claim 1, or a pharmaceutically acceptable salt thereof.

22. Use of a compound of formula I, as defined in claim 1, or a pharmaceutically acceptable salt thereof, in the manufacture of a skin-lightening or pigmentation-reducing medicament.

23. A cosmetic method of lightening skin or reducing the pigmentation of skin in a human patient, comprising administering to the patient a skin-lightening or skin pigmentation-reducing effective amount of a compound of formula I, as defined in claim 1, or a pharmaceutically acceptable salt thereof, until a cosmetically beneficial amount of skin-lightening or skin pigmentation-reduction has been achieved.

## Patentansprüche

1. Verbindung der Formel I 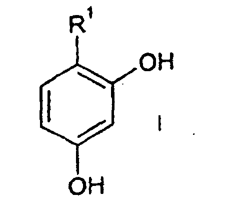 oder ein pharmazeutisch akzeptables Salz davon, wobei
R¹ folgende Bedeutungen hat: ein (C₃-C₈)-Cycloalkylring oder(C₅-C₈)-Cycloalkenylring, wobei der Cycloalkylring oder Cycloalkenylring mit 1 bis 3 Substituenten substituiert ist, die unabhängig voneinander aus folgender Gruppe ausgewählt sind: Cyano; Halogen; (C₁-C₆)-Alkyl; Aryl; (C₂-C₉)-Heterocycloalkyl; (C₂-C₉)-Heteroaryl; Aryl-(C₁-C₆)-alkyl-; =O; =CHO(C₁-C₆)-Alkyl; Amino; Hydroxy; (C₁-C₆)-Alkoxy; Aryl-(C₁-C₆)-alkoxy-; (C₁-C₆)-Acyl; (C₁-C₆)-Alkylamino-; Aryl-(C₁-C₆)-alkylamino-; Amino-(C₁-C₆)-alkyl-; (C₁-C₆)-Alkoxy-CO-NH-; (C₁-C₆)-Alkylamino-CO-; (C₂-C₆)-Alkenyl; (C₂-C₆)-Alkinyl; Hydroxy-(C₁-C₆)-alkyl-; (C₁-C₆)-Alkoxy-(C₁-C₆)-alkyl-; (C₁-C₆)-Acyloxy-(C₁-C₆)-alkyl-; Nitro; Cyano-(C₁-C₆)-alkyl-; Halogen-(C₁-C₆)-alkyl-; Nitro-(C₁-C₆)-alkyl-; Trifluormethyl; Trifluormethyl-(C₁-C₆)-alkyl-; (C₁-C₆)-Acylamino; (C₁-C₆)-Acylamino-(C₁-C₆)-alkyl-; (C₁-C₆)-Alkoxy-(C₁-C₆)-acylamino-; Amino-(C₁-C₆)-acyl; Amino-(C₁-C₆)-acyl-(C₁-C₆)-alkyl-; (C₁-C₆)-Alkylamino-(C₁-C₆)-acyl-; ((C₁-C₆)-Alkyl)₂-amino-(C₁-C₆)-acyl-; -CO₂R²; -(C₁-C₆)-Alkyl-CO₂R²; -C(O)N(R²)₂; -(C₁-C₆)-Alkyl-C(O)N(R²)₂; R²ON=; R²ON=(C₁-C₆)-Alkyl; R²ON=CR²-(C₁-C₆)-Alkyl-; NR²(OR²); -(C₁-C₆)-Alkyl-NR²(OR²); -C(O)(NR²OR²); -(C₁-C₆)-Alkyl-C(O)(NR²OR²); -S(O)ₘR²; wobei jedes R² unabhängig voneinander ausgewählt sind aus Wasserstoff, (C₁-C₆)-Alkyl, Aryl oder Aryl-(C₁-C₆)-alkyl; R³C(O)O-, wobei R³ (C₁-C₆)-Alkyl, Aryl oder Aryl-(C₁-C₆)-alkyl bedeutet; R³C(O)O-(C₁-C₆)-Alkyl-; R⁴R⁵N-C(O)-O-; R⁴R⁵NS(O)₂-; R⁴R⁵NS(O)₂(C₁-C₆)-alkyl-; R⁴S(O)₂R⁵N-; R⁴S(O)₂R⁵N(C₁-C₆)-Alkyl-; wobei m den Wert 0, 1 oder 2 hat und R⁴ und R⁵ jeweils unabhängig voneinander aus Wasserstoff oder (C₁-C₆)-Alkyl ausgewählt sind; -C(=NR⁶)(N(R⁴)₂); oder -(C₁-C₆)-Alkyl-C(=NR⁶)(N(R⁴)₂), wobei R⁶ OR² oder R² bedeutet, wobei R² die vorstehend definierte Bedeutung hat;
mit der Maßgabe, dass der Cycloalkenylring nicht aromatisch ist;
mit der Maßgabe, dass R¹ mit mindestens einem der folgenden Reste substituiert sein muss: R³C(O)O-(C₁-C₆)-Alkyl, R²ON=, R²ON=(C₁-C₆)-Alkyl, R²ON=CR²-(C₁-C₆)-Alkyl, -NR²(OR²), R⁴R⁵NS(O)₂-, R⁴R⁵NS(O)₂(C₁-C₆)-Alkyl-, R⁴S(O)₂R⁵N- oder R⁴S(O)₂R⁵N-(C₁-C₆)-Alkyl-;
mit der Maßgabe, dass wenn R¹ nur mit einem Rest der Formel R²ON= substituiert ist, R² nicht Wasserstoff bedeuten kann.

2. Verbindung nach Anspruch 1, wobei R¹ einen Cyclohexyl- oder Cyclohexenylring, der in der 3- oder 4-Position substituiert ist, oder einen Cyclopentyl- oder Cyclopentenylring, der in der 3-Position substituiert ist, bedeutet.

3. Verbindung nach Anspruch 1, wobei R¹ monosubstituiert ist.

4. Verbindung nach Anspruch 1, wobei R¹ disubstituiert ist.

5. Verbindung nach Anspruch 1, wobei R¹ mit mindestens einem der Reste R³C(O)O- oder R³C(O)O-(C₁-C₆)-Alkyl- substituiert ist.

6. Verbindung nach Anspruch 1, wobei R¹ durch mindestens einen der Reste R²ON=, R²ON=(C₁-C₆)-Alkyl- oder R²ON=CR²-(C₁-C₆)-Alkylsubstituiert ist.

7. Verbindung nach Anspruch 1, wobei R¹ mit mindestens einem Rest der Formel -NR²(OR²) substituiert ist.

8. Verbindung nach Anspruch 1, wobei R¹ mit mindestens einem der Reste R⁴R⁵NS(O)₂-, R⁴R⁵NS(O)₂-(C₁-C₆)-Alkyl-, R⁴S(O)₂R⁵N- oder R⁴S(O)₂R⁵N-(C₁-C₆)-Alkyl- substituiert ist.

9. Verbindung nach Anspruch 1, wobei R¹ mit mindestens einem der Reste R⁴S(O)₂R⁵N- oder R⁴S(O)₂R⁵N-(C₁-C₆)-Alkyl substituiert ist.

10. Verbindung nach Anspruch 1, wobei R¹ einen (C₃-C₈)-Cycloalkylring oder (C₅-C₈)-Cycloalkenylring bedeutet, wobei der Cycloalkylring oder Cycloalkenylring mit mindestens einem der folgenden Reste substituiert ist: R³C(O)O-(C₁-C₆)-Alkyl-, R²ON=, R²ON=(C₁-C₆)-Alkyl-, R²ON=CR²(C₁-C₆)-Alkyl-, -NR²(OR²), R⁴R⁵NS(O)₂-, R⁴R⁵NS(O)₂(C₁-C₆)-Alkyl-, R⁴S(O)₂R⁵N- oder R⁴S(O)₂R⁵N(C₁-C₆)-Alkyl-.

11. Verbindung nach Anspruch 10, wobei R¹ einen (C₃-C₈)-Cycloalkylring oder (C₅-C₈)-Cycloalkenylring bedeutet, wobei der Cycloalkylring oder Cycloalkenylring mit einem der Reste R³C(O)O-(C₁-C₆)-Alkyl-, R²ON= oder R⁴S(O)₂R⁵N- substituiert ist.

12. Verbindung nach Anspruch 10, wobei R¹ mit R³C(O)O-(C₁-C₆)-Alkyl substituiert ist.

13. Verbindung nach Anspruch 10, wobei R¹ mit R²ON=, R²ON=(C₁-C₆)-Alkyl- oder R²ON=CR²-(C₁-C₆)-Alkyl substituiert ist.

14. Verbindung nach Anspruch 10, wobei R¹ mit R²ON= substituiert ist.

15. Verbindung nach Anspruch 10, wobei R¹ mit -NR²(OR²) substituiert ist.

16. Verbindung nach Anspruch 10, wobei R¹ mit R⁴R⁵NS(O)₂-, R⁴R⁵NS(O)₂(C₁-C₆)-Alkyl-, R⁴S(O)₂R⁵N- oder R⁴S(O)₂R⁵N(C₁-C₆)-Alkylsubstituiert ist.

17. Verbindung nach Anspruch 1, wobei R¹ mit R⁴S(O)₂R⁵N- oder R⁴S(O)₂R⁵N-(C₁-C₆)-Alkyl substituiert ist.

18. Verbindung nach Anspruch 1, wobei es sich beim (C₂-C₉)-Heterocycloalkyl-Substituenten um eine Gruppe der folgenden Formel handelt 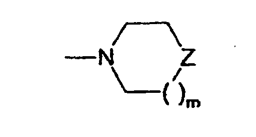 wobei m den Wert 0, 1 oder 2 hat und
Z CH₂, NR², O, S, SO oder SO₂ ist.

19. Verbindung nach Anspruch 1, ausgewählt aus der folgenden Gruppe:
O-Benzyl-4-(2,4-dihydroxyphenyl)cyclohexanonoxim;
(±)-N-[3-(2,4-Dihydroxyphenyl)cyclohexyl]methansulfonamid;
(±)-O-Methyl-3-(2,4-dihydroxyphenyl)cyclohexanonoxim;
(±)-O-Benzyl-3-(2,4-dihydroxyphenyl)cyclohexanonoxim;
und ein pharmazeutisch akzeptables Salz davon.

20. Verbindung nach Anspruch 1, ausgewählt aus der folgenden Gruppe:
O-Methyl-4-(2,4-dihydroxyphenyl)cyclohexanonoxim;
(±)-4-[3-(Hydroxyamino)cyclohexyl]-1,3-benzoldiol;
cis-N-[4-(2,4-Dihydroxyphenyl)cyclohexyl]-1-butansulfonamid;
trans-N-[4-(2,4-Dihydroxyphenyl)cyclohexyl]-methansulfonamid;
cis-N-[4-(2,4-Dihydroxyphenyl)cyclohexyl]-methansulfonamid;
cis/trans-4-[4-(Hydroxyamino)cyclohexyl]-1,3-benzoldiol;
trans-4-[4-(Methoxyamino)cyclohexyl]-1,3-benzoldiol;
und ein pharmazeutisch akzeptables Salz davon.

21. Topische pharmazeutische Zusammensetzung zur Aufhellung der Haut oder Verringerung der Pigmentierung der Haut beim Menschen, umfassend einen pharmazeutisch akzeptablenTräger und eine die Haut aufhellende oder die Pigmentierung verringernde Menge einer Verbindung der Formel I, wie in Anspruch 1 definiert, oder eines pharmazeutisch akzeptablenSalzes davon.

22. Verwendung einer Verbindung der Formel I, wie in Anspruch 1 definiert, oder eines pharmazeutisch akzeptablenSalzes davon bei der Herstellung eines die Haut aufhellenden oder die Pigmentierung verringernden Arzneimittels.

23. Kosmetisches Verfahren zur Aufhellung der Haut oder zur Verringerung der Pigmentierung der Haut bei einem humanen Patienten, umfassend die Verabreichung einer zur Hautaufhellung oder zur Verringerung der Hautpigmentierung wirksamen Menge einer Verbindung der Formel I, wie in Anspruch 1 definiert, oder eines pharmazeutisch akzeptablenSalzes davon an einen Patienten, bis ein kosmetisch günstiges Ausmaß einer Hautaufhellung oder Verringerung der Hautpigmentierung erreicht worden ist.

## Revendications

1. Composé de formule I : 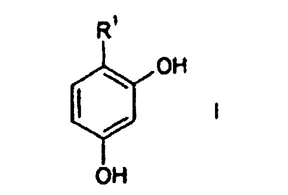 ou un de ses sels pharmaceutiquement acceptables, formule dans laquelle :
R¹ représente un noyau cycloalkyle en C₃ à C₈ ou un noyau cycloalcényle en C₅ à C₈, le noyau cycloalkyle ou noyau cycloalcényle étant substitué avec 1 à 3 substituants choisis indépendamment dans le groupe consistant en des substituants cyano ; halogéno ; alkyle en C₁ à C₆ ; aryle ; hétérocycloalkyle en C₂ à C₉ ; hétéroaryle en C₂ à C₉ ; aryl-(alkyle en C₁ à C₆)- ; =O ; =CHO(alkyle en C₁ à C₆) ; amino ; hydroxy ; alkoxy en C₁ à C₆ ; aryl (alkoxy en C₁ à C₆)- ; acyle en C₁ à C₆ ; (alkyle en C₁ à C₆)amino- ; aryl(alkyle en C₁ à C₆)amino- ; amino (alkyle en C₁ à C₆)- ; (alkoxy en C₁ à C₆)-CO-NH- ; (alkyle en C₁ à C₆)amino-CO- ; alcényle en C₂ à C₆ ; alcynyle en C₂ à C₆ ; hydroxy(alkyle en C₁ à C₆)- ; (alkoxy en C₁ à C₆) - (alkyle en C₁ à C₆)- ; (acyle en C₁ à C₆) oxy (alkyle en C₁ à C₆)- ; nitro ; cyano(alkyle en C₁ à C₆)- ; halogéno(alkyle en C₁ à C₆)- ; nitro(alkyle en C₁ à C₆)- ; trifluorométhyle ; trifluorométhyl(alkyle en C₁ à C₆)- ; (acyle en C₁ à C₆)amino- ; (acyle en C₁ à C₆) amino (alkyle en C₁ à C₆)- ; (alkoxy en C₁ à C₆)-(acyle en C₁ à C₆)amino- ; amino(acyle en C₁ à C₆)- ; amino(acyle en C₁ à C₆)-(alkyle en C₁ à C₆)- ; (alkyle en C₁ à C₆)amino(acyle en C₁ à C₆)- ; (alkyle en C₁ à C₆)₂amino(acyle en C₁ à C₆) - ; -CO₂R² ; (alkyle en C₁ à C₆)-CO₂R² ; -C(O)N(R²)₂ ; (alkyle en C₁ à C₆)-C(O)N(R²)₂ ; R²ON= ; R²ON= (alkyle en C₁ à C₆)- ; R²ON=CR²(alkyle en C₁ à C₆)- ; -NR²(OR²) ; (alkyle en C₁ à C₆)-NR²(OR²) ; -C(O)(NR²OR²) ; -(alkyle en C₁ à C₆)-C(O)(NR²OR²) ; -S(O)ₘR² ; dans lesquels chaque groupe R² est choisi indépendamment entre l'hydrogène, des groupes alkyle en C₁ à C₆, aryle et aryl(alkyle en C₁ à C₆)- ; R³C(O)O-, dans lequel R³ représente un groupe alkyle en C₁ à C₆, aryle ou aryl (alkyle en C₁ à C₆)- ; R³C(O)O-(alkyle en C₁ à C₆)- ; R⁴R⁵N-C(O)O- ; R⁴R⁵NS(O)₂- ; R⁴R⁵NS(O)₂(alkyle en C₁ à C₆)- ; R⁴S(O)₂R⁵N- ; R⁴S(O)₂R⁵N-(alkyle en C₁ à C₆)- ; dans lesquels m est égal à 0, 1 ou 2 et R⁴ et R⁵ sont choisis chacun indépendamment entre l'hydrogène et un groupe alkyle en C₁ à C₆ ; -C(=NR⁶)(N(R⁴)₂) ; et - (alkyle en C₁ à C₆) -C (=NR⁶)(N (R⁴)₂) dans lesquels R⁶ représente un groupe OR² ou R², le groupe R² répondant à la définition précitée ;
sous réserve que le noyau cycloalcényle ne soit pas aromatique ;
sous réserve que R¹ soit substitué avec au moins un substituant R³C(O)O-(alkyle en C₁ à C₆)-, R²ON=, R²ON=(alkyle en C₁ à C₆)-, R²ON=CR²(alkyle en C₁ à C₆)-, -NR²(OR²), R⁴R⁵NS(O)₂-, R⁴R⁵NS(O)₂(alkyle en C₁ à C₆)-, R⁴S(O)₂R⁵N- ou R⁴S(O)₂R⁵N-(alkyle en C₁ à C₆)- ;
sous réserve que, lorsque R¹ est seulement substitué avec un substituant R²ON=, alors R² ne puisse représenter l'hydrogène.

2. Composé suivant la revendication 1, dans lequel R¹ représente un noyau cyclohexyle ou cyclohexényle substitué en position 3 ou 4, ou un noyau cyclopentyle ou cyclopentényle substitué en position 3.

3. Composé suivant la revendication 1, dans lequel R¹ est monosubstitué.

4. Composé suivant la revendication 1, dans lequel R¹ est disubstitué.

5. Composé suivant la revendication 1, dans lequel R¹ est substitué avec au moins un substituant R³C(O)O- ou R³C(O)O-(alkyle en C₁ à C₆)-.

6. Composé suivant la revendication 1, dans lequel R¹ est substitué avec au moins un substituant R²ON=, R²ON= (alkyle en C₁ à C₆)- ou R²ON=CR²(alkyle en C₁ à C₆)-.

7. Composé suivant la revendication 1, dans lequel R¹ est substitué avec au moins un substituant -NR²(OR²).

8. Composé suivant la revendication 1, dans lequel R¹ est substitué avec au moins un substituant R⁴R⁵NS(O)₂-, R⁴R⁵NS(O)₂(alkyle en C₁ à C₆)-, R⁴S(O)₂R⁵N- ou R⁴S(O)₂R⁵N-(alkyle en C₁ à C₆)-.

9. Composé suivant la revendication 1, dans lequel R¹ est substitué avec au moins un substituant R⁴S(O)₂R⁵N- ou R⁴S(O)₂R⁵N-(alkyle en C₁ à C₆)-.

10. Composé suivant la revendication 1, dans lequel R¹ représente un noyau cycloalkyle en C₃ à C₈ ou un noyau cycloalcényle en C₅ à C₈, le noyau cycloalkyle ou le noyau cycloalcényle étant substitué avec un substituant R³C(O)O-(alkyle en C₁ à C₆)-, R²ON=, R²ON=(alkyle en C₁ à C₆)-, R²ON=CR²(alkyle en C₁ à C₆)-, -NR²(OR²), R⁴R⁵NS(O)₂-, R⁴R⁵NS(O)₂(alkyle en C₁ à C₆)-, R⁴S(O)₂R⁵N- ou R⁴S(O)₂R⁵N(alkyle en C₁ à C₆)-.

11. Composé suivant la revendication 10, dans lequel R¹ représente un noyau cycloalkyle en C₃ à C₈ ou un noyau cycloalcényle en C₅ à C₈, le noyau cycloalkyle ou le noyau cycloalcényle étant substitué avec un substituant R³C(O)O-(alkyle en C₁ à C₆)-, R²ON= ou R⁴S(O)₂R⁵N-.

12. Composé suivant la revendication 10, dans lequel R¹ est substitué avec un substituant R³C(O)O-(alkyle en C₁ à C₆)-.

13. Composé suivant la revendication 10, dans lequel R¹ est substitué avec un substituant R²ON=, R²ON=(alkyle en C₁ à C₆)- ou R²ON=CR²(alkyle en C₁ à C₆)-.

14. Composé suivant la revendication 10, dans lequel R¹ est substitué avec un substituant R²ON=.

15. Composé suivant la revendication 10, dans lequel R¹ est substitué avec un substituant -NR²(OR²).

16. Composé suivant la revendication 10, dans lequel R¹ est substitué avec un substituant R⁴R⁵NS(O)₂-, R⁴R⁵NS(O)₂(alkyle en C₁ à C₆) -, R⁴S(O)₂R⁵N- ou R⁴S(O)₂R⁵N(alkyle en C₁ à C₆)-.

17. Composé suivant la revendication 1, dans lequel R¹ est substitué avec un substituant R⁴S(O)₂R⁵N- ou R⁴S(O)₂R⁵N(alkyle en C₁ à C₆)-.

18. Composé suivant la revendication 1, dans lequel le substituant hétérocycloalkyle en C₂ à C₉ est un groupe de formule : 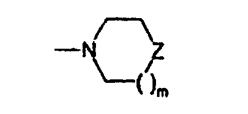 dans laquelle m est égal à 0, 1 ou 2, et
Z représente un groupe CH₂, NR², O, S, SO ou SO₂.

19. Composé suivant la revendication 1, choisi dans le groupe consistant en :
oxime de O-benzyl-4-(2,4-dihydrophényl)-cyclohexanone ;
(±)-N-[3-(2,4-dihydroxyphényl)-cyclohexyl]-méthanesulfonamide ;
oxime de (±)-O-méthyl-3-(2,4-dihydroxyphényl)-cyclohexanone ;
oxime de (±)-O-benzyl-3-(2,4-dihydroxyphényl)-cyclohexanone ;
et leurs sels pharmaceutiquement acceptables.

20. Composé suivant la revendication 1, choisi dans le groupe consistant en :
oxime de O-méthyl-4-(2,4-dihydroxyphényl)-cyclohexanone ;
(±)-4-[3-(hydroxyamino)cyclohexyl]-1,3-benzènediol ;
cis-N-[4-(2,4-dihydroxyphényl)cyclohexyl]-1-butanesulfonamide ;
trans-N-[4-(2,4-dihydroxyphényl)cyclohexyl]-méthanesulfonamide ;
cis-N-[4-(2,4-dihydroxyphényl)cyclohexyl]méthanesulfonamide ;
cis/trans-4-[4-(hydroxyamino)cyclohexyl]-1,3-benzènediol ;
trans-4-[4-(méthoxyamino)cyclohexyl]-1,3-benzènediol ; et leurs sels pharmaceutiquement acceptables.

21. Composition pharmaceutique topique pour l'éclaircissement de la peau ou la réduction de la pigmentation de la peau chez un patient humain, comprenant un support pharmaceutiquement acceptable et une quantité, éclaircissant la peau ou réduisant la pigmentation de la peau, d'un composé de formule I, tel que défini dans la revendication 1, ou d'un de ses sels pharmaceutiquement acceptables.

22. Utilisation d'un composé de formule I, tel que défini dans la revendication 1, ou d'un de ses sels pharmaceutiquement acceptables, dans la production d'un médicament pour l'éclaircissement de la peau ou la réduction de la pigmentation de la peau.

23. Méthode cosmétique pour l'éclaircissement de la peau ou la réduction de la pigmentation de la peau chez un patient humain, comprenant l'administration au patient d'une quantité, efficace pour éclaircir la peau ou réduire la pigmentation de la peau, d'un composé de formule I, tel que défini dans la revendication 1, ou d'un de ses sels pharmaceutiquement acceptables, jusqu'à obtention d'un degré cosmétiquement bénéfique d'éclaircissement de la peau ou de réduction de la pigmentation de la peau.
